# EUROPEAN PATENT SPECIFICATION

(11) **EP 3 613 735 B1**
(45) Date of publication and mention of the grant of the patent: **12.04.2023**
(21) Application number: 18787262.7
(22) Date of filing: 18.04.2018
(51) Int. Cl.: C07D 405/06, A61P 3/00, A61P 3/10, A61P 1/16, A61K 31/496, A61K 31/343, A61K 31/428, C07D 417/06, C07D 413/06

(54) **NOVEL SIRT 1 ACTIVATOR AND MEDICINAL USE THEREOF**
NEUARTIGER SIRT-1-AKTIVATOR UND MEDIZINISCHE VERWENDUNG DAVON
NOUVEL ACTIVATEUR DE SIRT1 ET UTILISATION MÉDICINALE CORRESPONDANTE

(30) Priority: 18.04.2017 KR 20170049956
(43) Date of publication of application: 26.02.2020
(73) Proprietor: Pusan National University Industry - University Cooperation Foundation, Busan 46241 (KR)
(72) Inventor: MOON, Hyung Ryong, Busan 48037 (KR); CHUNG, Hae Young, Busan 47872 (KR); AN, Hye Jin, Busan 46567 (KR); SON, Su Jin, Busan 46296 (KR); KIM, Do Hyun, Ulsan 44763 (KR)
(74) Representative: Roos, Peter
(86) International application number: PCT/KR2018/004502
(87) International publication number: WO 2018/194372

(56) References cited:
- WO-A1-2007/019345
- WO-A1-2007/019345
- WO-A1-2011/141458
- WO-A1-2011/141458
- WO-A2-2007/070173
- WO-A2-2010/039186
- PARK, H. R. et al: "3D-QSAR of SIRT1 Activators Targeting Against Diet-Induced Metabolic Syndrome", Bull. Korean Chem. Soc., vol. 30, no. 9, 1 January 2009 (2009-01-01), pages 2117-2120, XP055644328, ISSN: 0253-2964, DOI: 10.5012/bkcs.2009.30.9.2117
- KAYASHIMA, Y.: "Alkylresorcinols activate SIRT1 and delay ageing in Drosophila melanogaster", Scientific Reports, vol. 7, no. 1, 2 March 2017 (2017-03-02), pages 1-8, XP055644334, DOI: 10.1038/srep43679

## Description

### [Technical Field]

The present invention relates to a novel SIRT 1 (silent mating type information regulation 2 homolog; sirtuin 1) activator compound, a crystalline form thereof, a hydrate thereof or a salt thereof and a composition comprising the same as an active ingredient.

### [Background Art]

SIRT 1 is a NAD (nicotinamide adenine dinucleotide)-dependent histone deacetylase which is distributed in the nucleus or cytoplasm and mainly functions by deacetylating lysine of histone H3 (Lys9 or Lys14) or lysine of histone H4 (Lys16) of various transcriptional regulators in the nucleus. SIRT 1 in mammals affects the signaling system through deacetylation of transcriptional regulators in addition to histones. Transcriptional regulators Interacting with SIRT 1 are p53, forkhead transcription factor (FOXO), peroxisome proliferator-activated receptor γ coactivator 1-α (PGC1-α) and peroxisome proliferator-activated receptor-gamma (PPAR-γ) and they are known to be associated with aging, cell cycle regulation, apoptosis, metabolism, inflammation and the like.

Resveratrol is a phytoalexin, a type of polyphenol, found in many plants, including grapes, peanuts, mulberry, berry such as raspberry, cranberry. Resveratrol is known to have anti-cancer and potent antioxidant activity in addition to anti-viral, neuroprotective, anti-inflammatory, anti-aging and life extension effects. In addition, resveratrol is known to be a potent SIRT 1 activator and to enhance the survival rate of cells stressed by UV as well as SIRT 1 dependent cytoplasmic processes such as axonal protection, fat metabolism and inhibition of NF-κB dependent transcription.

Recently, research to discover a stronger SIRT 1 activator than resveratrol has attracted attention, and thus a compound called SRT1720 based on imdazo [2,1-b] thiazole backbone has been reported as a stronger SIRT 1 activator than resveratrol. Furthermore, recently, it has be reported that PPARβ/δ is a transcriptional regulator that regulates the expression of SIRT 1 and the activator of PPARβ/δ increases the expression of SIRT 1 to improve metabolic diseases and cellular aging.

WO 2007/019345 A1 relates to imidazopyridine derivatives which are sirtuin-modulating compounds and methods of use thereof. The sirtuin-modulating compounds may be used for increasing the lifespan of a cell, and treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing as well as diseases or disorders that would benfit from increased mitochondrial activity. WO 2011/141458 A1 relates to polysubstituted benzofuran compounds, as well as to a method for their preparation, pharmaceutical compositions comprising the same, and use thereof for the treatment and/or chemoprevention of cancer, aging related diseases or processes, diabetes and neurodegenerative diseases.

Accordingly, the present inventors synthesized various compounds based on benzo[d]furan or benzo[d]oxazole, which is similar to the backbone of imidazo[2,1-b] thiazole, which is the structure of SRT1720 and explored SIRT 1 activity and PPARβ/δ activity of the compounds to identify various effects in metabolic diseases (obesity, diabetes, dyslipidemia, etc.), inflammation, cellular aging and prolongation of life.

### [Disclosure]

### [Technical Problem]

It is an object of the present invention to provide novel SIRT 1 activator compound, a crystalline form thereof, a hydrate thereof or a salt thereof.

It is another object of the present invention is to provide a pharmaceutical composition for preventing or treating metabolic disease, a pharmaceutical composition for preventing or treating liver disease, a cosmetic composition for preventing or improving wrinkles, and a health food composition for improving cell aging or extending lifespan, comprising the compound, the crystalline form thereof, the hydrate thereof, or the salt thereof as an active ingredient.

### [Technical Solution]

In order to achieve the above object, the present invention provides a novel SIRT 1 activator compound, a crystalline form thereof, a hydrate thereof or a salt thereof as defined in claims 1 and 3.

In addition, the present invention provides a pharmaceutical composition for use in preventing or treating metabolic disease as defined in claim 5, a pharmaceutical composition for use in preventing or treating liver disease as defined in claim 7, a cosmetic composition for preventing or improving wrinkles as defined in claim 9, and a health food composition as defined in claim 10, comprising the compound, the crystalline form thereof, the hydrate thereof, or the salt thereof as an active ingredient.

### [Advantageous Effects]

In the present invention, a novel SIRT 1 activator compound based on benzo[d]furan or a benzo[d]oxazole backbone, a crystalline form thereof, a hydrate thereof, or a salt thereof was prepared, and that the compound has obesity, insulin resistance and dyslipidemia improvement effect, fatty liver improvement effect, cell aging protection, oxidative stress protection and yeast life extension effect, collagen synthesis and wrinkle improvement effect, was confirmed.

Therefore, the novel SIRT 1 activator compound having the above-mentioned effect is usefully used for pharmaceutical composition for preventing or treating metabolic diseases including obesity, diabetes mellitus, dyslipidemia, etc. and liver diseases including alcoholic or non-alcoholic fatty liver, fatty hepatitis, etc. a cosmetic composition for preventing or improving wrinkles, a health food composition for improving cell aging or extending lifespan, and the like.

### [Description of Drawings]

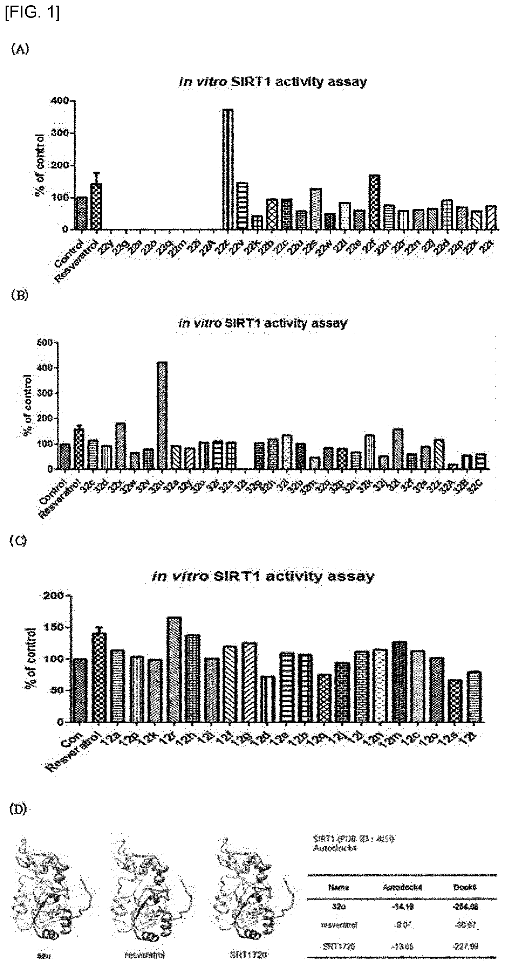

FIG. 1A to FIG. 1C show results of confirming SIRT 1 activity of a candidate SIRT 1 activator compound (benzo[d]furan backbone), and FIG. 1D shows the result of confirming the binding affinity of a SIRT 1 of compound 32u among a candidate SIRT 1 activator compound by performing a docking simulation.

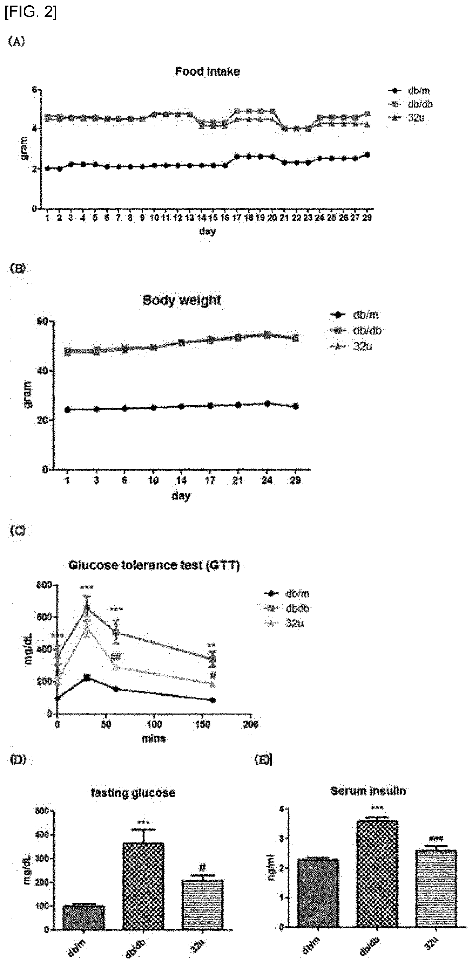

FIG. 2 shows results of confirming the change of A) dietary intake, B) weight, C) glucose tolerance, D) fasting blood sugar and E) serum insulin by analyzing the improvement of metabolic disease caused by compound 32u in the obese animal model of db/db mouse lacking the appetite-regulating hormone leptin receptor.

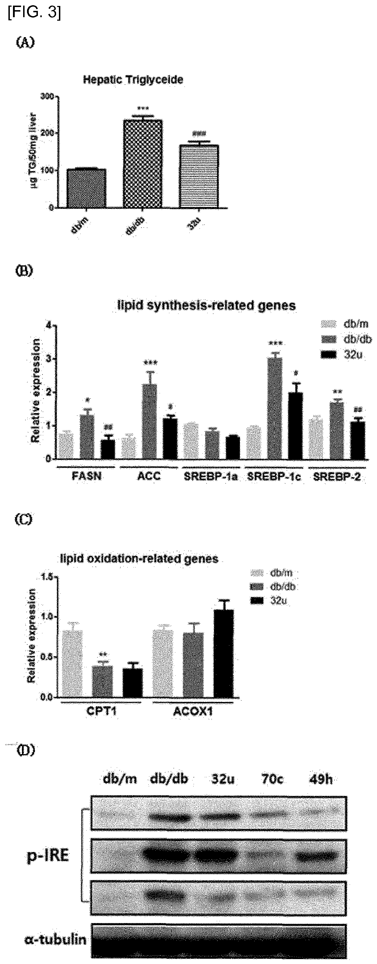

FIG. 3 shows results of confirming the change of A) triglycerides, B) lipid synthesis related genes, C) lipid oxidation related genes, and D) endoplasmic reticulum stress related markers (p-IRE) expression by analyzing fatty liver improvement effect of the compound 32u in db/db mouse obesity animal model.

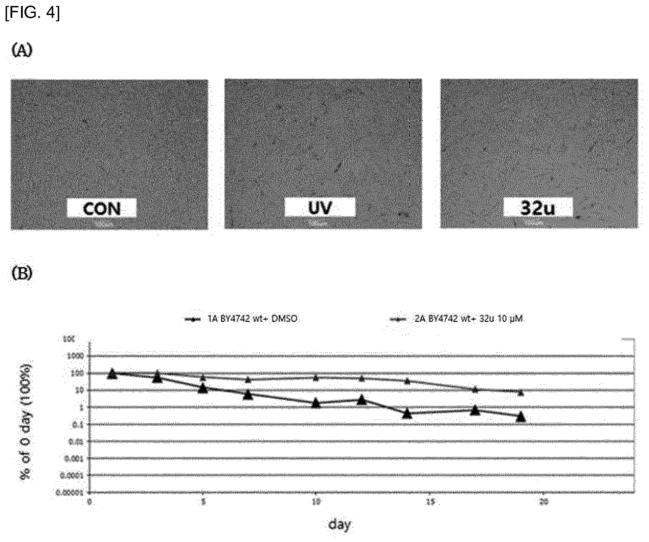

FIG. 4A shows results of confirming the cell aging improvement effect by the compound 32u in skin fibroblasts through the β-galactosidase staining; and FIG. 4B shows the result of confirming the life extension effect by the compound 32u in the yeast (Saccharomyces cerevisiae) model.

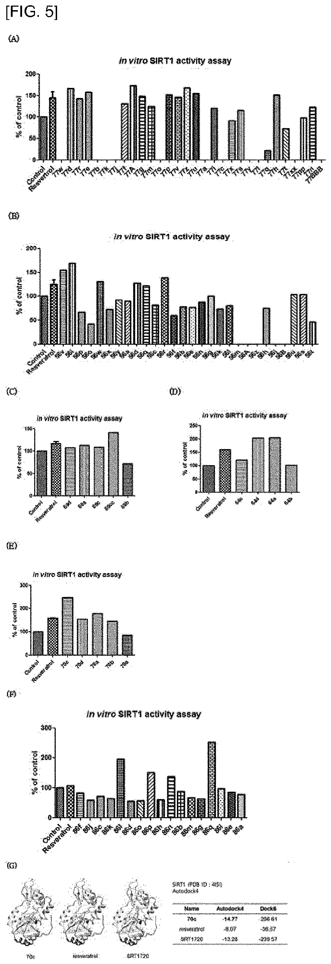

FIG. 5A to FIG. 5F show results of confirming SIRT 1 activity of a candidate SIRT 1 activator compound (benzo[d]oxazole backbone), and FIG. 5G shows the result of confirming the binding affinity of a SIRT 1 of compound 70c among a candidate SIRT 1 activator compound by performing a docking simulation.

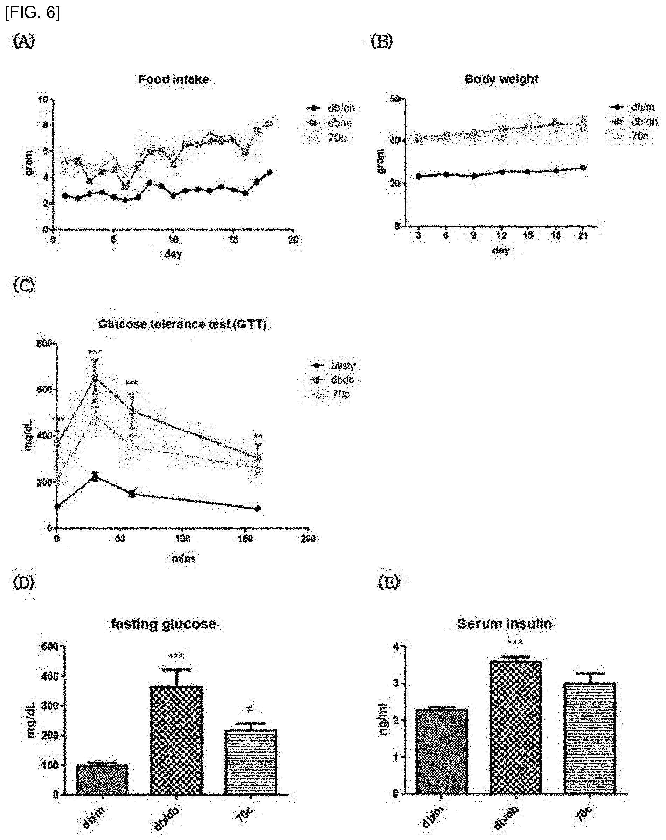

FIG. 6 shows results of confirming the change of A) dietary intake, B) weight, C) glucose tolerance, D) fasting blood sugar and E) serum insulin by analyzing the improvement of metabolic disease caused by compound 70c in the obese animal model of db/db mouse lacking the appetite-regulating hormone leptin receptor.

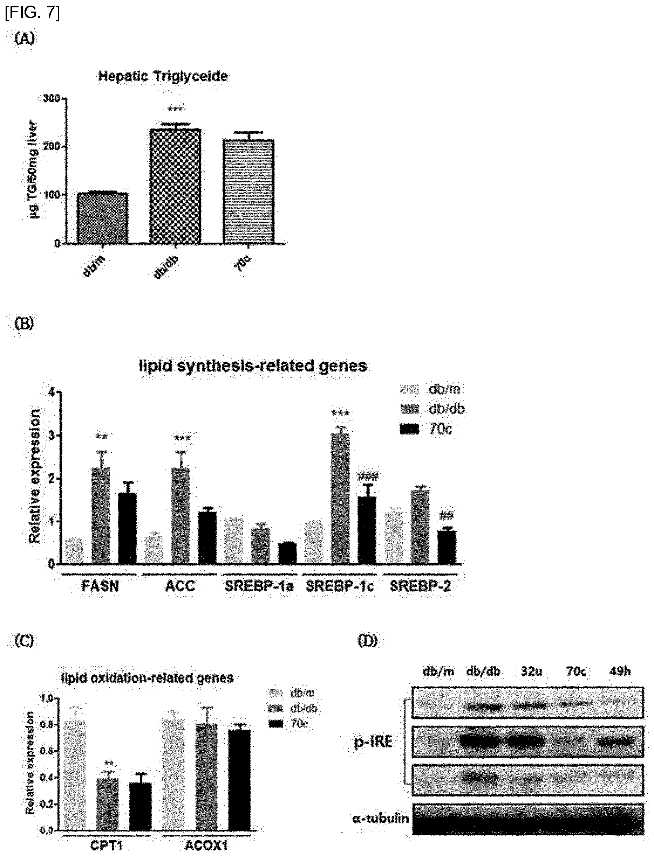

FIG. 7 shows the results of confirming the change of A) triglycerides, B) lipid synthesis related genes, C) lipid oxidation related genes, and D) endoplasmic reticulum stress related markers (p-IRE) expression by analyzing fatty liver improvement effect of the compound 70c in db/db mouse obesity animal model.

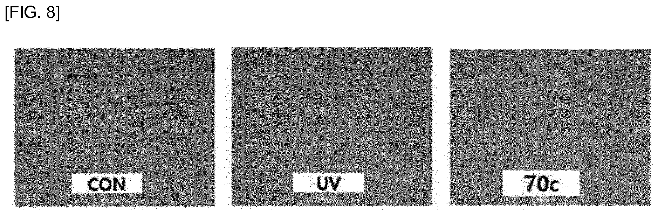

FIG. 8 shows results of confirming the cell aging improvement effect by the compound 70c in skin fibroblasts through the β-galactosidase staining.

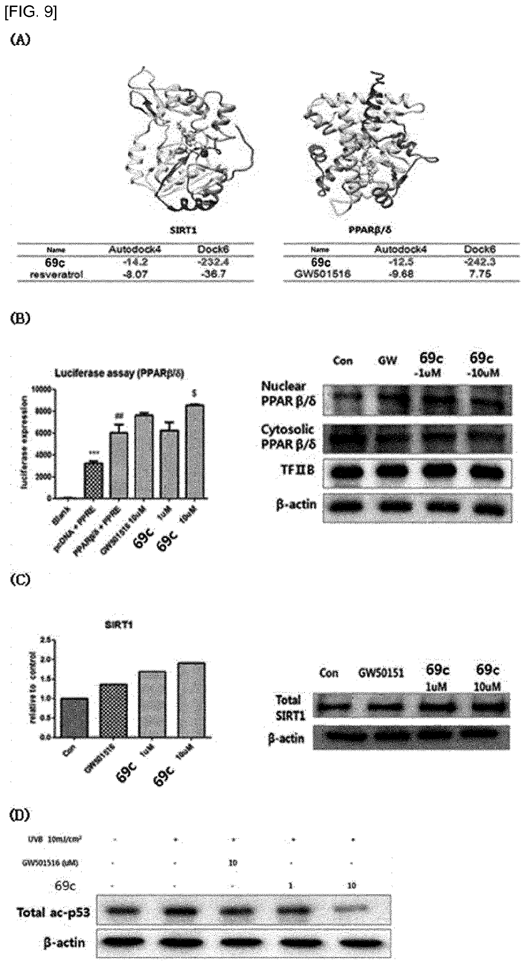

FIG. 9A shows results of confirming the binding affinity of SIRT 1 and PPARβ/δ of the compound 69c in candidate SIRT 1 activator compounds (benzo[d]oxazole backbone) by performing docking simulation, and the changes of B) PPARβ/δ expression, C) SIRT 1 expression and D) expression of the cellular aging marker (p53).

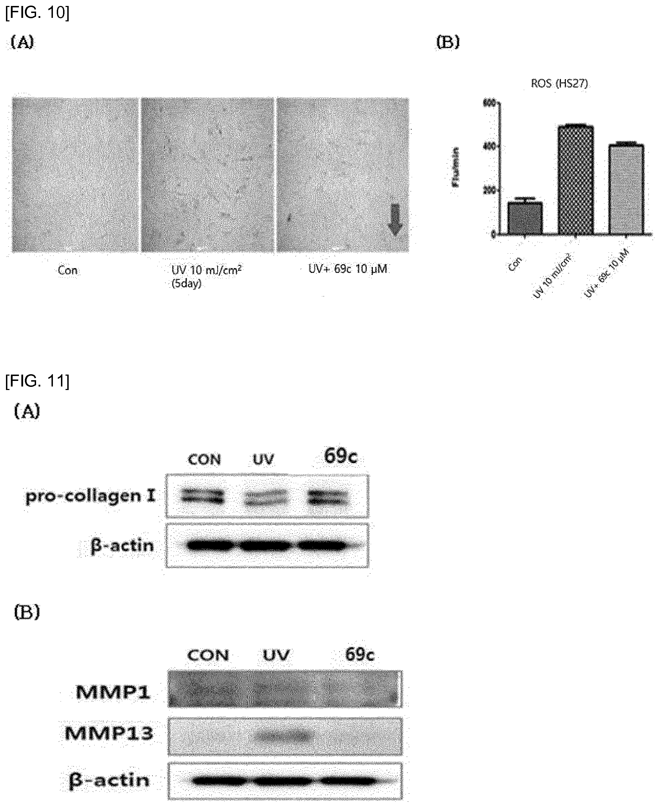

FIG. 10A shows a result of confirming the cell aging effect of the compound 69c in skin fibroblasts by the β-galactosidase staining; and FIG. 10B shows a result of confirming the inhibitory effect of reactive oxygen species by compound 69c.
FIG. 11 shows a result of confirming A) increased collagen synthesis and B) decreased matrix metalloproteinase (MMP) expression by compound 69c in the photoaging of skin fibroblast.

### [Best Mode]

The inventors of the present invention synthesized a novel SIRT 1 compound, a crystalline form thereof, a hydrate thereof, or a salt form thereof, based on the benzo[d]furan or benzo[d]oxazole backbone and confirmed that the compound has obesity, insulin resistance and dyslipidemia improvement effect, fatty liver improvement effect, cell aging protection, oxidative stress protection and yeast life extension effect, collagen synthesis and wrinkle improvement effect, and completed the present invention.

The present invention provides a compound according to claim 1 and represented by the following Chemical Formula 2-3, a crystalline form thereof, a hydrate thereof or a salt thereof: 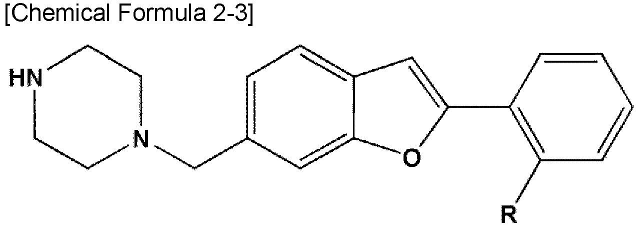 where R is any one selected from the group consisting of benzamide, nicotinamide, isonicotinamide, naphthaamide, quinoline-2-carboxamide, quinoxaline-2-carboxamide, cinnamamide, adamantane-1-carboxamide, phenylurea, naphthylurea and benzylurea, which is unsubstituted or substituted with one or more substituents selected from (C1-C4)alkyl, (C1-C4)alkoxy, halogen, nitro or phenyl.

The crystalline form means that is what at least 50%, preferably at least 70%, 80% or 90% by weight of the compound is crystalline.

The hydrate means that the compound or salts thereof constitutes equivalently or non-equivalently with water.

The salts can be prepared by themselves or specially prepared in the process of the synthesis, separation or purification of the compound. Examples of salts prepared as the above are at least basic salt selected from the group consisting of sodium salts, potassium salts, calcium salts, lithium salts, magnesium salts, cesium salts, aminum salts, ammonium salts, triethylaminium salts and pyridinium salts. It may be one or more acid salts selected from the group consisting of hydrochloric acid, bromic acid, sulfuric acid, sulfurous acid, phosphoric acid, citric acid, acetic acid, maleic acid, fumaric acid, gluconic acid, methanesulfonic acid, benzenesulfonic acid, camphorsulfonic acid, oxalic acid, malonic acid, glutaric acid, acetic acid, trifluoroacetic acid, glyconic acid, succinic acid, tartaric acid, 4-toluenesulfonic acid, galacturonic acid, embonic acid, glutamic acid, citric acid and aspartic acid, but it is not limited thereto.

Preferably, the compound represented by Chemical Formula 2-3 may be selected from the group consisting of [*N*-(2-(6-(Piperazin-1-ylmethyl)benzofuran-2-yl)phenyl)benzamide hydrochloride] (32a), [2-Methyl-*N*-(2-(6-(piperazin-1-ylmethyl)benzofuran-2-yl)phenyl)benzamide hydrochloride] (32b), [3-Methyl-*N*-(2-(6-(piperazin-1-ylmethyl)benzofuran-2-yl)phenyl)benzamide hydrochloride] (32c), [4-Methyl-*N*-(2-(6-(piperazin-1-ylmethyl)benzofuran-2-yl)phenyl)benzamide hydrochloride] (32d), [2-Fluoro-*N*-(2-(6-(piperazin-1-ylmethyl)benzofuran-2-yl)phenyl)benzamide hydrochloride] (32e), [3-Fluoro-*N*-(2-(6-(piperazin-1-ylmethyl)benzofuran-2-yl)phenyl)benzamide hydrochloride] (32f), [2-Bromo-*N*-(2-(6-(piperazin-1-ylmethyl)benzofuran-2-yl)phenyl)benzamide hydrochloride] (32g), [3-Bromo-*N*-(2-(6-(piperazin-1-ylmethyl)benzofuran-2-yl)phenyl)benzamide hydrochloride] (32h), [4-Bromo-*N*-(2-(6-(piperazin-1-ylmethyl)benzofuran-2-yl)phenyl)benzamide hydrochloride] (32i), [2-Chloro-*N*-(2-(6-(piperazin-1-ylmethyl)benzofuran-2-yl)phenyl)benzamide hydrochloride] (32j), [3-Chloro-*N*-(2-(6-(piperazin-1-ylmethyl)benzofuran-2-yl)phenyl)benzamide hydrochloride] (32k), [4-Chloro-*N*-(2-(6-(piperazin-1-ylmethyl)benzofuran-2-yl)phenyl)benzamide hydrochloride] (32l), [3-Nitro-*N*-(2-(6-(piperazin-1-ylmethyl)benzofuran-2-yl)phenyl)benzamide hydrochloride] (32m), [4-Nitro-*N*-(2-(6-(piperazin-1-ylmethyl)benzofuran-2-yl)phenyl)benzamide hydrochloride] (32n), [3,4,5-Trimethoxy-*N*-(2-(6-(piperazin-1-ylmethyl)benzofuran-2-yl)phenyl)benzamide hydrochloride] (32o), [2,4-Dichloro-*N*-(2-(6-(piperazin-1-ylmethyl)benzofuran-2-yl)phenyl)benzamide hydrochloride] (32p), [3,4-Dichloro-*N*-(2-(6-(piperazin-1-ylmethyl)benzofuran-2-yl)phenyl)benzamide hydrochloride] (32q), [*N*-(2-(6-(Piperazin-1-ylmethyl)benzofuran-2-yl)phenyl)nicotinamide hydrochloride] (32r), [*N*-(2-(6-(Piperazin-1-ylmethyl)benzofuran-2-yl)phenyl)isonicotinamide hydrochloride] (32s), [*N*-(2-(6-(Piperazin-1-ylmethyl)benzofuran-2-yl)phenyl)-1-naphthamide hydrochloride] (32t), [*N*-(2-(6-(Piperazin-1-ylmethyl)benzofuran-2-yl)phenyl)-2-naphthamide hydrochloride] (32u), [*N*-(2-(6-(Piperazin-1-ylmethyl)benzofuran-2-yl)phenyl)quinoline-2-carboxamide hydrochloride] (32v), [*N*-(2-(6-(Piperazin-1-ylmethyl)benzofuran-2-yl)phenyl)quinoxaline-2-carboxamide hydrochloride] (32w), [*N*-(2-(6-(Piperazin-1-ylmethyl)benzofuran-2-yl)phenyl)biphenyl-4-carboxamide hydrochloride (32x), [*N*-(2-(6-(Piperazin-1-ylmethyl)benzofuran-2-yl)phenyl)cinnamamide hydrochloride] (32y), [(3*r*,5*r*,7*r*)-*N*-(2-(6-(Piperazin-1-ylmethyl)benzofuran-2-yl)phenyl)adamantane-1-carboxamide hydrochloride] (32z), [1-Phenyl-3-(2-(6-(piperazin-1-ylmethyl)benzofuran-2-yl)phenyl)urea hydrochloride] (32A), [1-(Naphthalen-1-yl)-3-(2-(6-(piperazin-1-ylmethyl)benzofuran-2-yl)phenyl)urea hydrochloride] (32B) and [1-Benzyl-3-(2-(6-(piperazin-1-ylmethyl)benzofuran-2-yl)phenyl)urea hydrochloride] (32C), but it is not limited thereto.

Alsodescribed is a compound represented by the following Chemical Formula 4-2, a crystalline form thereof, a hydrate thereof or a salt thereof: 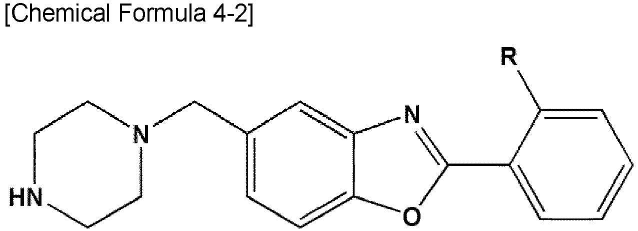 where R is any one selected from the group consisting of benzamide, naphthaamide and quinoxaline-2-carboxamide unsubstituted or substituted with one or more substituents selected from (C1-C4)alkoxy.

Preferably, the compound represented by Chemical Formula 4-2 may be selected from the group consisting of [*N*-(2-(5-(Piperazin-1-ylmethyl)benzo[d]oxazol-2-yl)phenyl)benzamide] (64a), [3,4,5-Trimethoxy-*N*-(2-(5-(piperazin-1-ylmethyl)benzo[*d*]oxazol-2-yl)phenyl)benzamide] (64b), [*N*-(2-(5-(Piperazin-1-ylmethyl)benzo[*d*]oxazol-2-yl)phenyl)-2-naphthamide] (64c) and [*N*-(2-(5-(Piperazin-1-ylmethyl)benzo[*d*]oxazol-2-yl)phenyl)quinoxaline-2-carboxamide 2,2,2-trifluoroacetate] (64d), but it is not limited thereto.

Also described is a compound represented by the following Chemical Formula 4-3, a crystalline form thereof, a hydrate thereof or a salt thereof: 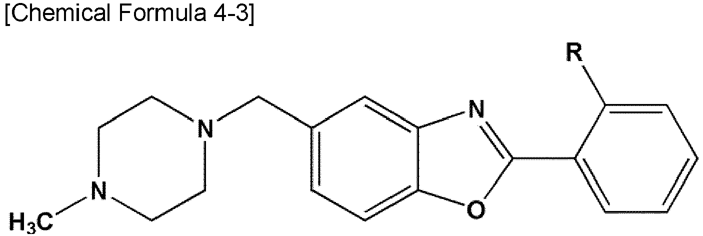 where R is any one selected from the group consisting of benzamide, naphthaamide and quinoxaline-2-carboxamide unsubstituted or substituted with one or more substituents selected from (C1-C4)alkoxy.

Preferably, the compound represented by Chemical Formula 4-3 may be selected from the group consisting of [*N*-(2-(5-((4-Methylpiperazin-1-yl)methyl)benzo[*d*]oxazol-2-yl)phenyl)benzamide] (69a), [3,4,5-Trimethoxy-*N*-(2-(5-((4-methylpiperazin-1-yl)methyl)benzo[*d*]oxazol-2-yl)phenyl)benzamide] (69b), [*N*-(2-(5-((4-Methylpiperazin-1-yl)methyl)benzo[*d*]oxazol-2-yl)phenyl)-2-naphthamide] (69c), [*N*-(2-(5-((4-Methylpiperazin-1-yl)methyl)benzo[*d*]oxazol-2-yl)phenyl)-2-naphthamide hydrochloride] (69cc) and [*N*-(2-(5-((4-Methylpiperazin-1-yl)methyl)benzo[*d*]oxazol-2-yl)phenyl)quinoxaline-2-carboxamide] (69d).

The present invention further provides a compound represented by the following Chemical Formula 4-4, a crystalline form thereof, a hydrate thereof or a salt thereof: 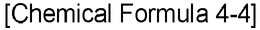 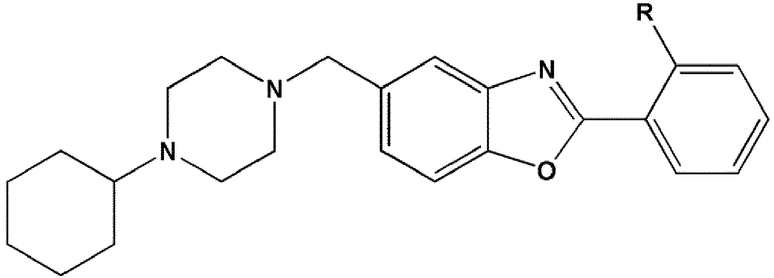 where R is any one selected from the group consisting of benzamide, naphthaamide and quinoxaline-2-carboxamide unsubstituted or substituted with one or more substituents selected from (C1-C4)alkoxy.

Preferably, the compound represented by Chemical Formula 4-4 may be selected from the group consisting of [N-(2-(5-((4-Cyclohexylpiperazin-1-yl)methyl)benzo[d]oxazol-2-yl)phenyl)benzamide] (70a), [N-(2-(5-((4-Cyclohexylpiperazin-1-yl)methyl)benzo[d]oxazol-2-yl)phenyl)-3,4,5-trimethoxybenzamide] (70b), [N-(2-(5-((4-Cyclohexylpiperazin-1-yl)methyl)benzo[d]oxazol-2-yl)phenyl)-2-naphthamide] (70c) and [N-(2-(5-((4-Cyclohexylpiperazin-1-yl)methyl)benzo[d]oxazol-2-yl)phenyl)quinoxaline-2-carboxamide] (70d), but it is not limited thereto.

Furthermore, the present invention provides a pharmaceutical composition for use in preventing or treating metabolic diseases, comprising the compound, a crystalline form thereof, a hydrate thereof, or a salt thereof according to the present invention as an active ingredient.

Preferably, the metabolic disease may be selected from the group consisting of obesity, diabetes, hyperinsulinemia, hyperuricemia, hyperlipidemia, hypercholesterolemia, hypertriglyceridemia, metabolic syndrome and endothelial dysfunction, but it is not limited thereto.

In addition, the present invention provides a pharmaceutical composition for use in preventing or treating liver disease, comprising the compound, a crystalline form thereof, a hydrate thereof, or a salt thereof according to the present invention as an active ingredient.

Preferably, the liver disease may be selected from the group consisting of alcoholic fatty liver, non-alcoholic fatty liver and fatty hepatitis, but it is not limited thereto.

When the composition of the invention is a pharmaceutical composition, it may be formulated as a cream, gel, patch, spray, ointment, plaster, lotion, liniment, pasta and cataplasma. On the other hand, the pharmaceutical composition may comprise a pharmaceutically acceptable carrier in addition to the active ingredient, and the pharmaceutically acceptable carrier is commonly used in pharmaceutical formulations and includes lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but it is not limited thereto. In addition, the pharmaceutical composition may further include lubricant, wetting agent, sweetener, flavoring agent, emulsifier, suspending agent, preservative, and the like as an additive.

The pharmaceutical composition is determined by the method of administration depending on the degree of symptoms, usually topical administration is preferred. In addition, the dosage of the active ingredient in the pharmaceutical composition may vary depending on the route of administration, the degree of the disease, the age, sex, and weight of the patient, and may be administered once to several times daily.

In addition, the present invention provides a cosmetic composition for preventing or improving wrinkles, comprising the compound, a crystalline form thereof, a hydrate thereof, or a salt thereof according to the present invention as an active ingredient.

When the composition of the present invention is a cosmetic composition, the cosmetic composition may include conventional adjuvants such as stabilizers, solubilizers, vitamins, pigments and flavors, and a carrier in addition to the active ingredient. In addition, the cosmetic composition may further include a skin absorption enhancer to enhance its effect.

When the composition of the present invention is a cosmetic composition, the formulation of the cosmetic composition may be prepared in any formulation commonly prepared in the art, for example, the cosmetic composition may be face lotion, emulsion, skin lotion, toner, lotion, essence, sunscreen, cream, makeup base, foundation, powder, lipstick, pack, gel, shampoo, rinse, spray, ointment, patch, aerosol, body cleanser, makeup remover and cleanser, etc., but it is not limited thereto.

When the formulation is paste, cream or gel, it may use animal oil, vegetable oil, wax, paraffin, starch, tragacanth, cellulose derivative, polyethylene glycol, silicone, bentonite, silica, talc or zinc oxide and the like, as the carrier component.

When the formulation is powder or spray, it may use lactose, talc, silica, aluminum hydroxide, calcium silicate or polyamide powder, as the carrier component, and in particular, the spray may additionally include a propellant such as chlorofluorohydrocarbon, propane/butane or dimethyl ether.

When the formulation is solution or emulsion, solvent, solubilizer or emulsifying agent are used as the carrier component, for examples water, ethanol, isopropanol, ethyl carbonate, ethyl acetate, benzyl alcohol, benzyl benzoate, propylene glycol, 1,3-butylglycol oil, glycerol aliphatic ester, polyethylene glycol or fatty acid esters of sorbitan.

When the formulation is suspension, it may use liquid diluents such as water, ethanol or propylene glycol, suspending agents such as ethoxylated isostearyl alcohol, polyoxyethylene sorbitol esters, and polyoxyethylene sorbitan esters, microcrystalline cellulose, aluminum metahydroxide, bentonite, agar or tragacanth, as the carrier component.

In addition, the present invention provides a health food composition comprising the compound, a crystalline form thereof, a hydrate thereof, or a salt thereof according to the present invention as an active ingredient.

The health food composition may be provided in the form of powder, granules, tablets, capsules, syrups or beverages. The health food composition may be used with other food or food additives in addition to an active ingredient, and may be appropriately used according to a conventional method. The mixed amount of the active ingredient can be suitably determined depending on the purpose of use thereof, for example, prophylaxis, health or therapeutic treatment.

The effective dose of the active ingredient contained in the health food composition may be used in accordance with the effective dose of the pharmaceutical composition, but in the case of prolonged intake for the purpose of health and hygiene or health control, it may be less than the above range and since the active ingredient has no problem in terms of safety, it is evident that the active ingredient may be used in an amount above the above range.

There is no particular limitation on the kind of the health food, for example, meat, sausage, bread, chocolate, candy, snacks, confectionery, pizza, ramen, other noodles, gums, dairy products including ice cream, various soups, beverages, tea, drinks, alcoholic drinks, vitamin complexes, etc.

Hereinafter, the present invention will be described in detail with reference to the following examples. It will be apparent to those skilled in the art that the following examples are intended to illustrate the contents of the present invention, but the scope of the present invention is not limited to the following examples.

### Example 1: Synthesis of benzofuran compound

### 1-1. Synthesis of Compound 23 to 32

Compounds 23 to 32 were synthesized as in Reaction Scheme 3 below.

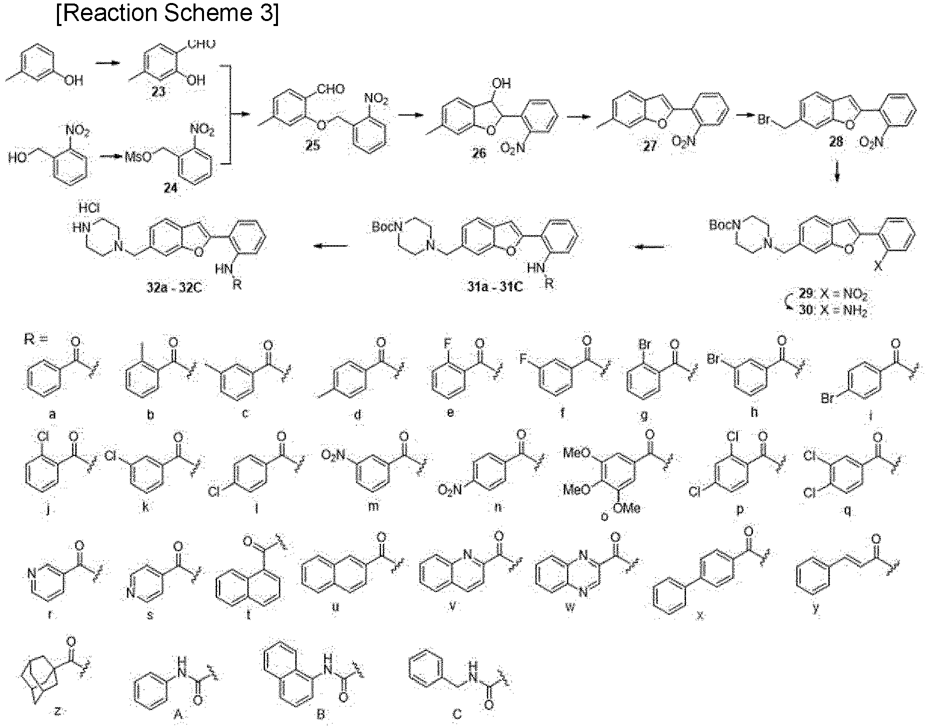

### 1) [2-Hydroxy-4-methylbenzaldehyde] (23)

MgCl₂ (26.0 g, 273.1 mmol), triethylamine (EtsN, 103 mL, 738.99 mmol) and paraformaldehyde (39.0 g, 1.30 mol) was added in anhydrous THF (tetrahydrofuran, 600 mL) solution containing *m*-cresol (20.0 g, 184.85 mmol) sequentially at 0 °C and then stirred under reflux for 1 hour. 1N-HCl was added until the insoluble solid disappeared and became a translucent liquid, extracted with diethyl ether, dried over anhydrous MgSO₄, filtered, and the filtrate was concentrated under reduced pressure. The residue obtained was filtered and washed with methanol to obtain Compound **23** as a solid. The residue obtained by concentrating the filtrate under reduced pressure again was purified by silica gel column chromatography (hexane:methylene chloride = 1:2) to obtain Compound **23**.

White solid; Reaction time, 1 hour; yield, 79.4%; ¹H NMR (400 MHz, CDCl₃) *δ* 11.04 (s, 1 H), 9.82 (s, 1 H), 7.42 (d, 1 H, *J* = 8.0 Hz), 6.82 (d, 1 H, *J* = 8.0 Hz), 6.79 (s, 1 H), 2.37 (s, 3 H); ¹³C NMR (100 MHz, CDCl₃) *δ* 197.1, 162.0, 149.2, 133.8, 121.4, 118.9, 118.0, 22.5.

### 2) [4-Methyl-2-(2-nitrobenzyloxy)benzaldehyde] (25)

2-nitrobenzyl alcohol (11.25 g, 73.46 mmol) was dissolved in anhydrous 1,4-dioxane (18 mL) and triethylamine (11.25 mL, 80.71 mmol) and methanesulfonyl chloride (6.12 mL, 79.35 mmol) were added slowly in order at 0 °C, followed by stirring at the same temperature for 30 minutes. The reaction mixture was filtered using celite as a filter filler to obtain a filtrate containing Compound **24** (2-nitrobenzyl methanesulfonate). The filtrates containing Compound **24** were added Into a solution of K₂CO₃ (10.0 g, 72.36 mmol) and Compound **23** in DMA (*N,N*-dimethylacetamide, 40 mL), respectively at room temperature and stirred for 41 hours. Ice water was added thereto and the resulting solid was filtered to obtain Compound **25** (13.2 g, 66.3%) as a solid.

¹H NMR (400 MHz, CDCl₃) *δ* 10.51 (s, 1 H), 8.21 (d, 1 H, *J* = 8.0 Hz), 7.98 (d, 1 H, *J* = 8.0 Hz), 7.77 (d, 1 H, *J* = 7.6 Hz), 7.74 (t, 1 H, *J* = 8.0 Hz), 7.54 (t, 1 H, *J* = 8.0 Hz), 6.91 (d, 1 H, *J* = 7.6 Hz), 6.87 (s, 1 H), 5.59 (s, 2 H), 2.41 (s, 3 H); ¹³C NMR (100 MHz, CDCl₃) δ 189.2, 160.3, 148.0, 146.9, 134.6, 133.3, 129.8, 128.9, 128.6, 125.4, 123.2, 122.8, 113.6, 67.4, 22.6.

### 3) [6-Methyl-2-(2-nitrophenyl)benzofuran] (27)

Compound **25** (13.2 g, 48.66 mmol) and NaOEt (430 mg, 6.32) were dissolved in DMA (67 mL) and stirred at room temperature for 30 minutes. Ethyl acetate and water were added, the organic layer was dried over anhydrous MgSO₄, filtered, and the filtrate was concentrated under reduced pressure to obtain the residue containing Compound **26** (6-methyl-2-(2-nitrophenyl)-2,3-dihydrobenzofuran-3-ol). Formic acid (50 mL) was added thereto and stirred at 40 °C. for 15 minutes. Methylene chloride and water were added, the organic layer was washed with sodium bicarbonate water, and the residue obtained by concentrating the organic layer under reduced pressure was recrystallized with ethanol and a small amount of methylene chloride to obtain Compound **27** (9.6 g, 77.9%).

¹H NMR (500 MHz, CDCl₃) *δ* 7.85 (dd, 1 H, *J* = 7.5, 1.0 Hz), 7.75 (d, 1 H, *J* = 8.0 Hz), 7.63 (td, 1 H, *J* = 7.0, 1.0 Hz), 7.48 (d, 1 H, *J* = 7.5 Hz), 7.48 (td, 1 H, *J* = 7.5, 1.0 Hz), 7.31 (s, 1 H), 7.09 (d, 1 H, *J* = 7.5 Hz), 6.97 (s, 1 H), 2.48 (s, 3 H); ¹³C NMR (100 MHz, CDCl₃) δ 155.9, 150.0, 148.3, 136.1, 132.2, 130.0, 129.3, 126.2, 125.0, 124.6, 124.3, 121.2, 111.8, 106.2, 22.1.

### 4) [6-(Bromomethyl)-2-(2-nitrophenyl)benzofuran] (28)

*N*-bromosuccinimide (351.4 mg, 1.97 mmol) and benzoyl peroxide (24 mg, 0.1 mmol) were added slowly into anhydrous chloroform (25 mL) solution containing Compound **27** (500 mg, 1.97 mmol) at room temperature, and followed by stirring under reflux for 5 hours. Methylene chloride and water were added, the organic layer was dried over anhydrous MgSO₄, filtered, and the residue obtained by concentrating the filtrate under reduced pressure was purified by silica gel column chromatography (hexane:ethyl acetate = 6:1) to obtain Compound **28** (520 mg, 79%) as a beige solid.

¹H NMR (400 MHz, CDCl₃) *δ* 7.79 (dd, 1 H, *J* = 7.6, 1.2 Hz), 7.75 (dd, 1 H, *J* = 8.4, 1.2 Hz), 7.60 (td, 1 H, *J* = 7.6, 1.2 Hz), 7.54 (d, 1 H, *J =* 8.0 Hz), 7.50 (s, 1 H), 7.47 (td, 1 H, *J* = 7.6, 1.2 Hz), 7.28 (dd, 1 H, *J* = 8.0, 1.2 Hz), 6.97 (d, 1 H, *J* = 1.2 Hz), 4.62 (s, 2 H); ¹³C NMR (100 MHz, CDCl₃) *δ* 155.2, 151.9, 148.3, 135.5, 132.4, 130.2, 129.9, 128.9, 124.9, 124.4, 124.1, 122.0, 112.2, 106.1, 34.4.

### 5) [tert-Butyl 4-((2-(2-nitrophenyl)benzofuran-6-yl)methyl)piperazine-1-carboxylate] (29)

*Tert*-butyl piperazine-1-carboxylate (1-Boc-piperazine, 229 mg, 1.23 mmol) in anhydrous THF (10 mL) solution was added slowly into anhydrous THF (10 mL) solution containing Compound **28** (341 mg, 1.03 mmol) and anhydrous triethylamine (EtsN, 0.43 mL, 3.09 mmol). After stirring at room temperature for 7.5 hours, methylene chloride and water were added, the organic layer was dried over anhydrous MgSO₄, filtered, and the residue obtained by concentrating the filtrate under reduced pressure was purified by silica gel column chromatography (methylene chloride:methanol = 50:1 ) to obtain Compound **29** (442 mg, 98.4%).

¹H NMR (400 MHz, CDCl₃) *δ* 7.84 (dd, 1 H, *J* = 8.0, 1.2 Hz, 6'-H), 7.77 (dd, 1 H, *J* = 8.0, 1.2 Hz, 3'-H), 7.64 (td, 1 H, *J* = 8.0, 1.2 Hz, 5'-H), 7.54 (d, 1 H, *J* = 8.0 Hz, 4-H), 7.50 (td, 1 H, *J =* 8.0, 1.2 Hz, 4'-H), 7.48 (s, 1 H, 3-H), 7.23 (dd, 1 H, *J=* 8.0, 1.2 Hz, 5-H), 6.99 (d, 1 H, *J =* 1.2 Hz, 7-H), 3.61 (s, 2 H, CH₂), 3.43 (t, 4 H, *J =* 4.8 Hz, 2"'-H, 6"'-H), 2.41 (t, 4 H, *J* = 4.8 Hz, 3"'-H, 5"'-H), 1.45 (s, 9 H, t-butyl); ¹³C NMR (100 MHz, CDCl₃) *δ* 155.7, 155.0, 150.8, 148.4, 136.2, 132.3, 130.1, 129.5, 127.8, 124.8, 124.5, 124.3, 121.3, 112.1, 106.1, 79.8, 63.4, 53.1, 44.2, 28.7.

### 6) [tert-Butyl 4-((2-(2-aminophenyl)benzofuran-6-yl)methyl)piperazine-1-carboxylate] (30)

10 wt% Pd/C (palladium on carbon, 20 mg) was added to a solution of methylene chloride (4 mL) containing Compound **29** (200 mg, 0.46 mmol), and the air in the reaction vessel was replaced with hydrogen gas and stirred for 4 hours at room temperature. The reaction mixture was filtered using celite as a filter and the filtrate was concentrated under reduced pressure to obtain Compound **30** (140 mg, 75%) as a solid.

¹H NMR (400 MHz, CDCl₃) *δ* 7.61 (d, 1 H, *J* = 7.6 Hz), 7.51 (d, 1 H, *J* = 8.0 Hz), 7.50 (s, 1 H), 7.20 (d, 1 H, *J* = 7.6 Hz), 7.17 (t, 1 H, *J* = 7.6 Hz), 6.91 (s, 1 H), 6.83 (t, 1 H, *J* = 7.6 Hz), 6.78 (d, 1 H, *J* = 8.0 Hz), 4.50 (s, 2 H), 3.63 (s, 2 H), 3.44 (t, 4 H, *J* = 4.4 Hz), 2.42 (t, 4 H, *J* = 4.4 Hz), 1.45 (s, 9 H); ¹³C NMR (100 MHz, CDCl₃) *δ* 156.0, 155.0, 154.7, 144.4, 134.5, 130.1, 128.7, 128.3 124.6, 120.5, 118.7, 117.1, 115.7, 111.8, 103.0, 79.8, 63.4, 53.1, 44.2, 28.7.

### 7) Synthesis of Compound 31 (a to z, and A to C)

Acyl chloride (1.5 equivalent) and anhydrous pyridine (5 equivalent) were added to anhydrous methylene chloride solution containing Compound **30** (1.0 equivalent), followed by stirring at room temperature. Methylene chloride and sodium bicarbonate water were added to the reaction mixture, and the organic layer was dried over anhydrous MgSO₄, filtered, and the residue obtained by concentrating the filtrate under reduced pressure was purified by silica gel column chromatography (methylene chloride:methanol = 40:1 to 80:1) to obtain Compound **31** (a to z).

Synthesis of Compound 31 (A to C): Isocyanate (1.5 equivalent) was added to anhydrous chloroform (2.0 mL) solution containing Compound **30** (100 mg), followed by stirring at 50 °C for 2 to 7 hours. A solid residue was obtained by concentrating under reduced pressure. For Compounds 31A and 31B, the obtained solid residue was filtered and washed with methanol and methylene chloride to obtain a pure solid. For Compound **31C**, the obtained residue was purified by silica gel column chromatography (methylene chloride:methanol = 30:1).

### [tert-Butyl 4-((2-(2-benzamidophenyl)benzofuran-6-yl)methyl)piperazine-1-carboxylate] (31a)

Solid; reaction time, 1 hour; yield, 67.1%; ¹H NMR (500 MHz, CDCl₃) *δ* 9.74 (s, 1 H), 8.64 (d, 1 H, *J* = 8.5 Hz), 7.98 (d, 2 H, *J* = 7.5 Hz), 7.72 (dd, 1 H, *J* = 7.5, 1.0 Hz), 7.58 (t, 1 H, *J* = 7.0 Hz), 7.56 (d, 1 H, *J* = 8.0 Hz), 7.53 (s, 1 H), 7.52 (t, 2 H, *J* = 7.5 Hz), 7.46 (td, 1 H, *J* = 8.0, 1.0 Hz), 7.26 (d, 1 H, *J* = 8.0 Hz), 7.23 (td, 1 H, *J* = 8.0, 1.0 Hz), 6.99 (s, 1 H), 3.65 (s, 2 H), 3.45 (brt, 4 H, *J* = 4.5 Hz), 2.44 (brt, 4 H, *J* = 4.5 Hz), 1.46 (s, 9 H); ¹³C NMR (100 MHz, CDCl₃) *δ* 165.5, 155.2, 155.1, 155.0, 135.8, 135.7, 135.4, 132.2, 130.3, 129.1, 128.7, 127.7, 127.3, 125.1, 124.6, 122.3, 121.1, 120.1, 111.4, 104.9, 79.9, 63.3, 53.1, 43.7, 28.7.

### [tert-Butyl 4-((2-(2-(2-methylbenzamido)phenyl)benzofuran-6-yl)methyl)piperazine-1-carboxylate] (31b)

Solid; reaction time, 3 hours; yield, 50.4%; ¹H NMR (500 MHz, CDCl₃) *δ* 9.16 (s, 1 H), 8.59 (brd, 1 H, *J* = 8.0 Hz), 7.70 (d, 1 H, *J* = 7.5 Hz), 7.60 (d, 1 H, *J* = 7.5 Hz), 7.53 (d, 1 H, *J* = 7.5 Hz), 7.45 (t, 1 H, *J* = 8.0 Hz), 7.39 (s, 1 H), 7.39 (t, 1 H, *J* = 7.5 Hz), 7.26 - 7.29 (m, 2 H), 7.22 - 7.24 (m, 2 H), 6.95 (s, 1 H), 3.61 (s, 2 H), 3.43 (brt, 4 H, *J* = 4.5 Hz), 2.54 (s, 3 H), 2.41 (brt, 4 H, *J =* 4.5 Hz), 1.45 (s, 9 H); ¹³C NMR (100 MHz, CDCl₃) *δ* 168.2, 155.0, 155.0, 154.9, 137.4, 136.6, 135.6, 135.4, 131.8, 130.7, 130.2, 128.8, 127.7, 126.8, 126.1, 125.0, 124.8, 122.5, 121.0, 120.4, 111.5, 104.9, 79.9, 63.2, 53.0, 43.5, 28.7, 20.3.

### [tert-Butyl 4-((2-(2-(3-methylbenzamido)phenyl)benzofuran-6-yl)methyl)piperazine-1-carboxylate] (31c)

Solid; reaction time, 3 hours; yield, 93.0%; ¹H NMR (400 MHz, CDCl₃) *δ* 9.79 (s, 1 H), 8.66 (d, 1 H, *J* = 8.4 Hz), 7.77 - 7.79 (m, 2 H), 7.70 (dd, 1 H, *J* = 7.6, 1.2 Hz), 7.55 (d, 1 H, *J* = 8.0 Hz), 7.54 (s, 1 H), 7.45 (td, 1 H, *J* = 8.0, 1.2 Hz), 7.38 - 7.40 (m, 2 H), 7.26 (d, 1 H, *J* = 8.0 Hz), 7.21 (td, 1 H, *J* = 8.0, 1.2 Hz), 6.99 (s, 1 H), 3.63 (s, 2 H), 3.44 (brt, 4 H, *J* = 4.4 Hz), 2.46 (s, 3 H), 2.43 (brt, 4 H, *J* = 4.4 Hz), 1.46 (s, 9 H); ¹³C NMR (100 MHz, CDCl₃) *δ* 165.6, 155.3, 155.1, 155.0, 138.8, 135.8, 135.3, 133.0, 130.3, 129.0, 128.6, 127.9, 127.7, 125.1, 124.5, 122.3, 121.1, 120.0, 111.4, 104.9, 78.9, 63.4, 53.1, 43.4, 28.7, 21.8.

### [tert-Butyl 4-((2-(2-(4-methylbenzamido)phenyl)benzofuran-6-yl)methyl)piperazine-1-carboxylate] (31d)

Yellow solid; reaction time, 3 hours; yield, 100%; ¹H NMR (400 MHz, CDCl₃) *δ* 9.66 (s, 1 H), 8.60 (d, 1 H, *J* = 8.0 Hz), 7.87 (d, 2 H, *J* = 8.0 Hz), 7.70 (dd, 1 H, *J* = 8.0, 1.2 Hz), 7.59 (s, 1 H), 7.56 (d, 1 H, *J* = 8.0 Hz), 7.44 (td, 1 H, *J* = 8.0, 1.2 Hz), 7.31 (d, 2 H, *J* = 8.0 Hz), 7.27 (dd, 1 H, *J* = 8.0, 0.8 Hz), 7.20 (td, 1 H, *J* = 8.0, 1.2 Hz), 6.97 (d, 1 H, *J* = 0.8 Hz), 3.77 (s, 2 H), 3.51 (brt, 4 H, *J* = 4.4 Hz), 2.53 (brt, 4 H, *J* = 4.4 Hz), 2.43 (s, 3 H), 1.45 (s, 9 H); ¹³C NMR (100 MHz, CDCl₃) *δ* 165.5, 155.4, 155.0, 154.9, 142.8, 135.8, 132.4, 130.3, 129.8, 128.7, 128.1, 127.3, 125.3, 124.5, 122.4, 121.2, 120.1, 111.8, 104.9, 80.1, 62.9, 52.8, 42.9, 28.6, 21.8.

### [tert-Butyl 4-((2-(2-(2-fluorobenzamido)phenyl)benzofuran-6-yl)methyl)piperazine-1-carboxylatel (31e)

Solid; reaction time, 3 hours; yield, 84.5%; ¹H NMR (500 MHz, CDCl₃) *δ* 9.82 (s, 1 H), 8.63 (d, 1 H, *J* = 8.0 Hz), 7.77 (d, 1 H, *J* = 7.5 Hz), 7.70 - 7.73 (m, 2 H), 7.57 (d, 1 H, *J* = 8.0 Hz), 7.56 (s, 1 H), 7.44 - 7.52 (m, 2 H), 7.22 - 7.30 (m, 3 H), 7.01 (s, 1 H), 3.66 (s, 2 H), 3.46 (brt, 4 H, *J* = 4.5 Hz), 2.45 (brt, 4 H, *J* = 4.5 Hz), 1.46 (s, 9 H); ¹³C NMR (100 MHz, CDCl₃) *δ* 164.01, 163.1 (d, *J =* 246.6 Hz), 155.2, 155.1, 155.0, 137.6 (d, *J* = 6.8 Hz), 135.3, 130.8 (d, *J* = 7.6 Hz), 130.3, 128.6, 127.6, 125.2, 124.8, 123.0, 123.0, 122.3, 121.2, 120.0, 119.2 (d, *J* = 21.2 Hz), 114.5 (d, *J* = 22.8 Hz), 111.4, 105.0, 79.9, 63.3, 53.1, 44.2, 28.7.

### [tert-Butyl 4-((2-(2-(3-fluorobenzamido)phenyl)benzofuran-6-yl)methyl)piperazine-1-carboxylate] (31f)

Solid; reaction time, 3 hours; yield, 95.3%; ¹H NMR (400 MHz, CDCl₃) *δ* 9.96 (brs, 1 H), 8.55 (d, 1 H, *J* = 8.4 Hz), 8.21 (td, 1 H, *J* = 7.6, 2.0 Hz), 7.71 (dd, 1 H, *J* = 7.6, 2.0 Hz), 7.50 - 7.56 (m, 3 H), 7.46 (td, 1 H, *J* = 8.0, 2.0 Hz), 7.32 (td, 1 H, *J* = 8.0, 1.2 Hz), 7.22 - 7.26 (m, 2 H), 7.17 (ddd, 1 H, *J* = 12.4, 8.4, 1.2 Hz), 6.97 (s, 1 H), 3.67 (s, 2 H), 3.46 (brt, 4 H, *J* = 4.4 Hz), 2.45 (brt, 4 H, *J* = 4.4 Hz), 1.46 (s, 9 H); ¹³C NMR (100 MHz, CDCl₃) *δ* 161.8, 160.6 (d, *J* = 246.5 Hz), 155.3, 155.0, 154.7, 138.2 (d, *J* = 7.6 Hz), 135.3, 134.1 (d, *J=* 9.1 Hz), 132.7, 130.0, 129.0, 127.9, 125.3, 125.1 (d, *J* = 21.3 Hz), 124.9, 123.6, 121.7, 121.3, 121.0, 116.4 (d, *J* = 25.0 Hz), 111.9, 104.9, 79.9, 63.3, 53.1, 43.6, 28.7.

### [tert-Butyl 4-((2-(2-(2-bromobenzamido)phenyl)benzofuran-6-yl)methyl)piperazine-1-carboxylate] (31g)

White solid; reaction time, 3 hours; yield, 69.0%; ¹H NMR (500 MHz, CDCl₃) <59.14 (s, 1 H), 8.58 (d, 1 H, *J* = 8.0 Hz), 7.64 - 7.71 (m, 3 H), 7.52 (d, 1 H, *J* = 7.5 Hz), 7.47 (t, 1 H, *J* = 7.5 Hz), 7.42 (t, 1 H, *J* = 7.5 Hz), 7.40 (s, 1 H), 7.34 (t, 1 H, *J* = 7.5 Hz), 7.25 (t, 1 H, *J* = 8.0 Hz), 7.22 (d, 1 H, *J* = 8.0 Hz), 6.97 (s, 1 H), 3.64 (s, 2 H), 3.44 (brt, 4 H, *J* = 4.5 Hz), 2.43 (brt, 4 H, *J* = 4.5 Hz), 1.46 (s, 9 H); ¹³C NMR (100 MHz, CDCl₃) *δ* 165.9, 155.1, 155.0, 154.6, 138.4, 135.5, 135.0, 134.0, 131.9, 130.2, 129.8, 128.9, 127.9, 127.7, 125.1, 124.9, 122.8, 121.0, 120.8, 119.6, 111.7, 105.1, 79.9, 63.2, 53.0, 43.3, 28.7.

### [tert-Butyl 4-((2-(2-(3-bromobenzamido)phenyl)benzofuran-6-yl)methyl)piperazine-1-carboxylate] (31h)

Solid; reaction time, 3 hours; yield, 69.0%; ¹H NMR (500 MHz, CDCl₃) *δ* 9.89 (s, 1 H), 8.64 (d, 1 H, *J* = 8.5 Hz), 8.14 (t, 1 H, *J* = 1.5 Hz), 7.95 (dt, 1 H, *J* = 8.0, 1.5 Hz), 7.71 (dd, 1 H, *J* = 8.0, 1.5 Hz), 7.70 (dt, 1 H, *J* = 8.0, 1.5 Hz), 7.67 (s, 1 H), 7.56 (d, 1 H, *J* = 8.5 Hz), 7.44 (td, 1 H, *J* = 8.0, 1.5 Hz), 7.40 (t, 1 H, *J* = 7.5 Hz), 7.28 (d, 1 H, *J* = 8.5 Hz), 7.22 (td, 1 H, *J* = 7.5, 1.0 Hz), 7.01 (s, 1 H), 3.64 (s, 2 H), 3.44 (brt, 4 H, *J* = 4.5 Hz), 2.43 (brt, 4 H, *J* = 4.5 Hz), 1.46 (s, 9 H); ¹³C NMR (100 MHz, CDCl₃) *δ* 163.8, 155.2, 155.0, 155.0, 137.4, 135.9, 135.3, 135.1, 130.8, 130.3, 129.9, 128.5, 127.5, 126.5, 125.3, 124.8, 123.1, 122.2, 121.1, 119.9, 111.8, 105.0, 79.9, 63.4, 53.1, 43.3, 28.7.

### [tert-Butyl 4-((2-(2-(4-bromobenzamido)phenyl)benzofuran-6-yl)methyl)piperazine-1-carboxylate] (31i)

Solid; Reaction time, 3 hours; yield, 48.3%; ¹H NMR (400 MHz, CDCl₃) *δ* 9.74 (s, 1 H), 8.58 (d, 1 H, *J* = 8.0 Hz), 7.84 (d, 2 H, *J* = 8.8 Hz), 7.71 (dd, 1 H, *J* = 8.0, 1.2 Hz), 7.65 (d, 2 H, *J* = 8.8 Hz), 7.56 (d, 1 H, *J* = 8.0 Hz), 7.52 (s, 1 H), 7.45 (td, 1 H, *J* = 8.0, 1.2 Hz), 7.26 (dd, 1 H, *J* = 8.0, 1.2 Hz), 7.28 (td, 1 H, *J* = 8.0, 1.2 Hz), 6.99 (d, 1 H, *J* = 0.8 Hz), 3.67 (s, 2 H), 3.47 (brt, 4 H, *J* = 4.4 Hz), 2.45 (brt, 4 H, *J* = 4.4 Hz), 1.45 (s, 9 H); ¹³C NMR (100 MHz, CDCl₃) *δ* 164.5, 155.1, 155.1, 155.0, 135.9, 135.4, 134.2, 132.3, 130.3, 128.9, 128.6, 127.7, 127.0, 125.2, 124.8, 122.3, 121.2, 120.1, 111.3, 105.0, 79.9, 63.2, 53.1, 43.6, 28.7.

### [tert-Butyl 4-((2-(2-(2-chlorobenzamido)phenyl)benzofuran-6-yl)methyl)piperazine-1-carboxylate] (31j)

White solid; reaction time, 3 hours; yield, 37.3%; ¹H NMR (400 MHz, CDCl₃) *δ* 9.36 (s, 1 H), 8.54 (d, 1 H, *J* = 8.0 Hz), 7.75 (d, 1 H, *J* = 8.0 Hz), 7.67 (d, 1 H, *J* = 7.6 Hz), 7.50 (d, 1 H, *J* = 8.0 Hz), 7.35 - 7.44 (m, 5 H), 7.20 - 7.26 (m, 2 H), 6.95 (s, 1 H), 3.60 (s, 2 H), 3.42 (brt, 4 H, *J* = 4.4 Hz), 2.39 (brt, 4 H, *J* = 4.4 Hz), 1.45 (s, 9 H); ¹³C NMR (100 MHz, CDCl₃) *δ* 164.9, 155.1, 155.0, 154.6, 135.8, 135.5, 135.1, 132.0, 131.0, 130.7, 130.4, 130.1, 128.9, 127.7, 127.4, 125.1, 124.9, 123.0, 121.0, 111.7, 105.1, 79.8, 63.2, 53.0, 43.7, 28.7.

### [tert-Butyl 4-((2-(2-(3-chlorobenzamido)phenyl)benzofuran-6-yl)methyl)piperazine-1-carboxylate] (31k)

Solid; reaction time, 3 hours; yield, 74.6%; ¹H NMR (500 MHz, CDCl₃) *δ* 9.90 (s, 1 H), 8.66 (d, 1 H, *J* = 8.0 Hz), 8.00 (s, 1 H), 7.90 (d, 1 H, *J* = 7.5 Hz), 7.72 (d, 1 H, *J* = 7.5 Hz), 7.65 (s, 1 H), 7.57 (d, 1 H, *J* = 7.5 Hz), 7.56 (d, 1 H, *J* = 7.5 Hz), 7.44 - 7.49 (m, 2 H), 7.29 (d, 1 H, *J* = 8.0 Hz), 7.23 (t, 1 H, *J* = 8.0 Hz), 7.02 (s, 1 H), 3.66 (s, 2 H), 3.45 (brt, 4 H, *J* = 4.5 Hz), 2.44 (brt, 4 H, *J* = 4.5 Hz), 1.46 (s, 9 H); ¹³C NMR (100 MHz, CDCl₃) *δ* 164.0, 155.3, 155.0, 155.0, 137.1, 135.3, 135.1, 132.2, 130.5, 130.3, 128.5, 127.6, 127.1, 126.0, 125.3, 124.8, 122.2, 121.1, 119.9, 111.7, 105.0, 79.9, 63.3, 53.1, 43.7, 28.7.

### [tert-Butyl 4-((2-(2-(4-chlorobenzamido)phenyl)benzofuran-6-yl)methyl)piperazine-1-carboxylate] (31l)

Solid; reaction time, 5 hours; yield 82%; It was used for the next reaction immediately after synthesis without NMR measurement.

### [tert-Butyl 4-((2-(2-(3-nitrobenzamido)phenyl)benzofuran-6-yl)methyl)piperazine-1-carboxylate (31m)

Solid; reaction time, 3 hours; yield, 100%; ¹H NMR (400 MHz, CDCl₃) *δ* 10.02 (s, 1 H), 8.83 (s, 1 H, *J* = 2.0 Hz), 8.64 (d, 1 H, *J* = 8.4 Hz), 8.44 (m, 1 H), 8.40 (td, 1 H, *J* = 8.0, 2.0 Hz), 7.74 (t, 1 H, *J* = 8.0 Hz), 7.73 (d, 1 H, *J* = 8.0 Hz), 7.67 (s, 1 H), 7.57 (d, 1 H, *J* = 8.0 Hz), 7.47 (td, 1 H, *J* = 8.0, 1.2 Hz), 7.29 (d, 1 H, *J* = 8.0 Hz), 7.26 (td, 1 H, *J* = 8.0, 1.2 Hz), 7.04 (s, 1 H), 3.67 (s, 2 H), 3.44 (brt, 4 H, *J* = 4.4 Hz), 2.44 (brt, 4 H, *J* = 4.4 Hz), 1.45 (s, 9 H); ¹³C NMR (100 MHz, CDCl₃) *δ* 162.8, 155.1, 155.1, 155.0, 148.6, 136.8, 134.9, 134.2, 130.5, 130.3, 128.7, 127.4, 126.8, 125.4, 125.2, 122.4, 121.3, 121.1, 120.2, 111.9, 105.3, 79.8, 63.3, 53.1, 43.4, 28.7.

### [tert-Butyl 4-((2-(2-(4-nitrobenzamido)phenyl)benzofuran-6-yl)methyl)piperazine-1-carboxylate] (31n)

White solid; reaction time, 3 hours; yield, 73.2%; ¹H NMR (500 MHz, CDCl₃) *δ* 9.86 (s, 1 H), 8.59 (d, 1 H, *J* = 7.5 Hz), 8.37 (d, 2 H, *J* = 8.5 Hz), 8.14 (d, 2 H, *J* = 8.5 Hz), 7.73 (d, 1 H, *J* = 8.0 Hz), 7.57 (d, 1 H, *J* = 8.0 Hz), 7.52 (s, 1 H), 7.47 (t, 1 H, *J* = 8.0 Hz), 7.25 - 7.29 (m, 2 H), 7.02 (s, 1 H), 3.67 (s, 2 H), 3.46 (brt, 4 H, *J* = 4.5 Hz), 2.45 (brt, 4 H, *J* = 4.5 Hz), 1.46 (s, 9 H); ¹³C NMR (100 MHz, CDCl₃) *δ* 163.3, 155.1, 155.0, 154.9, 150.0, 140.9, 136.2, 134.9, 130.3, 128.7, 128.5, 127.6, 125.4, 124.3, 122.4, 121.3, 120.3, 111.1, 105.2, 79.9, 63.2, 53.1, 43.4, 28.6.

### [tert-Butyl 4-((2-(2-(3,4,5-trimethoxybenzamido)phenyl)benzofuran-6-yl)methyl)piperaz-ine-1-carboxylate] (31o)

Yellow solid; reaction time, 3 hours; yield, 72.5%; ¹H NMR (500 MHz, CDCl₃) *δ* 9.42 (s, 1 H), 8.59 (d, 1 H, *J* = 8.0 Hz), 7.70 (d, 1 H, *J* = 8.0 Hz), 7.55 (d, 1 H, *J* = 8.0 Hz), 7.50 (s, 1 H), 7.47 (t, 1 H, *J* = 8.0 Hz), 7.27 (d, 1 H, *J* = 8.0 Hz), 7.23 (t, 1 H, *J* = 8.0 Hz), 7.15 (s, 2 H), 7.00 (s, 1 H), 3.91 (s, 3 H), 3.88 (s, 6 H), 3.62 (s, 2 H), 3.47 (brt, 4 H, *J* = 4.5 Hz), 2.42 (brt, 4 H, *J* = 4.5 Hz), 1.46 (s, 9 H); ¹³C NMR (100 MHz, CDCl₃) *δ* 165.3, 155.2, 155.0, 155.0, 153.6, 141.7, 135.8, 135.7, 130.7, 130.3, 128.9, 127.7, 125.1, 124.7, 122.3, 121.1, 120.4, 111.5, 105.1, 104.9, 79.9, 63.2, 61.2, 56.7, 53.1, 44.2, 28.7.

### [tert-Butyl 4-((2-(2-(2,4-dichlorobenzamido)phenyl)benzofuran-6-yl)methyl)piperazine-1-carboxylate] (31p)

Solid; reaction time, 3 hours; yield, 45.6%; ¹H NMR (400 MHz, CDCl₃) *δ* 9.39 (s, 1 H), 8.55 (d, 1 H, *J* = 8.0 Hz), 7.74 (d, 1 H, *J* = 8.4 Hz), 7.69 (dd, 1 H, *J* = 8.0, 1.2 Hz), 7.53 (d, 1 H, *J* = 8.0 Hz), 7.48 (d, 1 H, *J* = 1.2 Hz), 7.47 (td, 1 H, *J* = 8.0, 1.2 Hz), 7.42 (s, 1 H), 7.37 (dd, 1 H, *J* = 8.0, 1.2 Hz), 7.26 (td, 1 H, *J* = 8.0, 1.2 Hz), 7.23 (d, 1 H, *J* = 8.0 Hz), 6.96 (s, 1 H), 3.63 (s, 2 H), 3.44 (brt, 4 H, *J* = 4.4 Hz), 2.42 (brt, 4 H, *J* = 4.4 Hz), 1.46 (s, 9 H); ¹³C NMR (100 MHz, CDCl₃) *δ* 163.9, 155.2, 155.0, 154.6, 137.5, 135.7, 134.9, 134.1, 131.9, 131.6, 130.6, 130.2, 129.0, 127.9, 127.7, 125.3, 125.0, 122.8, 121.1, 120.9, 111.6, 105.2, 79.9, 63.2, 53.1, 43.6, 28.7.

### [tert-Butyl 4-((2-(2-(3,4-dichlorobenzamido)phenyl)benzofuran-6-yl)methyl)piperazine-1-carboxylate] (31q)

Yellow solid; reaction time, 3 hours; yield, 87.0%; ¹H NMR (500 MHz, CDCl₃) *δ* 9.88 (s, 1 H), 8.61 (d, 1 H, *J* = 8.5 Hz), 8.09 (d, 1 H, *J* = 2.5 Hz), 7.85 (dd, 1 H, *J* = 8.0, 2.0 Hz), 7.72 (dd, 1 H, *J* = 7.5, 1.5 Hz), 7.63 (s, 1 H), 7.61 (d, 1 H, *J* = 8.5 Hz), 7.57 (d, 1 H, *J* = 8.0 Hz), 7.45 (td, 1 H, *J* = 8.0, 1.5 Hz), 7.29 (dd, 1 H, *J* = 8.0, 1.0 Hz), 7.23 (td, 1 H, *J* = 7.5, 1.0 Hz), 7.02 (s, 1 H), 3.65 (s, 2 H), 3.46 (brt, 4 H, *J* = 4.5 Hz), 2.44 (brt, 4 H, *J* = 4.5 Hz), 1.46 (s, 9 H); ¹³C NMR (100 MHz, CDCl₃) *δ* 163.1, 155.2, 155.0, 155.0, 136.7, 136.1, 135.2, 135.1, 133.4, 131.3, 130.3, 128.9, 128.5, 127.5, 127.1, 125.3, 125.0, 122.2, 121.2, 119.9, 111.5, 105.1, 79.9, 63.3, 53.2, 43.5, 28.7.

### [tert-Butyl 4-((2-(2-(nicotinamido)phenyl)benzofuran-6-yl)methyl)piperazine-1-carboxylate] (31r)

Yellow solid; reaction time, 3 hours; yield, 79.5%; ¹H NMR (500 MHz, CDCl₃) *δ* 9.80 (s, 1 H), 9.22 (m, 1 H), 8.80 (dd, 1 H, *J* = 4.5, 1.5 Hz), 8.60 (d, 1 H, *J* = 8.0 Hz), 8.30 (td, 1 H, *J* = 8.0, 2.0 Hz), 7.73 (dd, 1 H, *J* = 8.0, 1.5 Hz), 7.57 (s, 1 H), 7.56 (d, 1 H, *J* = 7.5 Hz), 7.45-7.48 (m, 2 H), 7.26 (d, 1 H, *J* = 8.0 Hz), 7.25 (t, 1 H, *J* = 8.0 Hz), 7.01 (s, 1 H), 3.65 (s, 2 H), 3.45 (brt, 4 H, *J* = 4.0 Hz), 2.43 (brt, 4 H, *J* = 4.0 Hz), 1.46 (s, 9 H); ¹³C NMR (100 MHz, CDCl₃) *δ* 163.6, 155.1, 155.0, 152.8, 148.0, 135.9, 135.7, 135.1, 131.0, 130.3, 128.7, 127.6, 125.3, 125.1, 124.0, 122.6, 121.2, 120.4, 111.5, 105.2, 79.9, 63.2, 53.1, 44.3, 28.7.

### [tert-Butyl 4-((2-(2-(isonicotinamido)phenyl)benzofuran-6-yl)methyl)piperazine-1-carboxylate] (31s)

White solid; reaction time, 3 hours; yield, 62.8%; ¹H NMR (500 MHz, CDCl₃) <59.86 (s, 1 H), 8.84 (d, 2 H, *J* = 4.5 Hz), 8.61 (d, 1 H, *J* = 7.5 Hz), 7.81 (d, 2 H, *J* = 4.5 Hz), 7.73 (d, 1 H, *J* = 7.5 Hz), 7.58 (d, 1 H, *J* = 8.0 Hz), 7.52 (s, 1 H), 7.47 (t, 1 H, *J* = 8.0 Hz), 7.25 - 7.29 (m, 2 H), 7.02 (s, 1 H), 3.66 (s, 2 H), 3.46 (brt, 4 H, *J* = 4.0 Hz), 2.44 (brt, 4 H, *J* = 4.0 Hz), 1.46 (s, 9 H); ¹³C NMR (100 MHz, CDCl₃) *δ* 163.5, 155.1, 155.1, 154.9, 151.1, 142.4, 134.9, 130.3, 130.3, 128.7, 127.7, 125.4, 125.3, 122.4, 121.3, 121.1, 120.2, 111.4, 105.2, 80.0, 63.2, 53.1, 44.3, 28.7.

### [tert-Butyl 4-((2-(2-(1-naphthamido)phenyl)benzofuran-6-yl)methyl)piperazine-1-carboxyate] (31t)

Solid; reaction time, 1 hour; yield, 50.8%; ¹H NMR (500 MHz, CDCl₃) *δ* 9.42 (s, 1 H), 8.73 (d, 1 H, *J* = 8.0 Hz), 8.48 (d, 1 H, *J* = 7.5 Hz), 8.00 (d, 1 H, *J* = 8.5 Hz), 7.92 (d, 1 H, *J* = 8.5 Hz), 7.86 (d, 1 H, *J* = 7.0 Hz), 7.72 (d, 1 H, *J* = 8.0 Hz), 7.49 - 7.56 (m, 5 H), 7.26 (t, 1 H, *J* = 8.0 Hz), 7.20 (d, 1 H, *J* = 8.5 Hz), 7.17 (s, 1 H), 6.96 (s, 1 H), 3.56 (s, 2 H), 3.42 (brt, 4 H, *J =* 4.5 Hz), 2.37 (brt, 4 H, *J =* 4.5 Hz), 1.47 (s, 9 H); ¹³C NMR (100 MHz, CDCl₃) *δ* 167.7, 155.0, 155.0, 154.9, 135.6, 134.9, 134.1, 131.6, 130.4, 130.3, 128.8, 128.7, 127.6, 127.5, 126.8, 125.6, 125.6, 125.0, 125.0, 124.9, 122.6, 121.0, 120.5, 111.6, 105.0, 79.9, 63.2, 53.0, 44.2, 28.7.

### [tert-Butyl 4-((2-(2-(2-naphthamido)phenyl)benzofuran-6-yl)methyl)piperazine-1-carboxyate] (31u)

Solid; reaction time, 1 hour; yield, 72.6%; ¹H NMR (400 MHz, CDCl₃) *δ* 9.97 (s, 1 H), 8.70 (d, 1 H, *J* = 8.4 Hz), 8.47 (brs, 1 H), 8.05 (dd, 1 H, *J* = 8.4, 2.0 Hz), 7.97 (d, 1 H, *J* = 8.4 Hz), 7.96 (d, 1 H, *J* = 8.0 Hz), 7.92 (d, 1 H, *J* = 8.0 Hz), 7.74 (dd, 1 H, *J* = 8.0, 1.6 Hz), 7.56 - 7.63 (m, 4 H), 7.48 (td, 1 H, *J* = 8.0, 1.6 Hz), 7.22 - 7.30 (m, 2 H), 7.02 (s, 1 H), 3.65 (s, 2 H), 3.43 (brt, 4 H, *J* = 4.4 Hz), 2.44 (brt, 4 H, *J* = 4.4 Hz), 1.46 (s, 9 H); ¹³C NMR (100 MHz, CDCl₃) *δ* 165.6, 155.3, 155.1, 155.0, 136.3, 135.7, 135.2, 132.9, 132.6, 130.3, 129.2, 129.0, 128.6, 128.3, 128.1, 127.8, 127.7, 127.2, 125.2, 124.6, 123.9, 122.4, 121.2, 120.1, 111.4, 105.0, 79.9, 63.3, 53.1, 44.3, 28.7.

### [tert-Butyl 4-((2-(2-(quinoline-2-carboxamido)phenyl)benzofuran-6-yl)methyl)piperazine-1-carboxylatel (31v)

Solid; reaction time, 1 hour; yield, 99.2%; ¹H NMR (400 MHz, CDCl₃) *δ* 11.48 (s, 1 H), 8.75 (d, 1 H, *J* = 8.0 Hz), 8.43 (d, 1 H, *J* = 8.4 Hz), 8.39 (t, 1 H, *J* = 8.4 Hz), 8.04 (d, 1 H, *J* = 8.4 Hz), 7.92 (d, 1 H, *J* = 8.0 Hz), 7.83 (d, 1 H, *J* = 8.0 Hz), 7.76 (t, 1 H, *J* = 8.4 Hz), 7.66 (d, 1 H, *J* = 8.0 Hz), 7.65 (s, 1 H), 7.62 (d, 1 H, *J* = 8.0 Hz), 7.49 (t, 1 H, *J* = 8.0 Hz), 7.31 (d, 1 H, *J* = 8.0 Hz), 7.26 (t, 1 H, *J* = 7.6 Hz), 7.21 (s, 1 H), 3.68 (s, 2 H), 3.44 (brt, 4 H, *J* = 4.4 Hz), 2.46 (brt, 4 H, *J* = 4.4 Hz), 1.46 (s, 9 H); ¹³C NMR (100 MHz, CDCl₃) *δ* 162.6, 155.3, 155.0, 154.2, 150.2, 146.6, 138.1, 135.4, 135.3, 130.5, 130.1, 129.9, 129.7, 128.8, 128.4, 128.3, 128.1, 124.8, 124.6, 121.8, 121.0, 120.9, 119.2, 112.1, 105.4, 79.8, 63.5, 53.2, 43.4, 28.7.

### [tert-Butyl 4-((2-(2-(quinoxaline-2-carboxamido)phenyl)benzofuran-6-yl)methyl)piperazine-1-carboxylate] (31w)

Solid; reaction time, 1 hour; yield, 78.1%; ¹H NMR (400 MHz, CDCl₃) *δ* 11.30 (s, 1 H), 9.80 (s, 1 H), 8.74 (dd, 1 H, *J* = 8.4, 1.2 Hz), 8.23 (dd, 1 H, *J* = 8.0, 1.2 Hz), 8.08 (dd, 1 H, *J* = 8.0, 1.2 Hz), 7.91 (td, 1 H, *J* = 7.6, 1.6 Hz), 7.85 (td, 1 H, *J* = 7.6, 1.6 Hz), 7.80 (dd, 1 H, *J* = 8.0, 1.6 Hz), 7.66 (s, 1 H), 7.61 (d, 1 H, *J* = 7.6 Hz), 7.50 (td, 1 H, *J* = 8.0, 1.2 Hz), 7.32 (dd, 1 H, *J* = 8.0, 1.2 Hz), 7.28 (td, 1 H, *J* = 7.6, 1.2 Hz), 7.13 (d, 1 H, *J* = 1.2 Hz), 3.69 (s, 2 H), 3.45 (brt, 4 H, *J* = 4.4 Hz), 2.47 (brt, 4 H, *J* = 4.4 Hz), 1.46 (s, 9 H); ¹³C NMR (100 MHz, CDCl₃) *δ* 161.4, 155.4, 155.0, 154.4, 144.3, 144.3, 144.0, 140.4, 135.5, 134.9, 132.1, 131.2, 130.2, 129.9, 129.8, 128.8, 128.1, 125.0, 125.0, 122.0, 121.1, 120.9, 112.0, 105.3, 79.9, 63.5, 53.2, 43.7, 28.7.

### [tert-Butyl 4-((2-(2-([1,1'-biphenyl]-4-ylcarboxamido)phenyl)benzofuran-6-yl)methyl)piperazine-1-carboxylate] (31x)

Solid; reaction time, 1 hour; yield, 74.0%; ¹H NMR (400 MHz, CDCl₃) *δ* 9.80 (s, 1 H), 8.67 (d, 1 H, *J* = 8.4 Hz), 8.06 (d, 2 H, *J* = 8.4 Hz), 7.75 (d, 2 H, *J* = 8.4 Hz), 7.73 (dd, 1 H, *J* = 7.6, 1.6 Hz), 7.64 (d, 2 H, *J* = 7.6 Hz), 7.57 (s, 1 H), 7.57 (d, 1 H, *J* = 7.6 Hz), 7.41 (m, 1 H), 7.45 - 7.51 (m, 3 H), 7.26 (dd, 1 H, *J* = 8.0, 0.8 Hz), 7.23 (td, 1 H, *J* = 7.6, 1.2 Hz), 7.02 (d, 1 H, *J* = 0.8 Hz), 3.65 (s, 2 H), 3.43 (brt, 4 H, *J* = 4.4 Hz), 2.43 (brt, 4 H, *J* = 4.4 Hz), 1.44 (s, 9 H); ¹³C NMR (100 MHz, CDCl₃) *δ* 165.2, 155.2, 155.1, 154.9, 145.1, 140.1, 135.7, 134.0, 130.3, 129.3, 128.6, 128.4, 127.9, 127.7, 127.4, 125.1, 124.6, 122.3, 121.1, 120.1, 111.4, 104.9, 79.9, 63.3, 53.1, 43.3, 28.7.

### [(E)-tert-Butyl 4-((2-(2-cinnamamidophenyl)benzofuran-6-yl)methyl)piperazine-1-carboxyate] (31y)

Solid; reaction time, 1 hour; yield, 83.4%; ¹H NMR (400 MHz, CDCl₃) *δ* 8.61 (d, 1 H, *J* = 8.0 Hz), 7.76 (d, 1 H, *J* = 15.6 Hz), 7.70 (dd, 1 H, *J* = 8.0, 1.2 Hz), 7.55 - 7.58 (m, 3 H), 7.43 (td, 1 H, *J* = 8.0, 1.2 Hz), 7.37 - 7.41 (m, 4 H), 7.26 (d, 1 H, *J* = 8.0 Hz), 7.22 (td, 1 H, *J* = 8.0, 1.0 Hz), 6.99 (s, 1 H), 6.56 (d, 1 H, *J* = 15.6 Hz), 3.65 (s, 2 H), 3.44 (brt, 4 H, *J* = 4.8 Hz), 2.44 (brt, 4 H, *J* = 4.8 Hz), 1.46 (s, 9 H).

### [tert-Butyl 4-((2-(2-(3r,5r,7r)-adamantane-1-carboxamido)phenyl)benzofuran-6-yl)methyl)piperazine-1-carboxylate] (31z)

Solid; reaction time, 14 hours; yield, 43.2%; ¹H NMR (500 MHz, DMSO-*d*₆) <510.95 (s, 1 H), 7.82 - 7.71 (m, 3 H), 7.55 (brd, 1 H, *J* = 7.5 Hz), 7.43 - 7.39 (m, 2 H), 7.32 (t, 1 H, *J=* 7.0 Hz), 7.20 (s, 1 H), 4.44 (brs, 2 H), 3.33 (brs, 8 H), 2.02 (s, 3 H), 1.92 (s, 6 H), 1.69 (s, 6 H), 1.38 (s, 9 H); ¹³C NMR (150 MHz, DMSO-*d*₆) *δ* 176.2, 155.7, 154.1, 153.9, 135.9, 129.8, 128.6, 127.8, 126.9, 126.2, 125.2, 121.7, 114.4, 111.5, 105.0, 80.1, 59.4, 50.6, 41.1, 40.5, 39.0, 36.5, 28.4, 28.1.

### [tert-Butyl 4-((2-(2-(3-phenylureido)phenyl)benzofuran-6-yl)methyl)piperazine-1-carboxylate] (31A)

Solid; reaction time, 4 hours; yield, 19.8%; ¹H NMR (500 MHz, DMSO-*d*₆) *δ* 9.20 (s, 1 H), 8.27 (s, 1 H), 7.92 (d, 1 H, *J* = 8.5 Hz), 7.73 (d, 1 H, *J* = 8.0 Hz), 7.63 (d, 1 H, *J* = 7.5 Hz), 7.56 (s, 1 H), 7.44 (d, 2 H, *J* = 8.5 Hz), 7.39 (t, 1 H, *J* = 7.0 Hz), 7.26 (t, 2 H, *J* = 7.0 Hz), 7.25 (s, 1 H), 7.23 (d, 1 H, *J* = 7.5 Hz), 7.19 (t, 1 H, *J* = 7.5 Hz), 6.96 (t, 1 H, *J* = 7.5 Hz), 3.60 (s, 2 H), 3.31 (brs, 4 H), 2.33 (brt, 4 H, *J* = 4.5 Hz), 1.37 (s, 9 H); ¹³C NMR (150 MHz, DMSO-*d*₆) *δ* 154.7, 154.3, 153.6, 153.1, 140.2, 136.4, 135.5, 129.7, 129.3, 128.9, 128.1, 124.8, 124.1, 124.0, 122.4, 121.9, 121.2, 118.7, 111.9, 105.6, 79.2, 62.4, 52.8, 43.8, 28.5.

### [tert-Butyl 4-((2-(2-(3-(naphthalen-1-yl)ureido)phenyl)benzofuran-6-yl)methyl)piperazine-1-carboxylate] (31B)

Solid; reaction time, 7 hours; yield, 61.2%; ¹H NMR (500 MHz, DMSO-*d*₆) *δ* 9.17 (s, 1 H), 8.66 (s, 1 H), 8.14 (d, 1 H, *J* = 8.0 Hz), 7.92 (m, 3 H), 7.76 (d, 1 H, *J* = 7.5 Hz), 7.65 (d, 1 H, *J* = 8.5 Hz), 7.63 (d, 1 H, *J* = 7.5 Hz), 7.56 (t, 1 H, *J* = 7.0 Hz), 7.54 (s, 1 H), 7.53 (d, 1 H, *J* = 7.5 Hz), 7.46 (t, 1 H), 7.41 (t, 1 H, *J* = 7.5 Hz), 7.29 (s, 1 H), 7.23 (d, 1 H, *J* = 7.0 Hz), 7.22 (t, 1 H, *J* = 7.0 Hz), 3.59 (s, 2 H), 3.30 (brs, 4 H), 2.33 (s, 4 H), 1.37 (s, 9 H); ¹³C NMR (150 MHz, DMSO-*d*₆) *δ* 154.7, 154.3, 153.8, 153.7, 136.5, 135.5, 134.7, 134.2, 129.7, 128.9, 128.1, 126.9, 126.4, 126.3, 126.1, 124.8, 124.6, 124.2, 123.8, 122.4, 122.2, 121.2, 119.0, 111.9, 105.6, 79.2, 62.4, 52.8, 43.8, 28.5.

### [tert-Butyl 4-((2-(2-(3-benzylureido)phenyl)benzofuran-6-yl)methyl)piperazine-1-carboxylate] (31C)

Solid; reaction time, 2 hours; yield, 71.6%; ¹H NMR (500 MHz, DMSO-*d*₆) *δ* 8.11 (s, 1 H), 7.91 (d, 1 H, *J* = 8.5 Hz), 7.68 (d, 1 H, *J* = 7.5 Hz), 7.59 (d, 1 H, *J* = 7.5 Hz), 7.54 (s, 1 H), 7.34 (t, 1 H, *J* = 7.5 Hz), 7.31 - 7.29 (m, 4 H), 7.27- - 7.21 (m, 3 H), 7.16 (s, 1 H), 7.13 (t, 1 H, *J* = 7.5 Hz), 4.30 (d, 2 H, *J* = 5.5 Hz), 3.59 (s, 2 H), 3.30 (brs, 4 H), 2.33 (brs, 4 H), 1.37 (s, 9 H); ¹³C NMR (150 MHz, DMSO-*d*₆) *δ* 155.9, 154.7, 154.3, 153.8, 140.6, 137.3, 135.3, 129.7, 128.8, 128.1, 127.6, 127.2, 124.7, 123.8, 123.5, 121.6, 121.1, 111.9, 105.3, 79.2, 62.5, 52.8, 43.8, 43.4, 28.5.

### 8) Synthesis of Compound 32 (a to z, and A to C)

1,4-dioxane and 8N-HCl (42.0 equivalents, using 84 equivalents for the synthesis of Compounds 31z and 32B) in volume corresponding to 8N-HCl (42.0 equivalents) in Compound **31** (1.0 equivalents) was added in order and the reaction mixture was stirred at room temperature. The solvent was removed by concentration under reduced pressure, and the remaining residue was added to diethyl ether in the presence of a small amount of methanol or in the absence of methanol, and the resulting solid was filtered to obtain Compound **32** (a to z, and A to C).

### [N-(2-(6-(Piperazin-1-ylmethyl)benzofuran-2-yl)phenyl)benzamide hydrochloride] (32a)

Light yellow solid; reaction time, 40 minutes; yield, 71.4%; ¹H NMR (500 MHz, D₂O) *δ* 7.62 - 7.67 (m, 3 H), 7.20 - 7.42 (m, 8 H), 7.03 (d, 1 H, *J* = 7.0 Hz), 6.84 (s, 1 H), 4.21 (s, 2 H), 3.27 (s, 8 H); ¹³C NMR (100 MHz, D₂O) *δ* 170.2, 155.7, 154.2, 133.4, 133.2, 132.8, 130.7, 130.5, 129.1, 128.9, 128.2, 127.7, 126.5, 126.1, 123.5, 122.3, 113.9, 104.6, 61.1, 47.8, 40.7.

### [2-Methyl-N-(2-(6-(piperazin-1-ylmethyl)benzofuran-2-yl)phenyl)benzamide hydrochloride] (32b)

White solid; reaction time, 6 hours; yield, 70.0%; ¹H NMR (400 MHz, DMSO-*d*₆) *δ* 12.27 (brs, 1 H), 9.77 (brs, 2 H), 7.96 (brs, 1 H), 7.86 (dd, 1 H, *J* = 8.0, 1.6 Hz), 7.73 (d, 1 H, *J* = 8.0 Hz), 7.57 - 7.62 (m, 2 H), 7.47 - 7.51 (m, 2 H), 7.41 (d, 1 H, *J* = 7.6 Hz), 7.36 - 7.38 (m, 2 H), 7.27 (dd, 1 H, *J* = 8.0, 2.0 Hz), 7.26 (s, 1 H), 4.50 (s, 2 H), 3.42 (brs, 8 H), 2.36 (s, 3 H); ¹³C NMR (100 MHz, DMSO-*d*₆) *δ* 168.8, 155.9, 154.3, 137.2, 136.3, 135.6, 135.6, 131.3, 130.4, 130.3, 129.2, 128.8, 127.8, 127.2, 126.6, 126.5, 122.0, 114.8, 105.2, 59.3, 47.8, 40.8, 20.1.

### [3-Methyl-N-(2-(6-(piperazin-1-ylmethyl)benzofuran-2-yl)phenyl)benzamide hydrochloride] (32c)

White solid; reaction time, 3 hours; yield, 93%; ¹H NMR (400 MHz, DMSO-*d*₆) *δ* 12.20 (brs, 1 H), 9.72 (brs, 2 H), 7.45 - 7.94 (m, 8 H), 7.20 - 7.40 (m, 4 H), 4.49 (s, 2 H), 3.40 (brs, 8 H), 2.39 (s, 3 H).

### [4-Methyl-N-(2-(6-(piperazin-1-ylmethyl)benzofuran-2-yl)phenyl)benzamide hydrochloride] (32d)

White solid; reaction time, 6 hours; yield, 23.4%; ¹H NMR (400 MHz, DMSO-*d*₆) *δ* 12.16 (brs, 1 H), 9.67 (brs, 2 H), 7.89 - 7.93 (m, 4 H), 7.67 (d, 1 H, *J* = 8.4 Hz), 7.58 (d, 1 H, *J* = 7.6 Hz), 7.44 - 7.50 (m, 2 H), 7.42 (t, 1 H, *J* = 7.6 Hz), 7.35 (d, 2 H, *J* = 8.0 Hz), 7.18 (s, 1 H), 4.46 (s, 2 H), 3.39 (brs, 8 H), 2.38 (s, 3 H); ¹³C NMR (100 MHz, DMSO-*d*₆) *δ* 166.1, 155.4, 154.1, 142.5, 135.8, 132.1, 130.3, 130.2, 129.8, 129.2, 128.6, 128.3, 127.2, 127.1, 126.6, 122.1, 114.9, 105.1, 59.3, 47.8, 40.8, 21.7.

### [2-Fluoro-N-(2-(6-(piperazin-1-ylmethyl)benzofuran-2-yl)phenyl)benzamide hydrochloride] (32e)

White solid; reaction time, 6 hours; yield, 66.1%; ¹H NMR (400 MHz, DMSO-*d*₆) *δ* 12.20 (brs, 1 H), 9.72 (brs, 2 H), 7.92 (dd, 1 H, *J* = 7.6, 1.2 Hz), 7.85 - 7.88 (m, 2 H), 7.78 (d, 1 H, *J* = 9.6 Hz), 7.68 (d, 1 H, *J* = 7.6 Hz), 7.62 (td, 1 H, *J* = 8.0, 6.0 Hz), 7.56 (d, 1 H, *J=* 7.6 Hz), 7.42 - 7.51 (m, 4 H), 7.21 (s, 1 H), 4.46 (s, 2 H), 3.39 (brs, 8 H); ¹³C NMR (100 MHz, DMSO-*d*₆) *δ* 165.0, 162.7 (d, *J* = 243.5 Hz), 155.4, 154.2, 137.2 (d, *J* = 6.8 Hz), 135.4, 131.5 (d, *J* = 7.6 Hz), 130.4, 130.2, 129.3, 128.8, 127.6, 127.1, 126.8, 124.5, 122.1, 119.4 (d, *J* = 20.5 Hz), 115.2 (d, *J* = 22.8 Hz), 114.8, 105.3, 59.3, 47.8, 40.8.

### [3-Fluoro-N-(2-(6-(piperazin-1-ylmethyl)benzofuran-2-vl)phenyl)benzamide hydrochloride] (32f)

Beige solid; reaction time, 6 hours; yield, 70.6%; ¹H NMR (400 MHz, DMSO-*d*₆) *δ* 12.26 (brs, 1 H), 9.77 (brs, 2 H), 7.96 (s, 1 H), 7.89 (d, 1 H, *J* = 8.0 Hz), 7.78 (t, 1 H, *J* = 7.2 Hz), 7.72 (d, 1 H, *J* = 8.0 Hz), 7.69 (d, 1 H, *J* = 7.6 Hz), 7.59 (m, 1 H), 7.47 - 7.52 (m, 2 H), 7.35 - 7.43 (m, 3 H), 7.31 (s, 1 H), 4.50 (s, 2 H), 3.39 (brs, 8 H); ¹³C NMR (100 MHz, DMSO-*d*₆) *δ* 163.6, 159.9 (d, *J* = 248.1 Hz), 155.4, 154.3, 135.2, 133.6 (d, *J* = 8.3 Hz), 130.9, 130.4 (d, *J* = 6.1 Hz), 129.1, 128.1, 127.2, 125.8, 125.5 (d, *J* = 3.0 Hz), 124.5 (d, *J* = 14.4 Hz), 122.1, 117.1 (d, *J* = 22.0 Hz), 115.0, 105.4, 59.2, 47.8, 40.8.

### [2-Bromo-N-(2-(6-(piperazin-1-ylmethyl)benzofuran-2-yl)phenyl)benzamide hydrochloride] (32g)

Yellow solid; reaction time, 6 hours; yield, 54.9%; ¹H NMR (400 MHz, DMSO-*d*₆) *δ* 12.34 (brs, 1 H), 9.82 (brs, 2 H), 8.03 (s, 1 H), 7.87 (d, 1 H, *J* = 7.6 Hz), 7.74 (d, 1 H, *J* = 7.6 Hz), 7.70 (d, 1 H, *J* = 8.0 Hz), 7.66 (d, 1 H, *J* = 7.6 Hz), 7.57 - 7.61 (m, 2 H), 7.48 - 7.52 (m, 2 H), 7.39 - 7.44 (m, 2 H), 7.33 (s, 1 H), 4.52 (s, 2 H), 3.48 (brs, 8 H); ¹³C NMR (100 MHz, DMSO-*d*₆) *δ* 167.0, 155.5, 154.4, 139.4, 135.1, 133.5, 131.9, 130.5, 130.3, 129.4, 129.1, 128.6, 127.5, 127.2, 126.4, 126.0, 122.1, 120.0, 115.0, 105.7, 59.2, 47.8, 40.8.

### [3-Bromo-N-(2-(6-(piperazin-1-ylmethyl)benzofuran-2-yl)phenyl)benzamide hydrochloride] (32h)

Beige solid; reaction time, 6 hours; yield, 47.0%; ¹H NMR (400 MHz, DMSO-*d*₆) *δ* 12.15 (brs, 1 H), 9.65 (brs, 2 H), 8.16 (s, 1 H), 8.99 (d, 1 H, *J* = 8.0 Hz), 7.92 (d, 1 H, *J* = 7.6 Hz), 7.87 (brs, 1 H), 7.81 (d, 1 H, *J* = 8.0 Hz), 7.69 (d, 1 H, *J* = 7.6 Hz), 7.42 - 7.57 (m, 5 H), 7.21 (s, 1 H), 4.45 (s, 2 H), 3.39 (brs, 8 H); ¹³C NMR (100 MHz, DMSO-*d*₆) *δ* 164.9, 155.4, 154.2, 137.1, 135.3, 135.2, 131.6, 131.0, 130.3, 130.2, 129.2, 128.8, 127.5, 127.5, 126.7, 122.6, 122.1, 114.7, 105.2, 59.3, 47.9, 40.7.

### [4-Bromo-N-(2-(6-(piperazin-1-ylmethyl)benzofuran-2-yl)phenyl)benzamide hydrochloride] (32i)

Yellow solid; reaction time, 6 hours; yield, 49.6%; ¹H NMR (400 MHz, DMSO-*d*₆) *δ* 12.22 (brs, 1 H), 9.69 (brs, 2 H), 7.90 - 7.95 (m, 4 H), 7.78 (d, 2 H, *J* = 8.8 Hz), 7.68 (d, 1 H, *J* = 7.6 Hz), 7.57 (d, 1 H, *J* = 8.0 Hz), 7.41 - 7.50 (m, 3 H), 7.20 (s, 1 H), 4.46 (s, 2 H), 3.39 (brs, 8 H); ¹³C NMR (100 MHz, DMSO-*d*₆) *δ* 165.5, 155.5, 154.2, 135.4, 134.1, 132.3, 130.4, 130.2, 129.2, 128.8, 127.5, 127.2, 126.6, 126.2, 122.1, 114.8, 105.2, 59.3, 47.8, 40.8.

### [2-Chloro-N-(2-(6-(piperazin-1-ylmethyl)benzofuran-2-yl)phenyl)benzamide hydrochloride (32i)

White solid; reaction time, 6 hours; yield, 96.5%; ¹H NMR (400 MHz, DMSO-*d*₆) *δ* 12.26 (brs, 1 H), 9.74 (brs, 2 H), 7.99 (brs, 1 H), 7.88 (d, 1 H, *J* = 7.6 Hz), 7.73 (d, 1 H, *J* = 8.0 Hz), 7.69 (d, 1 H, *J* = 7.2 Hz), 7.39 - 7.60 (m, 6 H), 7.43 (t, 1 H, *J* = 7.6 Hz), 7.32 (s, 1 H), 4.50 (s, 2 H), 3.42 (brs, 8 H); ¹³C NMR (100 MHz, DMSO-*d*₆) *δ* 166.1, 155.5, 154.4, 137.3, 135.0, 131.8, 130.7, 130.5, 130.3, 129.5, 129.1, 128.7, 128.1, 127.5, 127.1, 126.5, 122.0, 114.9, 105.6, 59.3, 47.8, 40.8.

### [3-Chloro-N-(2-(6-(piperazin-1-ylmethyl)benzofuran-2-yl)phenyl)benzamide hydrochloride] (32k)

Beige solid; reaction time, 6 hours; yield, 57.7%; ¹H NMR (400 MHz, DMSO-*d*₆) *δ* 12.19 (brs, 1 H), 9.75 (brs, 2 H), 8.02 (brt, 1 H, *J* = 1.6 Hz), 7.96 (d, 1 H, *J* = 7.6 Hz), 7.92 (dd, 1 H, *J* = 7.6, 1.6 Hz), 7.88 (brs, 1 H), 7.65 - 7.69 (m, 2 H), 7.60 (t, 1 H, *J* = 8.0 Hz), 7.56 (dd, 1 H, *J* = 7.6, 1.2 Hz), 7.42 - 7.51 (m, 3 H), 7.21 (s, 1 H), 4.46 (s, 2 H), 3.40 (brs, 8 H); ¹³C NMR (100 MHz, DMSO-*d*₆) *δ* 165.0, 155.4, 154.2, 136.9, 135.3, 134.1, 132.3, 131.4, 130.4, 130.2, 129.2, 128.8, 128.2, 127.6, 127.1, 126.7, 122.1, 114.8, 105.3, 59.3, 47.8, 40.7.

### [4-Chloro-N-(2-(6-(piperazin-1-ylmethyl)benzofuran-2-yl)phenyl)benzamide hydrochloride] (32l)

Yellow solid; reaction time, 6 hours; yield, 68.8%; ¹H NMR (400 MHz, DMSO-*d*₆) *δ* 12.22 (brs, 1 H), 9.78 (brs, 2 H), 8.02 (d, 2 H, *J* = 8.4 Hz), 7.92 (d, 1 H, *J* = 8.0 Hz), 7.91 (s, 1 H), 7.67 (d, 1 H, *J* = 8.0 Hz), 7.64 (d, 2 H, *J* = 8.4 Hz), 7.57 (d, 1 H, *J* = 8.0 Hz), 7.40 - 7.50 (m, 3 H), 7.21 (s, 1 H), 4.48 (s, 2 H), 3.41 (brs, 8 H); ¹³C NMR (100 MHz, DMSO-*d*₆) δ 165.4, 155.5, 154.2, 137.3, 135.4, 133.7, 130.4, 130.3, 130.2, 129.4, 129.2, 128.8, 127.5, 127.2, 126.6, 122.1, 114.8, 105.2, 59.2, 47.8, 40.8.

### [3-Nitro-N-(2-(6-(piperazin-1-ylmethyl)benzofuran-2-yl)phenyl)benzamide hydrochloride] (32m)

Beige solid; reaction time, 6 hours; yield, 52.2%; ¹H NMR (500 MHz, D₂O+DMSO-*d*₆) *δ* 8.54 (s, 1 H), 8.36 (d, 1 H, *J* = 8.0 Hz), 8.15 (d, 1 H, *J* = 7.0 Hz), 7.83 (d, 1 H, *J* = 7.0 Hz), 7.69 (t, 1 H, *J* = 7.5 Hz), 7.59 (d, 1 H, *J* = 7.5 Hz), 7.41 - 7.48 (m, 4 H), 7.22 (d, 1 H, *J* = 8.0 Hz), 7.07 (s, 1 H), 4.38 (s, 2 H), 3.45 (brs, 8 H); ¹³C NMR (100 MHz, D₂O+DMSO-*d*₆) *δ* 164.4, 155.4, 154.3, 148.5, 136.4, 135.1, 134.8, 131.1, 130.4, 130.3, 129.3, 128.9, 127.7, 127.3, 127.0, 126.7, 123.2, 122.1, 114.9, 105.3, 59.2, 47.8, 40.8.

### [4-Nitro-N-(2-(6-(piperazin-1-ylmethyl)benzofuran-2-yl)phenyl)benzamide hydrochloride] (32n)

Yellow solid; reaction time, 6 hours; yield, 90.3%; ¹H NMR (500 MHz, D₂O) *δ* 8.36 (d, 2 H, *J* = 8.0 Hz), 8.09 (d, 2 H, *J* = 8.0 Hz), 7.94 (d, 1 H, *J* = 7.0 Hz), 7.70 (d, 1 H, *J* = 8.0 Hz), 7.62 (s, 1 H), 7.48 - 7.56 (m, 3 H), 7.33 (d, 1 H, *J* = 8.5 Hz), 7.22 (s, 1 H), 4.40 (s, 2 H), 3.39 (brs, 8 H); ¹³C NMR (100 MHz, D₂O+DMSO-*d*₆) *δ* 164.9, 155.5, 154.3, 149.9, 140.7, 135.0, 130.4, 130.3, 129.9, 129.2, 128.9, 127.7, 127.3, 126.7, 126.1, 124.5, 122.1, 114.9, 105.3, 59.1, 47.8, 40.8.

### [3,4,5-Trimethoxy-N-(2-(6-(piperazin-1-ylmethyl)benzofuran-2-yl)phenyl)benzamide hydrochloride] (32o)

Yellow solid; reaction time, 6 hours; yield, 95.3%; ¹H NMR (400 MHz, DMSO-*d*₆) *δ* 12.3 (brs, 1 H), 9.95 (brs, 2 H), 7.95 (s, 1 H), 7.93 (d, 1 H, *J* = 8.0 Hz), 7.69 (d, 1 H, *J* = 8.0 Hz), 7.41 - 7.55 (m, 4 H), 7.35 (s, 2 H), 7.22 (s, 1 H), 4.51 (s, 2 H), 3.82 (s, 6 H), 3.72 (s, 3 H), 3.45 (brs, 8 H); ¹³C NMR (100 MHz, DMSO-*d*₆) *δ* 165.7, 155.2, 154.1, 153.4, 141.0, 135.7, 130.6, 130.2, 129.9, 129.5, 128.6, 127.4, 127.3, 126.9, 125.9, 122.1, 115.0, 105.9, 105.4, 60.8, 59.1, 56.8, 47.7, 40.8.

### [2,4-Dichloro-N-(2-(6-(piperazin-1-ylmethyl)benzofuran-2-yl)phenyl)benzamide hydrochloride] (32p)

Solid; reaction time, 6 hours; yield, 97.5%; ¹H NMR (400 MHz, DMSO-*d*₆) *δ* 12.28 (brs, 1 H), 9.70 (brs, 2 H), 8.00 (brs, 1 H), 7.88 (d, 1 H, *J* = 7.6 Hz), 7.66 - 7.74 (m, 4 H), 7.59 (d, 1 H, *J* = 7.6 Hz), 7.48 - 7.52 (m, 2 H), 7.43 (t, 1 H, *J* = 7.2 Hz), 7.31 (s, 1 H), 4.50 (s, 2 H), 3.42 (brs, 8 H); ¹³C NMR (100 MHz, DMSO-*d*₆) *δ* 165.2, 155.5, 154.4, 136.1, 135.5, 134.8, 132.0, 130.9, 130.4, 130.0, 129.2, 128.6, 128.4, 127.6, 127.1, 126.4, 122.0, 114.8, 105.6, 59.3, 47.9, 40.8.

### [3,4-Dichloro-N-(2-(6-(piperazin-1-ylmethyl)benzofuran-2-yl)phenyl)benzamide hydrochloride] (32q)

White solid; reaction time, 6 hours; yield, 52.4%; ¹H NMR (400 MHz, DMSO-*d*₆) *δ* 12.13 (brs, 1 H), 9.64 (brs, 2 H), 8.22 (brs, 1 H), 7.97 (d, 1 H, *J* = 8.4 Hz), 7.92 (dd, 1 H, *J* = 7.6, 1.2 Hz), 7.88 (s, 1 H), 7.87 (d, 1 H, *J* = 8.0 Hz), 7.69 (d, 1 H, *J* = 7.6 Hz), 7.56 (d, 1 H, *J* = 7.6 Hz), 7.41 - 7.51 (m, 3 H), 7.22 (s, 1 H), 4.46 (s, 2 H), 3.39 (brs, 8 H); ¹³C NMR (100 MHz, DMSO-*d*₆) *δ* 164.2, 155.3, 154.2, 135.3, 135.3, 135.1, 135.1, 132.2, 131.7, 130.4, 130.3, 129.2, 128.8, 128.6, 127.7, 126.7, 122.2, 114.8, 105.3, 59.3, 47.8, 40.8.

### [N-(2-(6-(Piperazin-1-ylmethyl)benzofuran-2-yl)phenyl)nicotinamide hydrochloride] (32r)

Yellow solid; reaction time, 6 hours; yield, 94.6%; ¹H NMR (400 MHz, DMSO-*d*₆) *δ* 12.30 (brs, 1 H), 10.03 (brs, 2 H), 9.43 (s, 1 H), 9.07 (d, 1 H, *J* = 5.2 Hz), 8.96 (d, 1 H, *J* = 7.2 Hz), 8.15 (dd, 1 H, *J* = 7.6, 6.0 Hz), 7.95 (s, 1 H), 7.94 (d, 1 H, *J* = 7.6 Hz), 7.66 (d, 1 H, *J* = 8.0 Hz), 7.58 (d, 1 H, *J* = 8.0 Hz), 7.44 - 7.53 (m, 3 H), 7.35 (s, 1 H), 4.51 (s, 2 H), 3.46 (brs, 8 H); ¹³C NMR (100 MHz, DMSO-*d*₆) *δ* 162.6, 155.3, 154.2, 146.3, 143.6, 134.6, 133.0, 130.5, 130.3, 129.2, 128.9, 127.9, 127.4, 127.3, 126.7, 125.9, 122.1, 115.1, 105.6, 59.1, 47.7, 40.8.

### [N-(2-(6-(Piperazin-1-ylmethyl)benzofuran-2-yl)phenyl)isonicotinamide hydrochloride] (32s)

Yellow solid; reaction time, 6 hours; yield, 100%; ¹H NMR (400 MHz, DMSO-*d*₆) *δ* 12.32 (brs, 1 H), 9.93 (brs, 2 H), 9.07 (d, 2 H, *J* = 5.6 Hz), 8.30 (d, 2 H, *J* = 5.6 Hz), 7.96 (s, 1 H), 7.95 (dd, 1 H, *J* = 8.0, 1.2 Hz), 7.68 (d, 1 H, *J* = 8.0 Hz), 7.59 (d, 1 H, *J* = 7.2 Hz), 7.52 (t, 1 H, *J* = 7.2 Hz), 7.45 - 7.49 (m, 2 H), 7.32 (s, 1 H), 4.51 (s, 2 H), 3.46 (brs, 8 H); ¹³C NMR (100 MHz, DMSO-*d*₆) *δ* 163.6, 155.5, 154.3, 146.9, 146.5, 134.4, 130.4, 129.2, 129.1, 128.0, 127.3, 126.7, 126.0, 124.6, 122.1, 115.0, 105.5, 59.1, 47.7, 40.8.

### [N-(2-(6-(Piperazin-1-ylmethyl)benzofuran-2-yl)phenyl)-1-naphthamide hydrochloride] (32t)

Beige solid; reaction time, 6 hours; yield, 48.3%; ¹H NMR (400 MHz, DMSO-*d*₆) *δ* 12.28 (brs, 1 H), 9.77 (brs, 2 H), 8.20 (d, 1 H, *J* = 8.0 Hz), 8.07 (d, 1 H, *J* = 8.0 Hz), 7.99 (d, 1 H, *J* = 8.0 Hz), 7.96 (s, 1 H), 7.89 - 7.93 (m, 2 H), 7.67 - 7.70 (m, 3 H), 7.49 - 7.59 (m, 4 H), 7.44 (t, 1 H, *J* = 7.6 Hz), 7.31 (s, 1 H), 4.49 (s, 2 H), 3.44 (brs, 8 H); ¹³C NMR (100 MHz, DMSO-*d*₆) *δ* 168.2, 156.0, 154.4, 135.6, 134.8, 133.9, 130.9, 130.5, 130.4, 129.3, 129.0, 128.9, 127.6, 127.4, 127.4, 127.2, 127.1, 126.7, 126.2, 126.0, 125.9, 122.0, 121.6, 114.9, 105.3, 59.3, 47.8, 40.8.

### [N-(2-(6-(Piperazin-1-ylmethyl)benzofuran-2-yl)phenyl)-2-naphthamide hydrochloride] (32u)

Beige solid; reaction time, 6 hours; yield, 76.7%; ¹H NMR (500 MHz, D₂O+DMSO-*d*₆) *δ* 8.33 (s, 1 H), 7.90 - 7.95 (m, 3 H), 7.79 - 7.83 (m, 2 H), 7.59 (t, 1 H, *J* = 7.5 Hz), 7.53 - 7.58 (m, 2 H), 7.40 - 7.48 (m, 3 H), 7.31 (s, 1 H), 7.15 (d, 1 H, *J* = 8.0 Hz), 7.05 (s, 1 H), 4.28 (s, 2 H), 3.26 (brs, 8 H); ¹³C NMR (100 MHz, D₂O+DMSO-*d*₆) *δ* 166.4, 155.5, 154.2, 135.8, 135.1, 132.9, 132.3, 130.5, 130.3, 129.7, 129.2, 128.9, 128.8, 128.6, 128.4, 127.6, 127.4, 127.2, 126.7, 125.0, 122.1, 114.8, 105.2, 59.3, 47.8, 40.8.

### [N-(2-(6-(Piperazin-1-ylmethyl)benzofuran-2-yl)phenyl)quinoline-2-carboxamide hydrochloride] (32v)

White solid; reaction time, 6 hours; yield, 78.2%; ¹H NMR (400 MHz, DMSO-*d*₆) *δ* 12.31 (brs, 1 H), 9.77 (brs, 2 H), 8.66 (d, 1 H, *J* = 8.4 Hz), 8.42 (d, 1 H, *J* = 8.0 Hz), 8.26 (d, 1 H, *J* = 8.4 Hz), 8.13 (d, 1 H, *J* = 8.0 Hz), 8.08 (s, 1 H), 8.05 (d, 1 H, *J* = 8.4 Hz), 7.97 (t, 1 H, *J* = 8.0 Hz), 7.90 (d, 1 H, *J* = 7.6 Hz), 7.81 (d, 1 H, *J* = 7.6 Hz), 7.76 (t, 1 H, *J* = 7.6 Hz), 7.63 (d, 1 H, *J* = 7.6 Hz), 7.56 (t, 1 H, *J* = 8.0 Hz), 7.54 (s, 1 H), 7.36 (t, 1 H, *J* = 7.6 Hz), 4.55 (s, 2 H), 3.41 (brs, 8 H); ¹³C NMR (100 MHz, DMSO-*d*₆) *δ* 162.7, 155.1, 154.6, 150.1, 146.4, 139.4, 135.4, 135.3, 131.9, 130.8, 130.2, 129.8, 129.5, 129.3, 128.9, 125.8, 123.3, 122.3, 121.9, 119.3, 115.1, 106.3, 59.2, 47.9, 41.0.

### [N-(2-(6-(Piperazin-1-ylmethyl)benzofuran-2-yl)phenyl)quinoxaline-2-carboxamide hydrochloride] (32w)

Yellow solid; reaction time, 6 hours; yield, 89.8%; ¹H NMR (400 MHz, D₂O) *δ* 8.15 (s, 1 H), 7.57 (t, 1 H, *J* = 7.2 Hz), 7.46 (d, 1 H, *J* = 8.0 Hz), 7.40 (t, 1 H, *J* = 8.0 Hz), 7.07 (d, 1 H, *J* = 7.2 Hz), 6.91 (d, 1 H, *J* = 7.6 Hz), 6.87 (d, 1 H, *J* = 7.6 Hz), 6.81 (d, 1 H, *J* = 7.6 Hz), 6.78 (s, 1 H), 6.70 (d, 1 H, *J* = 8.0 Hz), 6.44 (m, 2 H), 6.14 (s, 1 H), 4.04 (s, 2 H), 3.55 (brs, 4 H), 3.42 (brs, 4 H); ¹³C NMR (100 MHz, D₂O) *δ* 159.6, 153.5, 152.7, 142.8, 141.7, 141.3, 141.1, 138.5, 133.1, 131.8, 129.4, 129.0, 128.1, 127.8, 127.2, 125.9, 124.8, 124.5, 121.7, 119.4, 117.2, 112.9, 104.0, 60.8, 48.1, 41.0.

### [N-(2-(6-(Piperazin-1-ylmethyl)benzofuran-2-yl)phenyl)bihenyl-4-carboxamide hydrochloride (32x)

Beige solid; reaction time, 6 hours; yield, 78.1%; ¹H NMR (500 MHz, D₂O + DMSO-*d*₆) *δ* 7.65 - 7.91 (m, 3 H), 7.74 (d, 2 H, *J* = 8.0 Hz), 7.63 - 7.67 (m, 2 H), 7.59 (d, 1 H, *J* = 8.5 Hz), 7.37 - 7.48 (m, 7 H), 7.23 (d, 1 H, *J* = 8.5 Hz), 7.08 (s, 1 H), 4.39 (s, 2 H), 3.44 (brs, 4 H), 3.40 (brs, 4 H); ¹³C NMR (100 MHz, D₂O+DMSO-*d*₆) *δ* 166.0, 155.5, 154.2, 143.9, 139.8, 135.7, 133.7, 130.4, 130.2, 129.8, 129.2, 129.0, 128.9, 128.7, 127.6, 127.5, 127.3, 127.2, 126.5, 122.1, 114.8, 105.2, 59.3, 47.8, 40.8.

### [N-(2-(6-(Piperazin-1-ylmethyl)benzofuran-2-yl)phenyl)cinnamamide hydrochloride] (32y)

Beige solid; reaction time, 6 hours; yield, 53.3%; ¹H NMR (400 MHz, DMSO-*d*₆) *δ* 12.24 (brs, 1 H), 9.71 (brs, 2 H), 7.91 (s, 1 H), 7.89 (dd, 1 H, *J* = 7.6, 1,2 Hz), 7.74 (d, 1 H, *J* = 8.0 Hz), 7.62 - 7.69 (m, 4 H), 7.58 (d, 1 H, *J* = 15.6 Hz), 7.36 - 7.51 (m, 6 H), 7.01 (d, 1 H, *J* = 15.6 Hz), 4.45 (s, 2 H), 3.39 (brs, 8 H); ¹³C NMR (100 MHz, DMSO-*d*₆) *δ* 164.9, 154.8, 154.2, 141.1, 135.5, 135.4, 133.9, 130.5, 130.5, 130.1, 129.7, 128.6, 128.5, 127.1, 126.8, 126.8, 125.2, 122.7, 122.2, 114.8, 105.8, 59.4, 47.9, 40.7.

### [(3r,5r,7r)-N-(2-(6-(Piperazin-1-ylmethyl)benzofuran-2-yl)phenyl)adamantane-1-carboxamide hydrochloride] (32z)

Solid; reaction time, 5 hours; yield, 74.6%; ¹H NMR (500 MHz, DMSO-*d*₆) *δ* 12.19 (brs, 1 H), 9.60 (s, 2 H), 7.91 (s, 1 H), 7.83 (d, 1 H, *J* = 8.0 Hz), 7.75 (d, 1 H, *J* = 8.0 Hz), 7.52 - 7.49 (m, 2 H), 7.42 (t, 1 H, *J* = 7.5 Hz), 7.33 (t, 1 H, *J* = 7.5 Hz), 7.21 (s, 1 H), 4.48 (brs, 2 H), 3.42 (brs, 8 H), 2.02 (s, 3 H), 1.92 (s, 6 H), 1.69 (s, 6 H); ¹³C NMR (150 MHz, DMSO-*d*₆) *δ* 176.3, 155.6, 154.0, 135.9, 130.1, 129.9, 128.6, 128.1, 127.0, 126.3, 125.4, 121.8, 114.5, 105.0, 59.1, 47.6, 41.1, 40.4, 39.0, 36.5, 28.1.

### [1-Phenyl-3-(2-(6-(piperazin-1-ylmethyl)benzofuran-2-yl)phenyl)urea hydrochloride] (32A)

Solid; reaction time, 2 hours; yield, 84.1%; ¹H NMR (500 MHz, DMSO-*d*₆) *δ* 9.52 (s, 3 H), 8.50 (s, 1 H), 7.95 (s, 1 H), 7.84 (d, 1 H, *J* = 8.0 Hz), 7.79 (d, 1 H, *J* = 8.0 Hz), 7.75 (d, 1 H, *J* = 8.5 Hz), 7.50 (brd, 1 H, *J* = 7.5 Hz), 7.46 (d, 2 H, *J* = 8.0 Hz), 7.41 (t, 1 H, *J* = 8.0 Hz), 7.39 (s, 1 H), 7.27 - 7.22 (m, 3 H), 6.94 (t, 1 H, *J* = 7.5 Hz), 4.47 (s, 2 H), 3.36 (brs, 8 H); ¹³C NMR (150 MHz, DMSO-*d*₆) *δ* 154.7, 154.1, 153.3, 140.3, 136.5, 130.2, 130.0, 129.2, 128.7, 126.9, 125.1, 124.4, 122.4, 122.3, 121.9, 118.6, 114.8, 105.6, 59.3, 47.8, 40.8.

### [1-(Naphthalen-1-yl)-3-(2-(6-(piperazin-1-ylmethyl)benzofuran-2-yl)phenyl)urea hydrochloride] (32B)

Solid; reaction time, 48 hours; yield, 65.3%; ¹H NMR (500 MHz, DMSO-*d*₆) *δ* 9.51 (brs, 2 H), 9.43 (s, 1 H), 9.01 (s, 1 H), 8.27 (d, 1 H, *J* = 7.0 Hz), 7.96 - 7.90 (m, 3 H), 7.82 (m, 2 H), 7.76 (d, 1 H, *J* = 7.0 Hz), 7.63 (d, 1 H, *J* = 7.0 Hz), 7.58 - 7.49 (m, 3 H), 7.46 (m, 3 H), 7.27 (t, 1 H, *J* = 7.0 Hz), 4.47 (s, 2 H), 3.25 (brs, 8 H); ¹³C NMR (150 MHz, DMSO-*d*₆) *δ* 154.8, 154.1, 154.0, 136.5, 134.9, 134.2, 130.3, 130.0, 128.8, 128.6, 126.9, 126.7, 126.4, 126.3, 126.1, 125.7, 124.7, 123.6, 123.0, 122.4, 121.9, 118.5, 114.8, 105.6, 59.2, 47.7, 40.8.

### [1-Benzyl-3-(2-(6-(piperazin-1-ylmethyl)benzofuran-2-yl)phenyl)urea hydrochloride] (32C

Solid; reaction time, 1 hour; yield, 87.8%; ¹H NMR (500 MHz, DMSO-*d*₆) *δ* 9.64 (s, 2 H), 8.23 (s, 1 H), 7.95 (s, 1 H), 7.86 (d, 1 H, *J* = 8.0 Hz), 7.73 (d, 1 H, *J* = 7.5 Hz), 7.73 (d, 1 H, *J* = 7.5 Hz), 7.52 (brd, 1 H, *J* = 7.5 Hz), 7.38 - 7.29 (m, 6 H), 7.27 (s, 1 H), 7.23 (t, 1 H, *J* = 7.0 Hz), 7.17 (t, 1 H, *J* = 7.5 Hz), 4.49 (s, 2 H), 4.30 (d, 2 H, *J* = 4.5 Hz), 3.43 (s, 8 H); ¹³C NMR (150 MHz, DMSO-*d*₆) *δ* 155.9, 154.9, 154.0, 140.6, 137.4, 130.3, 130.0, 128.8, 128.7, 127.6, 127.2, 126.9, 124.4, 123.8, 121.9, 121.8, 114.8, 105.4, 59.3, 47.7, 43.4.

### Example 2: Synthesis of benzoxazole compound

### 2-1. Synthesis of compound 57 to 70

Compounds 57 to 70 were synthesized as in Reaction Scheme 7 below.

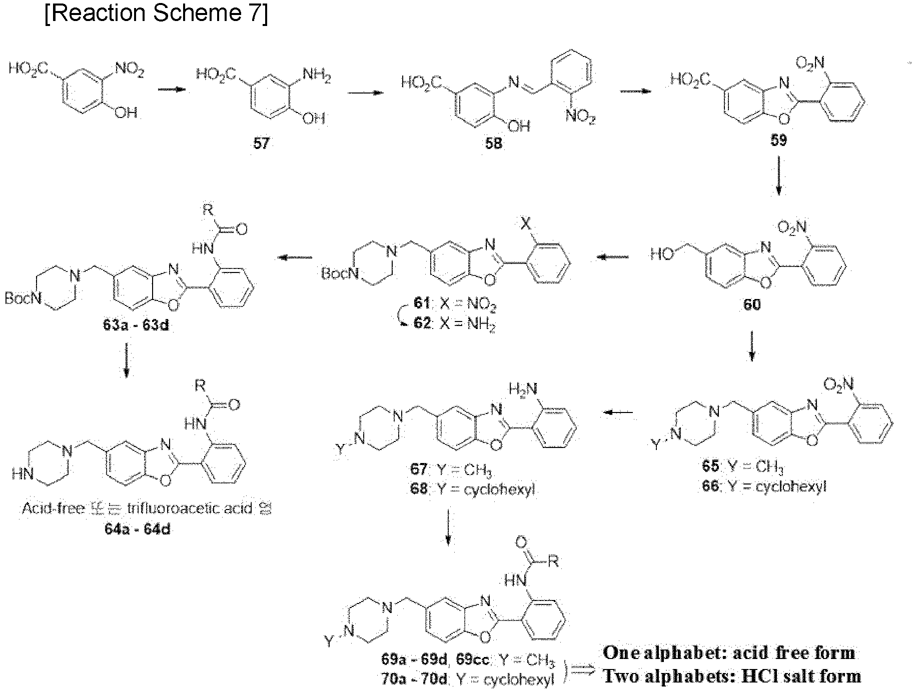

### 1) [3-Amino-4-hydroxybenzoic acid] (57)

Concentrated hydrochloric acid (12N-HCl 1.5 mL) was added to tin (II) chloride (207 mg, 1.09 mmol) at 0 °C, 4-hydroxy-3-nitrobenzoic acid (100 mg, 0.55 mmol) was added thereto, and the mixture was stirred under reflux for 1 hour. Then, water was added and 2N-NaOH was added to adjust to pH 1. The resulting solid was filtered, washed with water, the filtrate was concentrated under reduced pressure, methanol was added and the mixture was filtered again. The residue obtained by concentrating the filtrate under reduced pressure was purified by silica gel column chromatography (methylene chloride:methanol = 5:1) to obtain Compound **57** (82 mg, 98%) as a brown solid.

¹H NMR (500 MHz, DMSO-*d*₆) *δ* 11.30 (brs, 1 H), 9.42 (brs, 3 H), 7.79 (s, 1 H), 7.60 (d, 1 H, *J* = 8.0 Hz), 7.07 (d, 1 H, *J* = 8.0 Hz).

### 2) [(E)-4-Hydroxy-3-(2-nitrobenzylideneamino)benzoic acid] (58)

A solution of DMF (N, N-dimethylformamide, 12 mL) containing Compound **57** (1.25 g, 8.16 mmol) and 2-nitrobenzaldehyde (1.24 g, 8.21 mmol) was stirred at 80 °C for 3 hours. DMF was removed under reduced pressure and water was added, and then the resulting solid was filtered and washed with water to obtain Compound **58** (1.33 g, 57%) as a solid.

¹H NMR (500 MHz, DMSO-*d*₆) *δ* 12.58 (brs, 1 H), 10.09 (s, 1 H), 9.00 (s, 1 H), 8.38 (d, 1 H, *J* = 7.5 Hz), 8.10 (d, 1 H, *J* = 8.0 Hz), 7.87 (t, 1 H, *J* = 7.5 Hz), 7.76 (t, 1 H, *J* = 8.0 Hz), 7.73 (d, 1 H, *J* = 7.5 Hz), 7.72 (s, 1 H), 6.98 (d, 1 H, *J* = 8.5 Hz); ¹³C NMR (100 MHz, DMSO-*d*₆) *δ* 167.7, 157.6, 156.0, 150.0, 138.3, 134.3, 132.5, 131.0, 130.5, 130.3, 125.1, 122.8, 121.9, 116.9.

### 3) [2-(2-Nitrophenyl)benzo[d]oxazole-5-carboxylic acid] (59)

DDQ (2,3-dichloro-5,6-dicyano-*p*-benzoquinone, 888 mg, 3.91 mmol) was added in small quantities at room temperature into a DMF (10 mL) solution containing Compound **58** (1.0 g, 3.49 mmol). After the reaction mixture was stirred at room temperature overnight, DMF was distilled off under reduced pressure, water was added, and the resulting solid was filtered and washed with water to obtain Compound **59** (551 mg, 55.5%) as a solid.

¹H NMR (500 MHz, DMSO-*d*₆) *δ* 8.34 (d, 1 H, *J* = 1.5 Hz), 8.21 (dd, 1 H, *J* = 7.5, 1.5 Hz), 8.15 (dd, 1 H, *J* = 8.0, 1.5 Hz), 8.09 (dd, 1 H, *J* = 8.5, 2.0 Hz), 7.95 (td, 1 H, *J* = 8.0, 1.5 Hz), 7.93 - 7.89 (m, 2 H); ¹³C NMR (100 MHz, DMSO-*d*₆) *δ* 167.4, 160.5, 153.7, 149.3, 141.7, 134.1, 132.2, 128.9, 128.4, 125.3, 122.2, 120.3, 112.0.

### 4) [(2-(2-Nitrophenyl)benzo[d]oxazol-5-yl)methanol] (60)

N,N-diisopropylethylamine (0.06 mL, 0.34 mmol) and ethyl chloroformate (0.034 mL, 0.36 mmol) was added into anhydrous THF (tetrahydrofuran, 1 mL) solution containing Compound **59** (100 mg, 0.35 mmol) at 0 °C, stirred for 1 hour, NaBH₄ (13.3 mg, 0.35 mmol) was added slowly at 0 °C, ethanol (0.5 mL) was added and stirred for 12 hours. THF and ethanol were distilled off under reduced pressure, followed by extraction with water and methylene chloride. The organic layer was dried over anhydrous MgSO₄, filtered and the residue obtained by distilling the filtrate under reduced pressure was purified by silica gel column chromatography (hexane:ethyl acetate = 1:1) to obtain Compound **60** (56.6 mg, 59.5%).

¹H NMR (500 MHz, DMSO-*d*₆) δ 8.18 (dd, 1 H, *J* = 8.0, 1.5 Hz), 8.10 (dd, 1 H, *J* = 8.0, 1.5 Hz), 7.91 (td, 1 H, *J* = 7.5, 1.5 Hz), 7.87 (td, 1 H, *J* = 7.5, 1.5 Hz), 7.75 (d, 1 H, *J* = 1.5 Hz), 7.73 (d, 1 H, *J* = 8.5 Hz), 7.43 (dd, 1 H, *J* = 8.5, 1.5 Hz), 5.34 (t, 1 H, *J* = 5.5 Hz), 4.62 (d, 2 H, *J* = 5.5 Hz); ¹³C NMR (100 MHz, DMSO-*d*₆) *δ* 159.1, 150.0, 149.4, 141.6, 140.9, 133.9, 133.6, 131.8, 125.7, 125.1, 120.6, 118.5, 111.3, 63.4.

### 5) [tert-Butyl 4-((2-(2-nitrophenyl)benzo[d]oxazol-5-yl)methyl)piperazine-1-carboxylate] (61)

Triethylamine (0.15 mL, 1.08 mmol) and methanesulfonyl chloride (0.06 mL, 0.77 mmol) were added into a methylene chloride (10 mL) solution containing Compound 60 (196.4 mg, 0.73 mmol) at room temperature and after stirring for 15 minutes, methylene chloride and saturated brine were added, and the separated organic layer was dried over anhydrous Na₂SO₄, filtered and the resulting filtrate was concentrated under reduced pressure to obtain a residue. Anhydrous THF (2 mL) was added to the residue and anhydrous THF solution (2N solution, 2.0 mL, 4.0 mmol) containing *tert*-butyl piperazine-1-carboxylate (1-Boc-piperazine) was added and stirred at room temperature for 6 hours. THF was distilled off under reduced pressure, methylene chloride and water were added, the organic layer was dried over anhydrous Na₂SO₄, filtered and the residue obtained by concentrating the filtrate was concentrated under reduced pressure was purified by silica gel column chromatography (hexane:ethyl acetate = 2:1) to obtain Compound **61** (318.8 mg, 100%).

¹H NMR (500 MHz, CDCl₃) *δ* 8.13 (d, 1 H, *J* = 8.0 Hz), 7.88 (dd, 1 H, *J* = 7.5, 1.0 Hz), 7.76 (s, 1 H), 7.74 (t, 1 H, *J* = 8.0 Hz), 7.68 (td, 1 H, *J* = 8.0, 1.0 Hz), 7.51 (d, 1 H, *J* = 8.5 Hz), 7.40 (d, 1 H, *J* = 8.5 Hz), 3.64 (s, 2 H), 3.45 (brs, 4 H), 2.43 (brs, 4 H), 1.45 (s, 9 H); ¹³C NMR (100 MHz, CDCl₃) *δ* 159.3, 155.0, 150.5, 149.4, 141.9, 135.2, 132.6, 132.1, 131.6, 127.4, 124.4, 121.7, 121.2, 110.8, 79.8, 63.0, 53.0, 44.0, 28.6.

### 6) [tert-Butyl 4-((2-(2-aminophenyl)benzo[d]oxazol-5-yl)methyl)piperazine-1-carboxylate] (62)

10 wt% Pd/C (palladium on carbon, 60 mg) was added into a methanol (4 mL) solution containing Compound **61** (297.6 mg, 0.68 mmol), and air in the reaction vessel was replaced with hydrogen gas, followed by stirring of at room temperature for 1.5 hours. The reaction mixture was filtered using celite as a filter filler and washed with acetone and methanol. The residue obtained by concentrating the filtrate under reduced pressure was purified by silica gel column chromatography (methylene chloride:methanol = 40:1) to obtain Compound **62** (127.5 mg, 46%).

¹H NMR (500 MHz, DMSO-*d*₆) *δ* 7.89 (d, 1 H, *J* = 8.0 Hz), 7.66 (d, 1 H, *J* = 7.5 Hz), 7.66 (s, 1 H), 7.31 (d, 1 H, *J* = 8.5 Hz), 7.25 (t, 1 H, *J* = 8.0 Hz), 7.09 (s, 2 H), 6.88 (d, 1 H, *J* = 8.5 Hz), 6.66 (t, 1 H, *J* = 8.0 Hz), 3.59 (s, 2 H), 2.33 (brs, 8 H), 1.37 (s, 9 H); ¹³C NMR (100 MHz, DMSO-*d*₆) *δ* 163.7, 154.4, 149.5, 148.4, 142.0, 135.3, 133.3, 128.7, 126.6, 119.8, 116.7, 116.1, 110.7, 107.0, 79.4, 62.4, 52.9, 43.9, 28.7.

### 7) Synthesis of Compound 63 (a to d)

Pyridine (5 equivalents) and the appropriate acyl chloride (2 equivalents) were added into a methylene chloride solution containing Compound **62** (1 equivalent) at room temperature and stirred for 30 min to 2 hours, and after adding methylene chloride and washing with water and sodium bicarbonate water, the organic layer was dried over anhydrous MgSO₄, filtered, and the residue obtained by concentrating the filtrate under reduced pressure was purified by silica gel column chromatography (methylene chloride:methanol = 10:1 to 30: 1) to obtain Compound **63 (a to d)** (81.0 to 99.4%).

### [tert-Butyl 4-((2-(2-benzamidophenyl)benzo[d]oxazol-5-yl)methyl)piperazine-1-carboxylate] (63a)

Yield, 85%; ¹H NMR (500 MHz, CDCl₃) *δ* 12.76 (s, 1 H), 9.06 (d, 1 H, *J* = 8.5 Hz), 8.27 (m, 3 H), 7.72 (brs, 1 H), 7.63 - 7.58 (m, 5 H), 7.44 (brd, 1 H, *J* = 8.0 Hz), 7.25 (t, 1 H, *J* = 8.5 Hz), 3.69 (s, 2 H), 3.49 (brs, 4 H), 2.48 (brs, 4 H), 1.46 (s, 9 H); ¹³C NMR (100 MHz, CDCl₃) *δ* 166.4, 162.7, 155.4, 148.7, 141.2, 139.7, 135.5, 135.3, 133.3, 132.2, 129.0, 128.6, 128.0, 123.3, 120.8, 120.0, 113.4, 110.6, 79.8, 63.0, 53.0, 43.8, 28.6.

### [tert-Butyl 4-((2-(2-(3,4,5-trimethoxybenzamido)phenyl)benzo[d]oxazol-5-yl)methyl)piperazine-1-carboxylate] (63b)

Yield, 99.4%; ¹H NMR (500 MHz, CDCl₃) *δ* 12.56 (s, 1 H), 9.04 (d, 1 H, *J* = 8.5 Hz), 8.28 (dd, 1 H, *J* = 8.5, 1.5 Hz), 7.65 (s, 1 H), 7.60 (td, 1 H, *J* = 8.5, 1.5 Hz), 7.59 (d, 1 H, *J* = 8.5 Hz), 7.46 (s, 2 H), 7.41 (brd, 1 H, *J* = 8.5 Hz), 7.25 (t, 1 H, *J* = 8.5 Hz), 4.03 (s, 6 H), 3.97 (s, 3 H), 3.64 (s, 2 H), 3.46 (brs, 4 H), 2.44 (brs, 4 H), 1.46 (s, 9 H); ¹³C NMR (100 MHz, CDCl₃) *δ* 166.2, 162.8, 155.4, 153.5, 148.7, 141.7, 141.3, 139.7, 135.7, 133.3, 130.9, 128.7, 127.1, 123.3, 120.6, 119.6, 113.4, 110.5, 105.6, 79.8, 63.0, 61.2, 56.7, 53.0, 44.0, 28.6.

### [tert-Butyl 4-((2-(2-(2-naphthamido)phenyl)benzo[d]oxazol-5-yl)methyl)piperazine-1-carboxylate] (63c)

Yield, 95.3%; ¹H NMR (500 MHz, CDCl₃) *δ* 12.97 (s, 1 H), 9.11 (d, 1 H, *J* = 8.5 Hz), 8.82 (s, 1 H), 8.32 (d, 1 H, *J* = 9.0 Hz), 8.29 (d, 1 H, *J* = 8.5 Hz), 8.09 (d, 1 H, *J* = 9.5 Hz), 8.06 (d, 1 H, *J* = 9.0 Hz), 7.97 (d, 1 H, *J* = 8.0 Hz), 7.79 (s, 1 H), 7.65 - 7.61 (m, 3 H), 7.59 (d, 1 H, *J* = 8.5 Hz), 7.42 (brd, 1 H, *J* = 8.5 Hz), 7.26 (t, 1 H, *J* = 8.0 Hz), 3.69 (s, 2 H), 3.49 (m, 4 H), 2.48 (m, 4 H), 1.46 (s, 9 H); ¹³C NMR (100 MHz, CDCl₃) *δ* 166.4, 162.9, 155.0, 148.9, 141.3, 139.8, 135.3, 133.3, 133.0, 132.7, 129.4, 128.8, 128.8, 128.6, 128.2, 128.1, 127.0, 124.5, 123.3, 120.7, 119.8, 113.4, 110.7, 79.8, 62.9, 53.0, 43.3, 28.6.

### [tert-Butyl 4-((2-(2-(quinoxaline-2-carboxamido)phenyl)benzor[d]oxazol-5-yl)methyl)piperazine-1-carboxylate] (63d)

Yield, 81%; ¹H NMR (400 MHz, CDCl₃) *δ* 9.77 (s, 1 H), 9.09 (d, 1 H, *J* = 8.4 Hz), 8.42 (m, 1 H), 8.28-8.22 (m, 2 H), 7.96 - 7.90 (m, 3 H), 7.60 (t, 1 H, *J* = 8.4 Hz), 7.57 (d, 1 H, *J* = 8.0 Hz), 7.39 (d, 1 H, *J* = 8.0 Hz), 7.28 (t, 1 H, *J* = 7.6 Hz), 3.72 (s, 2 H), 3.49 (m, 4 H), 2.48 (m, 4 H), 1.44 (s, 9 H); ¹³C NMR (100 MHz, CDCl₃) *δ* 163.2, 162.4, 155.0, 149.1, 144.8, 144.5, 144.1, 141.8, 140.8, 138.6, 133.0, 132.0, 131.1, 130.1, 129.9, 128.8, 126.9, 124.0, 121.1, 120.0, 114.5, 110.6, 80.0, 63.0, 53.0, 44.2, 28.6.

### 8) Synthesis of Compound 64 (a to d)

Trifluoroaetic acid (13 equivalents) was added into a methylene chloride solution containing Compound **63** (1.0 equivalent) and stirred for 15 hours to 2 days. In the separation and purification method, Compounds **64a** and **64b** were neutralized with sodium bicarbonate water or 1N NaOH, and then methylene chloride and sodium bicarbonate water were added, the separated organic layer was dried over anhydrous MgSO₄, filtered, and the filtrate was concentrated under reduced pressure. The residue was obtained by purification by silica gel column chromatography (methylene chloride:methanol = 5:1). In the case of Compound **64d**, a volatile solvent was distilled off under reduced pressure, and then the resulting solid was filtered and washed with water, and in the case of Compound **64c**, the mixture was neutralized by adding methylene chloride and sodium bicarbonate water, and then the organic layer was dried over anhydrous MgSO₄, filtered, and the residue obtained by concentrating the filtrate under reduced pressure was recrystallized with a mixed solvent (hexane:methylene chloride = 3:1).

### [N-(2-(5-(Piperazin-1-ylmethyl)benzo[d]oxazol-2-yl)phenyl)benzamide] (64a)

Yield, 53%; ¹H NMR (500 MHz, CDCl₃) *δ* 12.76 (s, 1 H), 9.06 (d, 1 H, *J* = 8.5 Hz), 8.27 (m, 3 H), 7.67 (s, 1 H), 7.64 - 7.59 (m, 4 H), 7.58 (d, 1 H, *J* = 8.0 Hz), 7.39 (d, 1 H, *J* = 8.0 Hz), 7.25 (t, 1 H, *J* = 7.5 Hz), 3.69 (s, 2 H), 3.02 (t, 4 H, *J* = 5.0 Hz), 2.58 (brs, 4 H); ¹³C NMR (100 MHz, CDCl₃) *δ* 166.8, 163.0, 148.8, 141.0, 138.9, 134.9, 134.2, 132.9, 132.3, 128.9, 128.5, 127.7, 127.1, 123.5, 120.5, 119.9, 113.4, 110.6, 62.6, 53.6, 43.9.

### [3,4,5-Trimethoxy-N-(2-(5-(piperazin-1-ylmethyl)benzo[d]oxazol-2-yl)phenyl)benzamidel (64b)

Yield, 73%; ¹H NMR (500 MHz, CDCl₃) *δ* 12.55 (s, 1 H), 9.03 (d, 1 H, *J* = 8.5 Hz), 8.26 (d, 1 H, *J* = 8.0 Hz), 7.63 (s, 1 H), 7.58 (t, 1 H, *J* = 8.0 Hz), 7.55 (d, 1 H, *J* = 8.5 Hz), 7.46 (s, 2 H), 7.37 (d, 1 H, *J* = 8.5 Hz), 7.24 (t, 1 H, *J* = 8.0 Hz), 4.03 (s, 6 H), 3.97 (s, 3 H), 3.61 (s, 2 H), 2.98 (t, 4 H, *J* = 4.5 Hz), 2.53 (brs, 4 H); ¹³C NMR (100 MHz, CDCl₃) *δ* 166.1, 162.9, 153.5, 148.8, 141.7, 141.3, 139.6, 135.2, 133.3, 130.9, 128.7, 127.0, 123.3, 120.6, 119.6, 113.3, 110.7, 105.7, 63.1, 61.3, 56.7, 52.0, 44.9.

### [N-(2-(5-(Piperazin-1-ylmethyl)benzo[d]oxazol-2-yl)phenyl)-2-naphthamide] (64c)

Yield, 31.4%; ¹H NMR (500 MHz, CDCl₃) *δ* 12.99 (s, 1 H), 9.11 (d, 1 H, *J* = 9.0 Hz), 8.83 (s, 1 H), 8.32 (d, 1 H, *J* = 8.5 Hz), 8.29 (d, 1 H, *J* = 8.5 Hz), 8.09 (m, 1 H), 8.05 (d, 1 H, *J* = 9.0 Hz), 7.97 (m, 1 H), 7.77 (s, 1 H), 7.65 - 7.63 (m, 2 H), 7.62 (t, 1 H, *J* = 7.5 Hz), 7.58 (d, 1 H, *J* = 8.5 Hz), 7.40 (d, 1 H, *J* = 8.5 Hz), 7.26 (t, 1 H, *J* = 7.5 Hz), 3.67 (s, 2 H), 3.00 (brt, 4 H, *J* = 5.0 Hz), 2.57 (brs, 4 H); ¹³C NMR (100 MHz, CDCl₃) *δ* 166.4, 163.0, 148.7, 141.2, 139.7, 135.6, 135.3, 133.2, 133.0, 132.7, 129.4, 128.8, 128.6, 128.2, 128.1, 127.0, 124.5, 123.3, 120.7, 119.7, 113.4, 110.6, 63.3, 53.7, 45.8.

### [N-(2-(5-(Piperazin-1-ylmethyl)benzo[d]oxazol-2-yl)phenyl)quinoxaline-2-carboxamide 2,2,2-trifluoroacetatel (64d)

Yield, 55.1%; ¹H NMR (500 MHz, DMSO-*d*₆) *δ* 13.65 (s, 1 H), 9.63 (s, 1 H), 8.99 (d, 1 H, *J* = 8.5 Hz), 8.73 (brs, 2 H), 8.39 (d, 1 H, *J* = 8.0 Hz), 8.28 - 8.25 (m, 2 H), 8.14 (t, 1 H, *J* = 7.5 Hz), 8.08 (t, 1 H, *J* = 7.5 Hz), 7.93 (s, 1 H), 7.83 (d, 1 H, *J* = 8.5 Hz), 7.71 (t, 1 H, *J* = 8.0 Hz), 7.47 (d, 1 H, *J* = 8.5 Hz), 7.39 (t, 1 H, *J* = 8.0 Hz), 3.80 (s, 2 H), 3.11 (brm, 4 H), 2.68 (brm, 4 H).

### 9) Synthesis of Compounds 65 and 66

Triethylamine (EtsN, 1.64 equivalents) and methanesulfonyl chloride (1.18 equivalents) were added to a solution of methylene chloride (5 mL per 100 mg weight of Compound **60**) containing Compound **60** (1.0 equivalent) at room temperature and stirred for 15 minutes, methylene chloride and saturated brine were added, the organic layer was dried over anhydrous Na₂SO₄, and the filtrate obtained by filtration was concentrated under reduced pressure to obtain a residue. Anhydrous THF (1 mL per 100 mg weight of Compound **60**) was added to the residue and anhydrous THF solution containing 1-methylpiperazine (5.45 equivalents) or 1-cyclohexylpiperazine (5.45 equivalents) was added and then stirred at room temperature for 1 hour. THF was distilled off under reduced pressure, methylene chloride and water were added, the organic layer was dried over anhydrous Na₂SO₄, filtered and the filtrate was concentrated under reduced pressure to obtain the residue. The residue obtained was purified by silica gel column chromatography (methylene chloride:methanol = 30:1) to obtain Compounds **65** (95.5%) and **66** (74.3%), respectively.

### [5-((4-Methylpiperazin-1-yl)methyl)-2-(2-nitrophenyl)benzo[d]oxazole] (65)

¹H NMR (400 MHz, DMSO-*d*₆) *δ* 8.12 (d, 1 H, *J* = 8.0 Hz), 8.06 (d, 1 H, *J* = 8.0 Hz), 7.85 (t, 1 H, *J* = 8.0 Hz), 7.82 (t, 1 H, *J* = 8.0 Hz), 7.66 (s, 1 H), 7.65 (d, 1 H, *J* = 8.0 Hz), 7.34 (d, 1 H, *J* = 8.4 Hz), 3.50 (s, 2 H), 2.27 (brs, 8 H), 2.07 (s, 3 H); ¹³C NMR (100 MHz, DMSO-*d*₆) *δ* 159.0, 150.1, 149.3, 141.7, 136.4, 133.7, 133.5, 131.7, 127.7, 125.0, 120.7, 120.5, 111.2, 62.3, 55.3, 53.0, 46.2.

### [5-((4-Cyclohexylpiperazin-1-yl)methyl)-2-(2-nitrophenyl)benzo[d]oxazole] (66)

¹H NMR (500 MHz, DMSO-*d*₆) δ 8.18 (dd, 1 H, *J* = 7.5, 1.5 Hz), 8.10 (dd, 1 H, *J* = 7.5, 1.5 Hz), 7.91 (td, 1 H, *J* = 7.5, 1.5 Hz), 7.87 (td, 1 H, *J* = 8.0, 1.5 Hz), 7.72 (d, 1 H, *J* = 8.5 Hz), 7.71 (s, 1 H), 7.40 (dd, 1 H, *J* = 8.5, 1.5 Hz), 3.55 (s, 2 H), 2.48 (brs, 4 H), 2.36 (brs, 4 H), 2.15 (m, 1 H), 1.70 (m, 4 H), 1.53 (m, 1 H), 1.14 (m, 4 H), 1.26 (m, 1 H).

### 10) Synthesis of Compounds 67 and 68

10 wt% Pd/C (palladium on carbon, 20 mg per 100 mg of starting material) was added into a methanol (1 mL per 20 mg of Compounds **65** or **66**) solution containing Compounds **65** (1.0 equivalent) or **66** (1.0 equivalent) and air in the reaction vessel was replaced with hydrogen gas and stirred at room temperature for 2 to 3 hours. The reaction mixture was filtered using celite as the filter filler and washed with acetone. The filtrate was concentrated under reduced pressure to obtain Compounds **67** (96.7%) and **68** (100%), respectively.

### [2-(5-((4-Methylpiperazin-1-yl)methyl)benzo[d]oxazol-2-yl)aniline] (67)

¹H NMR (400 MHz, DMSO-*d*₆) *δ* 7.86 (d, 1 H, *J* = 8.4 Hz), 7.61 (d, 1 H, *J* = 8.4 Hz), 7.60 (s, 1 H), 7.26 (d, 1 H, *J* = 8.4 Hz), 7.23 (t, 1 H, *J* = 7.6 Hz), 7.09 (s, 2 H), 6.86 (d, 1 H, *J* = 8.4 Hz), 6.63 (t, 1 H, *J* = 7.6 Hz), 3.51 (s, 2 H), 2.32 (brs, 8 H), 2.11 (s, 3 H); ¹³C NMR (100 MHz, DMSO-*d*₆) *δ* 163.6, 149.5, 148.3, 141.9, 135.7, 133.2, 128.6, 126.4, 119.6, 116.7, 116.0, 110.5, 107.0, 62.5, 55.3, 53.0, 46.3.

### [2-(5-((4-Cyclohexylpiperazin-1-yl)methyl)benzo[d]oxazol-2-yl)aniline] (68)

¹H NMR (500 MHz, DMSO-*d*₆) *δ* 7.88 (d, 1 H, *J* = 8.0 Hz), 7.64 (d, 1 H, *J* = 8.5 Hz), 7.62 (s, 1 H), 7.28 (d, 1 H, *J* = 8.5 Hz), 7.24 (t, 1 H, *J* = 7.5 Hz), 7.09 (s, 2 H), 6.88 (d, 1 H, *J* = 8.0 Hz), 6.65 (t, 1 H, *J* = 7.5 Hz), 3.53 (s, 2 H), 2.46 (brs, 4 H), 2.36 (brs, 4 H), 2.15 (m, 1 H), 1.70 (m, 4 H), 1.52 (m, 1 H), 1.13 (m, 4 H), 1.03 (m, 1 H); ¹³C NMR (100 MHz, DMSO-*d*₆) *δ* 163.6, 149.4, 148.3, 141.9, 135.7, 133.2, 128.6, 126.5, 119.7, 116.7, 116.1, 110.5, 107.0, 63.2, 62.6, 53.7, 49.0, 29.0, 26.5, 25.9.

### 11) Synthesis of Compounds 69 and 70

Pyridine (5.0 equivalents) and the appropriate acyl chloride (2.0 equivalents) were added into a methylene chloride solution containing Compound **67** (1.0 equivalent) or Compound **68** (1.0 equivalent) at room temperature and stirred for 30 minutes to overnight, After adding methylene chloride and washing each with water and sodium bicarbonate water, the organic layer was dried over anhydrous MgSO₄, filtered, and the residue obtained by concentrating the filtrate under reduced pressure was purified by silica gel column chromatography (methylene chloride:methanol = 40: 1 to 12:1) to obtain Compound **69 (a to d)** (34.6 to 58.2%) and Compound **70 (a to d)** (50.2 to 96%) in the free acid state, respectively.

1,4-dioxane and 8N-HCl (42.0 equivalents) in volume equivalent to 8N-HCl (42.0 equivalents) were added to **69cc** (HCl salt of Compound **69c**): Compound **69c** (1.0 equivalent), respectively and the reaction mixture was stirred at room temperature for 4 hours. The solvent was distilled off under reduced pressure to obtain Compound **69cc** in the form of HCl salt.

### [N-(2-(5-((4-Methylpiperazin-1-yl)methyl)benzo[d]oxazol-2-yl)phenyl)benzamide] (69a)

Yield, 34.6%; ¹H NMR (400 MHz, CDCl₃) *δ* 9.03 (d, 1 H, *J* = 8.4 Hz), 8.25 - 8.24 (m, 3 H), 7.67 (s, 1 H), 7.61 - 7.57 (m, 4 H), 7.54 (d, 1 H, *J* = 8.8 Hz), 7.38 (d, 1 H, *J* = 8.8 Hz), 7.22 (t, 1 H, *J* = 7.6 Hz), 3.63 (s, 2 H), 2.53 (brs, 8 H), 2.31 (s, 3 H); ¹³C NMR (100 MHz, CDCl₃) δ 166.4, 162.7, 148.7, 141.2, 139.6, 135.7, 135.5, 133.1, 132.1, 128.9, 128.6, 128.1, 127.2, 123.3, 120.7, 120.0, 113.4, 110.4, 63.0, 55.2, 53.1, 46.1.

### [3,4,5-Trimethoxy-N-(2-(5-((4-methylpiperazin-1-yl)methyl)benzo[d]oxazol-2-yl)phenyl)benzamide] (69b)

Yield, 48.4%; ¹H NMR (500 MHz, CDCl₃) *δ* 12.59 (s, 1 H), 9.05 (d, 1 H, *J* = 8.5 Hz), 8.28 (dd, 1 H, *J* = 8.5, 1.5 Hz), 7.66 (d, 1 H, *J* = 1.5 Hz), 7.59 (td, 1 H, *J* = 8.5, 1.5 Hz), 7.56 (d, 1 H, *J* = 8.5 Hz), 7.47 (s, 2 H), 7.37 (dd, 1 H, *J* = 8.5, 1.5 Hz), 7.25 (td, 1 H, *J* = 8.0, 1.5 Hz), 4.04 (s, 6 H), 3.98 (s, 3 H), 3.62 (s, 2 H), 2.51 (brs, 8 H), 2.30 (s, 3 H); ¹³C NMR (100 MHz, CDCl₃) *δ* 166.1, 162.8, 153.5, 148.7, 141.7, 141.3, 139.6, 136.0, 133.2, 130.9, 128.7, 127.1, 123.3, 120.6, 119.5, 113.4, 110.5, 105.6, 63.0, 61.2, 56.7, 55.3, 53.2, 46.2.

### [N-(2-(5-((4-Methylpiperazin-1-yl)methyl)benzo[d]oxazol-2-yl)phenyl)-2-naphthamide] (69c) and [N-(2-(5-((4-Methylpiperazin-1-yl)methyl)benzo[d]oxazol-2-yl)phenyl)-2-naphthamide hydrochloride] (69cc)

Compound **69c**: yield, 58.2%; ¹H NMR (500 MHz, CDCl₃) *δ* 12.97 (s, 1 H), 9.10 (d, 1 H, *J* = 8.5 Hz), 8.81 (s, 1 H), 8.31 (d, 1 H, *J* = 8.0 Hz), 8.27 (d, 1 H, *J* = 8.0 Hz), 8.09 (m, 1 H), 8.05 (d, 1 H, *J=* 9.0 Hz), 7.96 (m, 1 H), 7.76 (s, 1 H), 7.64 (m, 2 H), 7.61 (t, 1 H, *J* = 8.5 Hz), 7.57 (d, 1 H, *J* = 8.5 Hz), 7.40 (d, 1 H, *J* = 8.0 Hz), 7.26 (t, 1 H, *J* = 8.0 Hz), 3.69 (s, 2 H), 2.65 (brs, 8 H), 2.41 (s, 3 H); ¹³C NMR (100 MHz, CDCl₃) *δ* 166.4, 162.8, 148.8, 141.2, 139.7, 135.5, 135.3, 133.2, 133.0, 132.7, 129.4, 128.8, 128.8, 128.6, 128.1, 128.1, 127.1 (x2), 124.5, 123.3, 120.7, 119.8, 113.4, 110.6, 62.7, 55.0, 52.5, 45.6.

Compound **69cc**: ¹H NMR (500 MHz, DMSO-*d*₆) *δ* 12.53 (s, 1 H), 8.87 (d, 1 H, *J* = 8.0 Hz), 8.73 (s, 1 H), 8.30 - 8.27 (m, 5 H), 8.04 (m, 1 H), 7.92 (d, 1 H, *J* = 8.5 Hz), 7.74 - 7.70 (m, 4 H), 7.38 (td, 1 H, *J* = 8.0, 1.0 Hz), 4.50 (s, 2 H), 3.46 (brs, 8 H), 2.82 (s, 3 H).

### [N-(2-(5-((4-Methylpiperazin-1-yl)methyl)benzo[d]oxazol-2-yl)phenyl)quinoxaline-2-carboxamide] (69d)

Yield, 51.5%; ¹H NMR (400 MHz, CDCl₃) *δ* 9.78 (s, 1 H), 9.10 (d, 1 H, *J* = 8.4 Hz), 8.44 (dd, 1 H, *J* = 8.0, 1.2 Hz), 8.28 (dd, 1 H, *J* = 8.0, 1.2 Hz), 8.25 (dd, 1 H, *J* = 8.4, 1.6 Hz), 7.99 (td, 1 H, *J* = 8.4, 1.6 Hz), 7.97 (td, 1 H, *J* = 8.0, 1.6 Hz), 7.91 (d, 1 H, *J* = 1.2 Hz), 7.61 (td, 1 H, *J* = 8.4, 1.2 Hz), 7.57 (d, 1 H, *J* = 8.0 Hz), 7.39 (dd, 1 H, *J* = 8.4, 1.2 Hz), 7.29 (td, 1 H, *J* = 8.0, 1.2 Hz), 3.71 (s, 2 H), 2.61 (brs, 8 H), 2.35 (s, 3 H).

### [N-(2-(5-((4-Cyclohexylpiperazin-1-yl)methyl)benzo[d]oxazol-2-yl)phenyl)benzamide] (70a)

Yield, 52.6%; ¹H NMR (500 MHz, CDCl₃) *δ* 12.79 (s, 1 H), 9.06 (d, 1 H, *J* = 8.5 Hz), 8.27 (m, 3 H), 7.68 (s, 1 H), 7.63 - 7.58 (m, 4 H), 7.56 (d, 1 H, *J* = 8.5 Hz), 7.39 (d, 1 H, *J* = 8.0 Hz), 7.25 (t, 1 H, *J* = 8.0 Hz), 3.66 (s, 2 H), 2.69 (brs, 4 H), 2.62 (brs, 4 H), 2.34 (m, 1 H), 1.95 (brm, 2 H), 1.80 (brm, 2 H), 1.63 (m, 1 H), 1.24 (m, 4 H), 1.12 (m, 1 H); ¹³C NMR (100 MHz, CDCl₃) *δ* 166.4, 162.7, 148.7, 141.2, 139.7, 135.5, 133.2, 132.1, 129.0, 128.5, 128.0, 127.3, 123.3, 120.7, 120.1, 113.5, 110.4, 64.0, 63.0, 53.2, 49.0, 28.9, 26.3, 26.0.

### [N-(2-(5-((4-Cyclohexylpiperazin-1-yl)methyl)benzo[d]oxazol-2-yl)phenyl)-3,4,5-trimethoxybenzamide] (70b)

Yield, 61.4%; ¹H NMR (500 MHz, CDCl₃) *δ* 12.58 (s, 1 H), 9.04 (d, 1 H, *J* = 9.0 Hz), 8.28 (d, 1 H, *J* = 8.0 Hz), 7.65 (s, 1 H), 7.59 (t, 1 H, *J* = 8.5 Hz), 7.56 (d, 1 H, *J* = 8.5 Hz), 7.47 (s, 2 H), 7.36 (d, 1 H, *J* = 8.5 Hz), 7.25 (t, 1 H, *J* = 8.0 Hz), 4.03 (s, 6 H), 3.98 (s, 3 H), 3.63 (s, 2 H), 2.66 (brs, 4 H), 2.56 (brs, 4 H), 2.32 (m, 1 H), 1.93 (brm, 2 H), 1.79 (brm, 2 H), 1.63 (brm, 1 H), 1.25 (brm, 4 H), 1.11 (m, 1 H); ¹³C NMR (100 MHz, CDCl₃) *δ* 166.1, 162.8, 153.5, 148.7, 141.7, 141.3, 139.6, 135.7, 133.2, 130.9, 128.7, 127.1, 123.3, 120.6, 119.6, 113.4, 110.5, 105.6, 64.2, 62.8, 61.2, 56.7, 52.9, 49.0, 28.6, 26.2, 25.9.

### [N-(2-(5-((4-Cyclohexylpiperazin-1-yl)methyl)benzo[d]oxazol-2-yl)phenyl)-2-naphthamide] (70c)

Yield, 96.0%; ¹H NMR (500 MHz, CDCl₃) *δ* 12.98 (s, 1 H), 9.11 (d, 1 H, *J* = 8.5 Hz), 8.82 (s, 1 H), 8.32 (d, 1 H, *J* = 9.0 Hz), 8.28 (d, 1 H, *J* = 8.0 Hz), 8.09 (m, 1 H), 8.06 (d, 1 H, *J* = 9.0 Hz), 7.97 (m, 1 H), 7.75 (s, 1 H), 7.65 - 7.63 (m, 2 H), 7.62 (t, 1 H, *J* = 9.0 Hz), 7.57 (d, 1 H, *J* = 8.0 Hz), 7.39 (d, 1 H, *J* = 8.0 Hz), 7.26 (t, 1 H, *J* = 8.0 Hz), 3.70 (s, 2 H), 2.72 (brs, 8 H), 2.30 (brm, 1 H), 2.02 (m, 2 H), 1.83 (brm, 2 H), 1.65 (brm, 1 H), 1.25 (brm, 4 H), 1.13 (m, 1 H); ¹³C NMR (100 MHz, CDCl₃) *δ* 166.5, 163.0, 148.9, 141.4, 139.8, 135.3, 134.8, 133.4, 133.1, 132.7, 129.4, 129.0, 128.8, 128.6, 128.2, 128.1, 127.2, 127.0, 124.3, 123.3, 120.8, 119.9, 113.3, 110.8, 64.0, 62.9, 52.7, 48.7, 29.9, 26.7, 25.3.

### [N-(2-(5-((4-Cyclohexylpiperazin-1-yl)methyl)benzo[d]oxazol-2-yl)phenyl)quinoxaline-2-carboxamide] (70d)

Yield, 50.2%;; ¹H NMR (500 MHz, CDCl₃) *δ* 13.81 (s, 1 H), 9.79 (s, 1 H), 9.11 (d, 1 H, *J* = 9.0 Hz), 8.44 (d, 1 H, *J* = 7.5 Hz), 8.28 (dd, 1 H, *J* = 8.0, 1.5 Hz), 8.26 (d, 1 H, *J* = 8.0 Hz), 8.01 (td, 1 H, *J* = 8.0, 1.0 Hz), 7.95 (td, 1 H, *J* = 8.5, 1.5 Hz), 7.88 (s, 1 H), 7.62 (td, 1 H, *J* = 8.0, 1.5 Hz), 7.58 (d, 1 H, *J* = 8.0 Hz), 7.40 (dd, 1 H, *J* = 8.5, 1.0 Hz), 7.30 (t, 1 H, *J* = 8.0 Hz), 3.73 (s, 2 H), 2.73 (brs, 8 H), 2.44 (brm, 1 H), 1.99 (m, 2 H), 1.82 (brm, 2 H), 1.64 (brm, 1 H), 1.26 (m, 4 H), 1.11 (m, 1 H); ¹³C NMR (100 MHz, CDCl₃) *δ* 163.2, 162.3, 149.0, 144.8, 144.5, 144.1, 141.7, 140.8, 138.6, 135.3, 132.9, 132.0, 131.3, 130.1, 129.9, 128.8, 127.0, 124.0, 121.1, 120.2, 114.6, 110.6, 64.3, 62.9, 52.7, 49.1, 28.6, 26.1, 25.9.

### Example 3: Investigation of SIRT 1 activity of derivatives synthesized based on benzo[d]furan backbone and SIRT 1 activator (Compound 32u)

### 3-1. Confirmation of SIRT 1 Activity of Compound 32u

SIRT 1 activity of candidate SIRT 1 activator compounds synthesized based on the benzo[d]furan backbone were measured using a SIRT 1 fluorometric drug discovery kit (AK-555, Biomol). Briefly, as the substrate for analysis, 0.2 U/L of Fluor de Lys-SIRT 1 substrate developer mixture is used, which is amino acid 379382 of human p53 (Arg-His-Lys-Lys AC), and SIRT 1 incubated with cosubstrate NAD⁺. The degree of initial deacetylation of SIRT 1 for 100 µM candidate was measured using 100 µM resveratrol as a positive control in 25 µM Fluor de Lys-Sirt1 and 25 µM NAD⁺ environments. The reaction was stopped using Fluor de Lys^{™} Developer II/2 mM nicotinamide, followed by measuring fluorescence (Excitation: 360 nm, Emission: 460 nm).

As a result, referring to FIG. 1A to FIG. 1C, it was confirmed that the Compound 32u shows a higher SIRT 1 activity effect than resveratrol known as a strong SIRT 1 activator. Compounds with zero values have too high self-fluorescence to measure with their SIRT 1 fluorometric kit (AK-555, Biomol).

In addition, docking simulation was performed using the AutoDock4 and DOCK6 programs to confirm the binding affinity with SIRT 1 for Compound 32u. Docking simulation is one of the most widely used methods in structure-based drug screening analysis. In particular, when the three-dimensional structure of the target molecule is known, it can be very useful. The docking simulation searches for compounds that can bind to the target molecule, and calculates the optimal position and binding affinity of the compound in the pocket of the target molecule. The most popular academic programs in docking simulations are the DOCK6 and AutoDock4 programs. Generally, docking simulations start with the coordinates of the three-dimensional atoms of the target molecule and in particular define pockets which are key sites for protein regulation. A three-dimensional lattice box is prepared in the defined pocket, and each compound is matched to the lattice box to calculate the binding affinity between the target molecule and the compound. In particular, the binding affinity between the target molecule and the compound is called a docking score, the docking score value is expressed as a negative number, the larger the negative value can be the higher the binding affinity.

As a result, referring to FIG. 1D, it was confirmed that the Compound 32u has a higher binding affinity with SIRT 1 than resveratrol and SRT1720, which are known as strong SIRT 1 activators.

### 3-2. Confirmation of improved insulin resistance of Compound 32u

The metabolic disease control ability of Compound 32u was analyzed using db/db mice lacking an appetite-regulating hormone leptin receptor as an obese animal model. Compound 32u was orally administered to db/db mice (9 weeks old) at a concentration of 5 mg/kg daily for 4 weeks.

As a result, referring to FIG. 2A and FIG. 2B, it was confirmed that the dietary intake and weight of the homozygote db/db mice significantly increased compared to the heterozygote db/m mice without obesity phenotype, which means that the obese animal model is well induced. The group orally administered Compound 32u to db/db mice showed no difference in dietary intake and weight compared to db/db mice group.

Three weeks after oral administration of Compound 32u, a glucose tolerance test was performed. Mice were fasted for 12 hours or more, and glucose (2 g/kg) was injected intraperitoneally, and blood from the tail of the mouse was measured for blood glucose at each time using a glucometer (ACCU-CHECK).

As a result, referring to FIG. 2C, it was confirmed that the db/db mouse group has poor insulin signaling, resulting in glucose tolerance that maintains high levels of blood glucose, whereas the glucose tolerance was restored in the group administered orally with Compound 32u to reduce the blood glucose concentration. In addition, referring to FIG. 2D, when compared to the db/db mouse group, it was confirmed that fasting blood glucose is reduced in the Compound 32u oral administration group.

Next, the blood insulin concentration was measured using an enzyme-linked immunosorbent assay (ELISA). Mouse insulin ELISA (TMB) kit (AKRIN-011T, SHIBAYAGI, Japan) was used, and serum separated from animal blood was added to a 96 well plate coated with insulin primary antibody and after reacting, the secondary antibody and the substrate were added, reacted and the changed color was measured by an absorbance meter.

As a result, referring to FIG. 2E, compared with the db/db mouse group, it was confirmed that the serum insulin concentration is reduced in the Compound 32u oral administration group.

### 3-3. Confirmation of fatty liver improvement of Compound 32u

Since obesity and insulin resistance are representative diseases directly related to the development of non-alcoholic fatty liver, the improvement effect of Compound 32u on fatty liver was analyzed. Compound 32u was orally administered to db/db mice (9 weeks old) at a concentration of 5 mg/kg daily for 4 weeks. First, the accumulation of fat in the liver, the most representative phenotype, was measured. Briefly, about 50 mg of liver was quantified in cold phosphate buffered saline (PBS) or 0.9% sodium chloride (NaCl) and crushed, followed by treating with mixed solvent of chloroform and methanol (MeOH) in a volume ratio of 2:1 and reacted for 2 hours. The sample was then filtered using a paper filter, and the filtered liquid was removed in a dry-oven at 60 °C. Thereafter, the amount of neutral fat was measured by absorbance using a neutral fat measurement kit (Stanbio laboratory, TX USA).

As a result, referring to FIG. 3A, it was confirmed that neutral fat is accumulated in the liver of the db/db mouse group, i.e, the obese animal model, which means that non-alcoholic fatty liver is induced. On the other hand, the oral administration of Compound 32u group was confirmed to be reduced neutral fat, which means that non-alcoholic fatty liver is alleviated.

In order to confirm the corresponding mechanism, mRNA expression of genes related to fat synthesis and degradation was analyzed. Briefly, about 25 mg of liver was quantified and RNA was isolated using RiboEx Total RNA (GeneAll Biotechnology), and then cDNA was synthesized using Hyperscript^{™} One-Step RT-PCR Master mix (GeneAll Biotechnology). For the synthesized cDNA, a quantative real-time polymerase chain reaction (quantative real-time PCR) was performed to target the desired gene as a primer and quantify it in real time.

As a result, referring to FIG. 3B, it was confirmed that the expression of fat synthesis genes (FASN, ACC, SREBP-1a, SREBP-1c and SREBP-2) in the db/db mouse group was increased, while in Compound 32u orally administered group, the genes related to fat synthesis were significantly reduced. In contrast, referring to FIG. 3C, the expression of fat oxidation related genes (CPT1 and ACOX1) was not significantly different in the two groups. The results suggest that decreased expression of fat synthesis-related genes by Compound 32u may have contributed to the improvement of non-alcoholic fatty liver by reducing hepatic neutral fat.

The cause of non-alcoholic liver disease (NAFLD) is unclear, but recent studies report that endoplasmic reticulum (ER) stress can be involved in fatty liver development and progression to non-alcoholic fatty hepatitis. Thus, in order to analyze the effect of Compound 32u on endoplasmic reticulum stress-related marker (p-IRE) was identified by Western blot. Briefly, about 50 mg of liver was quantified, homogenated and lysed to extract proteins. After quantifying the extracted protein and loading the same amount thereof in the polyacrylamide gel, the protein was separated according to molecular weight through SDS-PAGE (sodium dodecyl sulfate-polyacrylamide gel electrophoresis). Proteins separated according to molecular weight were transferred to a membrane by applying an electric field, and the desired protein was detected using a specific antibody. After reacting the primary antibody that specifically binds to the protein to be detected for at least 12 hours, the secondary antibody that specifically binds to the primary antibody is reacted for 1 hour, and the expression of the protein can be analyzed by treating the developer liquid containing the substrate HRP (horse radish peroxidase) present in the secondary antibody.

As a result, referring to FIG. 3D, it was confirmed that increased protein expression of endoplasmic reticulum stress-related markers (p-IRE) in the db/db mouse group, i.e. an obese animal model, was significantly reduced in the orally treated compound 32u. α-tubulin is a housekeeping gene, confirming that the protein is quantitatively loaded. This suggests that Compound 32u reduced endoplasmic reticulum stress induced by obesity, which contributed to alleviation of fatty liver.

### 3-4. Confirmation of cellular aging protection of Compound 32u

β-galactosidase is used to induce cellular aging of fibroblasts, which are the main cells of the epidermal layer of skin, by using UVB, the most important environmental factor in skin aging, as the most common marker for cell aging and β-galactosidase staining was performed to analyze the cell aging protection of Compound 32u.

As a result, referring to FIG. 4A, it was confirmed that the cells stained with β-galactosidase were increased by UVB of 12 mJ/cm², and the group pretreated with Compound 32u at a concentration of 1 µM for 24 hours was confirmed that β-galactosidase staining was reduced. This means that Compound 32u has a protective effect of the cell aging.

The effect of prolonging lifespan in rapidly dividing yeast (Saccharomyces cerevisiae) model was analyzed for Compound 32u which has been shown to inhibit cell aging.

As a result, referring to FIG. 4B, when compared with the control group, it was confirmed that the lifespan is significantly extended in the yeast group treated with Compound 32u. From these results, it was confirmed that the Compound 32u has a cell aging protective effect and life extension effect.

### Example 4: Investigation of SIRT 1 activity of derivatives synthesized based on benzo[d]oxazole backbone and SIRT 1 activator (Compound 70c)

### 4-1. Confirmation of SIRT 1 activity of Compound 70c

The SIRT 1 activity for candidate SIRT 1 activator compounds synthesized based on the benzo[d]oxazole backbone were measured using SIRT 1 fluorometric drug discovery kit (AK-555, Biomol). Briefly, 0.2 U/L of Fluor de Lys-SIRT 1 substrate developer mixture liquid was used as the analytical substrate, meaning amino acid 379382 of human p53 (Arg-His-Lys-Lys AC) and SIRT 1 incubated with ancillary substrate NAD⁺. The initial degree of deacetylation of SIRT 1 for 100 µM candidate was measured using 100 µM resveratrol as a positive control in 25 µM Fluor de Lys-Sirt1 and 25 µM NAD⁺. The reaction was stopped using Fluor de Lys^{™} Developer II/2 mM nicotinamide, followed by fluorescence (Excitation: 360 nm, Emission: 460 nm).

As a result, referring to FIG. 5A to FIG. 5F, it was confirmed that the Compound 70c shows a higher SIRT 1 activity effect than resveratrol known as a strong SIRT 1 activator. Compounds with zero values had too high their own fluorescence value to measure by their SIRT 1 fluorometric kit (AK-555, Biomol).

In addition, docking simulation was performed using the AutoDock4 and DOCK6 programs to confirm the binding affinity with SIRT 1 of Compound 70c.

As a result, referring to FIG. 5G, Compound 70c was confirmed to have a higher binding affinity with SIRT 1 than resveratrol and SRT1720, which are known as strong SIRT 1 activators.

### 4-2. Confirmation of improved insulin resistance of Compound 70c

The metabolic disease control ability of Compound 70c was analyzed using db/db mice lacking an appetite-regulating hormone leptin receptor as an obese animal model. Compound 70c was orally administered to db/db mice (9 weeks old) at a concentration of 5 mg/kg daily for 4 weeks.

As a result, referring to FIG. 6A and FIG. 6B, it was confirmed that the dietary intake and weight of the homozygote db/db mice significantly increased compared to the heterozygote db/m mice without obesity phenotype, which means that the obese animal model is well induced. The group orally administered Compound 70c to db/db mice showed no difference in dietary intake and weight compared to db/db mice group.

Three weeks after oral administration of Compound 70c, a glucose tolerance test was performed. Mice were fasted for 12 hours or more, and glucose (2 g/kg) was injected intraperitoneally, and blood from the tail of the mouse was measured for blood glucose at each time using a glucometer (ACCU-CHECK).

As a result, referring to FIG. 6C, it was confirmed that the db/db mouse group has poor insulin signaling, resulting in glucose tolerance that maintains high levels of blood glucose, whereas the glucose tolerance was restored in the group administered orally with Compound 70c to reduce the blood glucose concentration. In addition, referring to FIG. 6D, when compared to the db/db mouse group, it was confirmed that fasting blood glucose is reduced in the Compound 70c oral administration group.

Next, the blood insulin concentration was measured using an enzyme-linked immunosorbent assay (ELISA). Mouse insulin ELISA (TMB) kit (AKRIN-011T, SHIBAYAGI, Japan) was used, and serum separated from animal blood was added to a 96 well plate coated with insulin primary antibody and after reacting, the secondary antibody and the substrate were added, reacted and the changed color was measured by an absorbance meter.

As a result, referring to FIG. 6E, compared with the db/db mouse group, it was confirmed that the serum insulin concentration is reduced in the Compound 70c oral administration group.

### 4-3. Confirmation of fatty liver improvement of Compound 70c

Since obesity and insulin resistance are representative diseases directly related to the development of non-alcoholic fatty liver, the improvement effect of Compound 70c on fatty liver was analyzed. Compound 70c was orally administered to db/db mice (9 weeks old) at a concentration of 5 mg/kg daily for 4 weeks. First, the accumulation of fat in the liver, the most representative phenotype, was measured. Briefly, about 50 mg of liver was quantified in cold phosphate buffered saline (PBS) or 0.9% sodium chloride (NaCl) and crushed, followed by treating with mixed solvent of chloroform and methanol (MeOH) in a volume ratio of 2:1 and reacted for 2 hours. The sample was then filtered using a paper filter, and the filtered liquid was removed in a dry-oven at 60 °C. Thereafter, the amount of neutral fat was measured by absorbance using a neutral fat measurement kit (Stanbio laboratory, TX USA).

As a result, referring to FIG. 7A, it was confirmed that neutral fat is accumulated in the liver of the db/db mouse group, i.e, the obese animal model, which means that non-alcoholic fatty liver is induced. On the other hand, the oral administration of Compound 70c group was confirmed to be reduced neutral fat, which means that non-alcoholic fatty liver is alleviated.

In order to confirm the corresponding mechanism, mRNA expression of genes related to fat synthesis and degradation was analyzed. Briefly, about 25 mg of liver was quantified and RNA was isolated using RiboEx Total RNA (GeneAll Biotechnology), and then cDNA was synthesized using Hyperscript^{™} One-Step RT-PCR Master mix (GeneAll Biotechnology). For the synthesized cDNA, a quantative real-time polymerase chain reaction (quantative real-time PCR) was performed to target the desired gene as a primer and quantify it in real time.

As a result, referring to FIG. 7B, it was confirmed that the expression of fat synthesis genes (FASN, ACC, SREBP-1a, SREBP-1c and SREBP-2) in the db/db mouse group was increased, while in Compound 70c orally administered group, the genes related to fat synthesis were significantly reduced. In contrast, referring to FIG. 7C, the expression of fat oxidation related genes (CPT1 and ACOX1) was not significantly different in the two groups. The results suggest that decreased expression of fat synthesis-related genes by Compound 70c may have contributed to the improvement of non-alcoholic fatty liver by reducing hepatic neutral fat.

The cause of non-alcoholic liver disease (NAFLD) is unclear, but recent studies report that endoplasmic reticulum (ER) stress can be involved in fatty liver development and progression to non-alcoholic fatty hepatitis. Thus, in order to analyze the effect of Compound 70c on endoplasmic reticulum stress-related marker (p-IRE) was identified by Western blot. Briefly, about 50 mg of liver was quantified, homogenated and lysed to extract proteins. After quantifying the extracted protein and loading the same amount thereof in the polyacrylamide gel, the protein was separated according to molecular weight through SDS-PAGE (sodium dodecyl sulfate-polyacrylamide gel electrophoresis). Proteins separated according to molecular weight were transferred to a membrane by applying an electric field, and the desired protein was detected using a specific antibody. After reacting the primary antibody that specifically binds to the protein to be detected for at least 12 hours, the secondary antibody that specifically binds to the primary antibody is reacted for 1 hour, and the expression of the protein can be analyzed by treating the developer liquid containing the substrate HRP (horse radish peroxidase) present in the secondary antibody.

As a result, referring to FIG. 7D, it was confirmed that increased protein expression of endoplasmic reticulum stress-related markers (p-IRE) in the db/db mouse group, i.e. an obese animal model, was significantly reduced in the orally treated Compound 70c. α-tubulin is a housekeeping gene, confirming that the protein is quantitatively loaded. This suggests that Compound 70c reduced endoplasmic reticulum stress induced by obesity, which contributed to alleviation of fatty liver.

### 4-4. Confirmation of cellular aging protection of Compound 32u

β-galactosidase is used to induce cellular aging of fibroblasts, which are the main cells of the epidermal layer of skin, by using UVB, the most important environmental factor in skin aging, as the most common marker for cell aging and β-galactosidase staining was performed to analyze the cell aging protection of Compound 32u.

As a result, referring to FIG. 8, it was confirmed that the cells stained with β-galactosidase were increased by UVB of 12 mJ/cm², and the group pretreated with Compound 70c at a concentration of 1 µM for 24 hours was confirmed that β-galactosidase staining was reduced. This means that Compound 70c has a protective effect of the cell aging.

### Example 5: Investigation of SIRT 1 and PPARβ/δ activity of derivative Compound 69c synthesized based on benzo[d]oxazole backbone

### 5-1. Confirmation of SIRT 1 and PPARβ/δ activity of Compound 69c

To confirm the binding affinity of SIRT 1 and PPARβ/δ to Compound 69c, docking simulations were performed using AutoDock4 and DOCK6 programs.

As a result, referring to FIG. 9A, Compound 69c was confirmed to have a higher binding affinity with SIRT 1 than resveratrol known as a strong SIRT 1 activator. In addition, as a result of measuring the binding affinity with PPARβ/δ reported to modulate the transcription of SIRT 1, it was confirmed that Compound 69c has a higher binding affinity with PPARβ/δ than GW501516 known as a PPARβ/δ agonist.

Next, to analyze the effect of PPARβ/δ activity of Compound 69c, a PPAR response element (PPRE)-luciferase assay was performed on fibroblasts, which are the main cells of the skin epithelial layer. A binding site region of PPARβ/δ, i.e. plasmids in which the PPRE region was bound upstream of the luciferase gene were transfected by treating the cells with lipofectamine. Through this process, DNA is surrounded by lipofectamine, making it easier to pass the cell membrane. In addition, it is possible to identify which of the PPAR subtypes is effective by overexpressing PPARβ/δ in addition to the endogenous PPAR subtype. 18 to 24 hours after transduction, at least 5 hours after treatment with the candidate compound, fluorescence was measured by addition of the substrate of the luciferase assay system. Luciferase expression amount can be analyzed by the fluorescence value, and the activity level of PPARβ/δ can be analyzed through the above results.

As a result, referring to FIG. 9B, it was confirmed that Compound 69c has a higher PPARβ/δ activity than GW501516. In addition, 1 hour and 30 minutes after treating cells with Compound 69c, western blot results show an increase in PPARβ/δ transfer in the nucleus, indicating that PPARβ/δ was activated and migrated into the nucleus.

To confirm whether Compound 69c increases the expression of SIRT 1 through the activation of PPARβ/δ, real-time PCR and western blot were performed.

As a result, referring to FIG. 9C, it was confirmed that the mRNA and protein expression levels of SIRT 1 were increased by Compound 69c.

Furthermore, SIRT 1 is known to exert its function through deacetylation of various substrate proteins. Referring to FIG. 9D, it was confirmed that acetylation of p53, which is a marker of cell aging and target protein of SIRT 1, was increased by UVB, and Compound 69c inhibited acetylation of p53 increased by UVB.

Therefore, to summarize the above results, Compound 69c was confirmed to increase the expression of SIRT 1 through PPARβ/δ activation, thereby inducing deacetylation of p53, a marker of cellular aging.

### 5-2. Confirmation of cellular aging and oxidative stress protection of Compound 69c

β-galactosidase is used to induce cellular aging of fibroblasts, which are the main cells of the epidermal layer of skin, by using UVB, the most important environmental factor in skin aging, as the most common marker for cell aging and β-galactosidase staining was performed to analyze the cell aging protection of Compound 69c.

As a result, referring to FIG. 10A, it was confirmed that the cells stained with β-galactosidase were increased by UVB of 10 mJ/cm², and the group pretreated with Compound 69c at a concentration of 10 µM for 24 hours was confirmed that β-galactosidase staining was reduced. This means that Compound 69c has a protective effect of the cell aging.

In addition, as a result of analyzing the reactive oxygen species (ROS), a kind of oxidative stress with DCF-DA (2',7'-dichlorofluorescin diacetate) fluorescent dye, as shown in FIG. 10B, It was confirmed that the reactive oxygen species induced by UVB was inhibited by Compound 69c.

Thus, in summary, it was confirmed that Compound 69c increased the expression of SIRT 1 through PPARβ/δ activation, thereby inhibiting cellular aging and oxidative stress.

### 5-3. Confirmation of collagen synthesis/degradation of Compound 69c in skin cell photoaging

Wrinkles are grooves that form on the surface of the skin due to the loss of collagen and elastic fibers in the dermal layer of skin. The main mechanisms involved in the formation of skin wrinkles are that as the skin is continuously exposed to oxidative stress from harmful environments such as air pollution, UV exposure, stress or disease, etc. the increased radicals in the body destroy the dermal connective tissue such as e collagen, elastin and hyaluronic acid to cause subsidence of certain parts of the skin.

In order to improve skin wrinkles, ascorbic acid, α-tocopherol, retinol and derivatives thereof, and super oxide dismutase (SOD) are conventionally incorporated into cosmetics or medicines for improving skin wrinkles thereby using to prevent wrinkles and other skin diseases, but the materials are not only expensive, but also have a poor chemical stability, so there is a problem that it is difficult to expect a substantial effect.

In order to solve these problems, many researches have been conducted to develop materials that are more safe and effective in improving skin wrinkles in medicine, food and cosmetics. In particular, recently, studies on the effects of wrinkle improvement by SIRT 1 have been reported, and thus SIRT 1 is considered as the target protein of wrinkle improvement.

First, the effect of Compound 69c on pro-collagen expression by cell photoaging was analyzed. A collagen reduction model by UVB was constructed using skin fibroblasts to perform the experiments. Skin fibroblasts were pretreated with 10 µM Compound 69c one hour ago and irradiated with 8 mJ/cm² of UVB. After 6 hours, the cells were lysed and the expression of pro-collagen was confirmed via Western blotting.

As a result, referring to FIG. 11A, it was confirmed that the expression of pro-collagen reduced by UVB was increased by Compound 69c.

Next, studies on the formation of skin wrinkles due to photoaging have been shown to increase the activity of matrix metalloproteinases (MMPs) which acts to decompose elastic collagen and other connective tissue components of the dermis according the increases of UV irradiation dose, and the reason why collagen decreases during light damage has been reported to be a phenomenon in which collagen synthesis is inhibited due to increased activity of MMP1/13.

Based on this, in order to confirm the effect of Compound 69c on the expression level of intracellular MMPs, 10 µM of Compound 69c was pretreated 1 hour prior to skin fibroblasts and irradiated with UVB of 8 mJ/cm². After 6 hours, cells were lysed and the expression levels of MMP1 and MMP13 were confirmed by Western blotting.

As a result, referring to FIG. 11B, it was confirmed that the expression of MMP1 and MMP13 increased by UVB is reduced by Compound 69c.

Accordingly, to summarize the above results, Compound 69c was confirmed to increase the synthesis of pro-collagen and reduce the expression of MMPs (MMP1/13) and thus it is effective in improving wrinkles by photoaging.

## Claims

1. A compound represented by a following Chemical Formula 2-3, a crystalline form thereof, a hydrate thereof or a salt thereof: 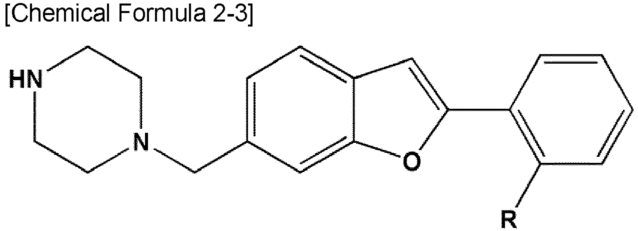 where R is any one selected from the group consisting of benzamide, nicotinamide, isonicotinamide, naphthaamide, quinoline-2-carboxamide, quinoxaline-2-carboxamide, cinnamamide, adamantane-1-carboxamide, phenylurea, naphthylurea and benzylurea, which is unsubstituted or substituted with one or more substituents selected from (C1-C4)alkyl, (C1-C4)alkoxy, halogen, nitro or phenyl.

2. The compound, crystalline form thereof, hydrate thereof or salt thereof of claim 1, wherein the compound represented by the Chemical Formula 2-3 is selected from the group consisting of [*N*-(2-(6-(Piperazin-1-ylmethyl)benzofuran-2-yl)phenyl)benzamide hydrochloride] (32a), [2-Methyl-*N*-(2-(6-(piperazin-1-ylmethyl)benzofuran-2-yl)phenyl)benzamide hydrochloride] (32b), [3-Methyl-*N*-(2-(6-(piperazin-1-ylmethyl)benzofuran-2-yl)phenyl)benzamide hydrochloride] (32c), [4-Methyl-*N*-(2-(6-(piperazin-1-ylmethyl)benzofuran-2-yl)phenyl)benzamide hydrochloride] (32d), [2-Fluoro-*N*-(2-(6-(piperazin-1-ylmethyl)benzofuran-2-yl)phenyl)benzamide hydrochloride] (32e), [3-Fluoro-*N*-(2-(6-(piperazin-1-ylmethyl)benzofuran-2-yl)phenyl)benzamide hydrochloride] (32f), [2-Bromo-*N*-(2-(6-(piperazin-1-ylmethyl)benzofuran-2-yl)phenyl)benzamide hydrochloride] (32g), [3-Bromo-*N*-(2-(6-(piperazin-1-ylmethyl)benzofuran-2-yl)phenyl)benzamide hydrochloride] (32h), [4-Bromo-*N*-(2-(6-(piperazin-1-ylmethyl)benzofuran-2-yl)phenyl)benzamide hydrochloride] (32i), [2-Chloro-*N*-(2-(6-(piperazin-1-ylmethyl)benzofuran-2-yl)phenyl)benzamide hydrochloride] (32j), [3-Chloro-*N*-(2-(6-(piperazin-1-ylmethyl)benzofuran-2-yl)phenyl)benzamide hydrochloride] (32k), [4-Chloro-*N*-(2-(6-(piperazin-1-ylmethyl)benzofuran-2-yl)phenyl)benzamide hydrochloride] (32l), [3-Nitro-*N*-(2-(6-(piperazin-1-ylmethyl)benzofuran-2-yl)phenyl)benzamide hydrochloride] (32m), [4-Nitro-*N*-(2-(6-(piperazin-1-ylmethyl)benzofuran-2-yl)phenyl)benzamide hydrochloride] (32n), [3,4,5-Trimethoxy-*N*-(2-(6-(piperazin-1-ylmethyl)benzofuran-2-yl)phenyl)benzamide hydrochloride] (32o), [2,4-Dichloro-*N*-(2-(6-(piperazin-1-ylmethyl)benzofuran-2-yl)phenyl)benzamide hydrochloride] (32p), [3,4-Dichloro-*N*-(2-(6-(piperazin-1-ylmethyl)benzofuran-2-yl)phenyl)benzamide hydrochloride] (32q), [*N*-(2-(6-(Piperazin-1-ylmethyl)benzofuran-2-yl)phenyl)nicotinamide hydrochloride] (32r), [*N*-(2-(6-(Piperazin-1-ylmethyl)benzofuran-2-yl)phenyl)isonicotinamide hydrochloride] (32s), [*N*-(2-(6-(Piperazin-1-ylmethyl)benzofuran-2-yl)phenyl)-1-naphthamide hydrochloride] (32t), [*N*-(2-(6-(Piperazin-1-ylmethyl)benzofuran-2-yl)phenyl)-2-naphthamide hydrochloride] (32u), [*N*-(2-(6-(Piperazin-1-ylmethyl)benzofuran-2-yl)phenyl)quinoline-2-carboxamide hydrochloride] (32v), [*N*-(2-(6-(Piperazin-1-ylmethyl)benzofuran-2-yl)phenyl)quinoxaline-2-carboxamide hydrochloride] (32w), [*N*-(2-(6-(Piperazin-1-ylmethyl)benzofuran-2-yl)phenyl)biphenyl-4-carboxamide hydrochloride (32x), [*N*-(2-(6-(Piperazin-1-ylmethyl)benzofuran-2-yl)phenyl)cinnamamide hydrochloride] (32y), [(3*r*,5*r*,7*r*)-*N*-(2-(6-(Piperazin-1-ylmethyl)benzofuran-2-yl)phenyl)adamantane-1-carboxamide hydrochloride] (32z), [1-Phenyl-3-(2-(6-(piperazin-1-ylmethyl)benzofuran-2-yl)phenyl)urea hydrochloride] (32A), [1-(Naphthalen-1-yl)-3-(2-(6-(piperazin-1-ylmethyl)benzofuran-2-yl)phenyl)urea hydrochloride] (32B) and [1-Benzyl-3-(2-(6-(piperazin-1-ylmethyl)benzofuran-2-yl)phenyl)urea hydrochloride] (32C).

3. A compound represented by a following Chemical Formula 4-4, a crystalline form thereof, a hydrate thereof or a salt thereof: 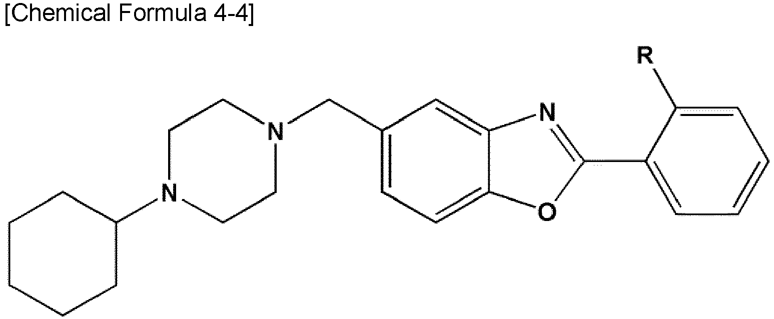 where R is any one selected from the group consisting of benzamide, naphthaamide and quinoxaline-2-carboxamide unsubstituted or substituted with one or more substituents selected from (C1-C4)alkoxy.

4. The compound, crystalline form thereof, hydrate thereof or salt thereof of claim 3, wherein the compound represented by the Chemical Formula 4-4 is selected from the group consisting of [*N*-(2-(5-((4-Cyclohexylpiperazin-1-yl)methyl)benzo[*d*]oxazol-2-yl)phenyl)benzamide] (70a), [*N*-(2-(5-((4-Cyclohexylpiperazin-1-yl)methyl)benzo[*d*]oxazol-2-yl)phenyl)-3,4,5-trimethoxybenzamide] (70b), [*N*-(2-(5-((4-Cyclohexylpiperazin-1 - yl)methyl)benzo[*d*]oxazol-2-yl)phenyl)-2-naphthamide] (70c) and [*N*-(2-(5-((4-Cyclohexylpiperazin-1-yl)methyl)benzo[*d*]oxazol-2-yl)phenyl)quinoxaline-2-carboxamide] (70d).

5. A pharmaceutical composition for use in preventing or treating metabolic diseases, comprising the compound, a crystalline form thereof, a hydrate thereof, or a salt thereof of any one of claims 1 to 4 as an active ingredient.

6. The pharmaceutical composition for use in preventing or treating metabolic diseases of claim 5, wherein the metabolic disease is selected from the group consisting of obesity, diabetes, hyperinsulinemia, hyperuricemia, hyperlipidemia, hypercholesterolemia, hypertriglyceridemia, metabolic syndrome and endothelial dysfunction.

7. A pharmaceutical composition for use in preventing or treating liver diseases, comprising the compound, crystalline form thereof, hydrate thereof, or salt thereof of any one of claims 1 to 4 as an active ingredient.

8. The pharmaceutical composition for use in preventing or treating liver diseases of claim 7, wherein the liver disease is selected from the group consisting of alcoholic fatty liver, non-alcoholic fatty liver and fatty hepatitis.

9. A cosmetic composition for preventing or improving wrinkles, comprising the compound, crystalline form thereof, hydrate thereof, or salt thereof of any one of claims 1 to 4 as an active ingredient.

10. A health food composition comprising the compound crystalline form thereof, hydrate thereof, or salt thereof of any one of claims 1 to 4 as an active ingredient.

## Patentansprüche

1. Verbindung, dargestellt durch eine folgende chemische Formel 2-3, eine kristalline Form davon, ein Hydrat davon oder ein Salz davon: 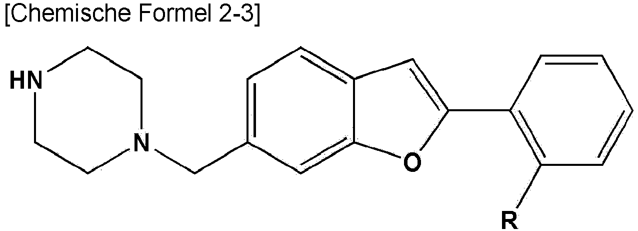 wobei R ausgewählt ist aus der Gruppe bestehend aus Benzamid, Nicotinamid, Isonicotinamid, Naphthaamid, Chinolin-2-carboxamid, Chinoxalin-2-carboxamid, Cinnamamid, Adamantan-1-carboxamid, Phenylharnstoff, Naphthylharnstoff und Benzylharnstoff, unsubstituiert oder substituiert mit einem oder mehreren Substituenten, ausgewählt aus (C1-C4)Alkyl, (C1-C4)Alkoxy, Halogen, Nitro oder Phenyl.

2. Verbindung, kristalline Form davon, Hydrat davon oder Salz davon nach Anspruch 1, wobei die durch die chemische Formel 2-3 dargestellte Verbindung ausgewählt ist aus der Gruppe bestehend aus [N-(2-(6-(Piperazin-1-ylmethyl)benzofuran-2-yl)phenyl)benzamidhydrochlorid] (32a), [2-Methyl-N-(2-(6-(Piperazin-1-ylmethyl)benzofuran-2-yl)phenyl)benzamidhydrochlorid] (32b), [3-Methyl-N-(2-(6-(piperazin-1-ylmethyl)benzofuran-2-yl)phenyl)benzamidhydrochlorid] (32c), [4-Methyl-N-(2-(6-(piperazin-1-ylmethyl)benzofuran-2-yl)phenyl)benzamidhydrochlorid] (32d), [2-Fluor-N-(2-(6-(Piperazin-1-ylmethyl)benzofuran-2-yl)phenyl)benzamidhydrochlorid] (32e), [3-Fluor-N-(2-(6-(Piperazin-1-ylmethyl)benzofuran-2-yl)phenyl)benzamidhydrochlorid] (32f), [2-Brom-N-(2-(6-(Piperazin-1-ylmethyl)benzofuran-2-yl)phenyl)benzamidhydrochlorid] (32g), [3-Brom-N-(2-(6-(Piperazin-1-ylmethyl)benzofuran-2-yl)phenyl)benzamidhydrochlorid] (32h), [4-Brom-N-(2-(6-(Piperazin-1-ylmethyl)benzofuran-2-yl)phenyl)benzamidhydrochlorid] (32i), [2-Chlor-N-(2-(6-(piperazin-1-ylmethyl)benzofuran-2-yl)phenyl)benzamidhydrochlorid] (32j), [3-Chlor-N-(2-(6-(Piperazin-1-ylmethyl)benzofuran-2-yl)phenyl)benzamidhydrochlorid] (32k), [4-Chlor-N-(2-(6-(Piperazin-1-ylmethyl)benzofuran-2-yl)phenyl)benzamidhydrochlorid] (32l), [3-Nitro-N-(2-(6-(piperazin-1-ylmethyl)benzofuran-2-yl)phenyl)benzamidhydrochlorid] (32m), [4-Nitro-N-(2-(6-(Piperazin-1-ylmethyl)benzofuran-2-yl)phenyl)benzamidhydrochlorid] (32n), [3,4,5-Trimethoxy-N-(2-(6-(piperazin-1-ylmethyl)benzofuran-2-yl)phenyl)benzamidhydrochlorid] (32o), [2,4-Dichlor-N-(2-(6-(piperazin-1-ylmethyl)benzofuran-2-yl)phenyl)benzamidhydrochlorid] (32p), [3,4-Dichlor-N-(2-(6-(piperazin-1-ylmethyl)benzofuran-2-yl)phenyl)benzamidhydrochlorid] (32q), [N-(2-(6-(Piperazin-1-ylmethyl)benzofuran-2-yl)phenyl)nicotinamidhydrochlorid] (32r), [N-(2-(6-(Piperazin-1-ylmethyl)benzofuran-2-yl)phenyl)isonicotinamidhydrochlorid] (32s), [N-(2-(6-(Piperazin-1-ylmethyl)benzofuran-2-yl)phenyl)-1-naphthamidhydrochlorid] (32t), [N-(2-(6-(Piperazin-1-ylmethyl)benzofuran-2-yl)phenyl)-2-naphthamidhydrochlorid] (32u), [N-(2-(6-(Piperazin-1-ylmethyl)benzofuran-2-yl)phenyl)chinolin-2-carboxamid-Hydrochlorid] (32v), [N-(2-(6-(Piperazin-1-ylmethyl)benzofuran-2-yl)phenyl)chinoxalin-2-carboxamidhydrochlorid] (32w), [N-(2-(6-(Piperazin-1-ylmethyl)benzofuran-2-yl)phenyl)biphenyl-4-carboxamid Hydrochlorid (32x), [N-(2-(6-(Piperazin-1-ylmethyl)benzofuran-2-yl)phenyl)cinnamamid Hydrochlorid] (32y), [(3r,5r,7r)-N-(2-(6-(Piperazin-1-ylmethyl)benzofuran-2-yl)phenyl)adamantan-1-carboxamid-Hydrochlorid] (32z), [1-Phenyl-3-(2-(6-(Piperazin-1-ylmethyl)benzofuran-2-yl)phenyl)harnstoffhydrochlorid] (32A), [1-(Naphthalin-1-yl)-3-(2-(6-(Piperazin-1-ylmethyl)benzofuran-2-yl)phenyl)harnstoffhydrochlorid] (32B) und [1-Benzyl-3-(2-(6-(piperazin-1-ylmethyl)benzofuran-2-yl)phenyl)harnstoffhydrochlorid] (32C).

3. Verbindung, dargestellt durch die folgende chemische Formel 4-4, eine kristalline Form davon, ein Hydrat davon oder ein Salz davon: 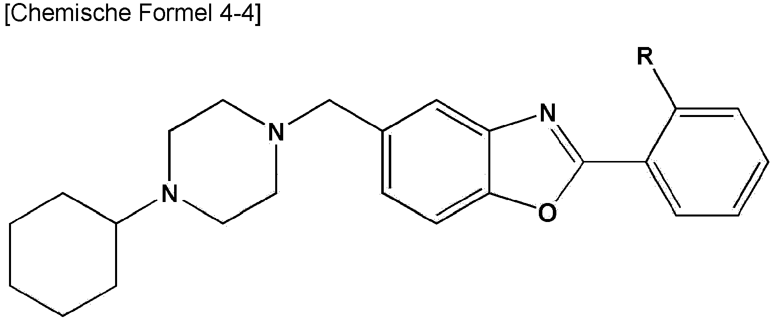 wobei R ausgewählt ist aus der Gruppe bestehend aus Benzamid, Naphthaamid und Chinoxalin-2-carboxamid, unsubstituiert oder substituiert mit einem oder mehreren Substituenten, ausgewählt aus (C1-C4)Alkoxy.

4. Verbindung, kristalline Form davon, Hydrat davon oder Salz davon nach Anspruch 3, wobei die durch die chemische Formel 4-4 dargestellte Verbindung ausgewählt ist aus der Gruppe bestehend aus [N-(2-(5-((4-Cyclohexylpiperazin-1-yl)methyl)benzo[d]oxazol-2-yl)phenyl)benzamid] (70a), [N-(2-(5-((4-Cyclohexylpiperazin-1-yl)methyl)benzo[d]oxazol-2-yl)phenyl)-3,4,5-trimethoxybenzamid] (70b), [N-(2-(5-((4-Cyclohexylpiperazin-1-yl)methyl)benzo[d]oxazol-2-yl)phenyl)-2-naphthamid] (70c) und [N-(2-(5-((4-Cyclohexylpiperazin-1-yl)methyl)benzo[d]oxazol-2-yl)phenyl)chinoxalin-2-carboxamid] (70d).

5. Pharmazeutische Zusammensetzung zur Verwendung bei der Vorbeugung oder Behandlung von Stoffwechselkrankheiten, umfassend die Verbindung, kristalline Form davon, Hydrat davon oder Salz davon nach einem der Ansprüche 1 bis 4 als einen aktiven Inhaltsstoff.

6. Pharmazeutische Zusammensetzung zur Verwendung bei der Vorbeugung oder Behandlung von Stoffwechselerkrankungen nach Anspruch 5, wobei die Stoffwechselerkrankung ausgewählt ist aus der Gruppe bestehend aus Fettleibigkeit, Diabetes, Hyperinsulinämie, Hyperurikämie, Hyperlipidämie, Hypercholesterinämie, Hypertriglyceridämie, metabolischem Syndrom und endothelialer Dysfunktion.

7. Pharmazeutische Zusammensetzung zur Verwendung bei der Vorbeugung oder Behandlung von Lebererkrankungen, umfassend die Verbindung, kristalline Form davon, Hydrat davon oder Salz davon nach einem der Ansprüche 1 bis 4 als einen aktiven Inhaltsstoff.

8. Pharmazeutische Zusammensetzung zur Verwendung bei der Vorbeugung oder Behandlung von Lebererkrankungen nach Anspruch 7, wobei die Lebererkrankung ausgewählt ist aus der Gruppe bestehend aus alkoholischer Fettleber, nicht-alkoholischer Fettleber und Fetthepatitis.

9. Kosmetische Zusammensetzung zur Vorbeugung oder Verbesserung von Falten, umfassend die Verbindung, kristalline Form davon, Hydrat davon oder Salz davon nach einem der Ansprüche 1 bis 4 als einen aktiven Inhaltsstoff.

10. Gesundheitsnahrungsmittelzusammensetzung, umfassend die Verbindung, kristalline Form davon, Hydrat davon oder Salz davon nach einem der Ansprüche 1 bis 4 als einen aktiven Inhaltsstoff.

## Revendications

1. Un composé représenté par une formule chimique 2-3 suivante, une forme cristalline de celui-ci, un hydrate de celui-ci ou un sel de celui-ci : 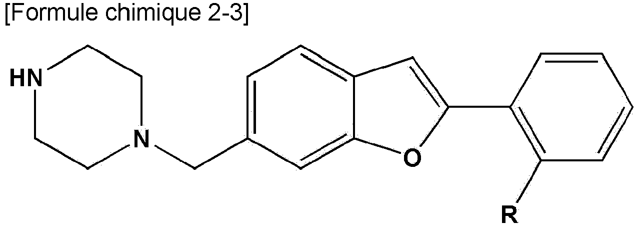 dans laquelle R est choisi parmi le groupe constitué par le benzamide, le nicotinamide, l'isonicotinamide, le naphtaamide, le quinoléine-2-carboxamide, le quinoxaline-2-carboxamide, le cinnamamide, l'adamantane-1-carboxamide, la phénylurée, la naphtylurée et la benzylurée, qui est non substitué ou substitué par un ou plusieurs substituants choisis parmi les groupes alkyle en C1-C4, alcoxy en C1-C4, halogène, nitro ou phényle.

2. Composé, forme cristalline de celui-ci, hydrate de celui-ci ou sel de celui-ci selon la revendication 1, dans lequel le composé représenté par la formule chimique 2-3 est choisi parmi le groupe constitué du chlorhydrate de [N-(2-(6-(pipérazine-1-ylméthyl)benzofuran-2-yl)phényl)benzamide] (32a), le chlorhydrate de [2-Méthyl-N-(2-(6-(pipérazine-1-ylméthyl)benzofuran-2-yl)phényl)benzamide] (32b), le chlorhydrate de [3-Méthyl-N-(2-(6-(pipérazin-1-ylméthyl)benzofuran-2-yl)phényl)benzamide] (32c), le chlorhydrate de [4-Méthyl-N-(2-(6-(pipérazin-1-ylméthyl)benzofuran-2-yl)phényl)benzamide] (32d), le chlorhydrate de [2-Fluoro-N-(2-(6-(pipérazin-1-ylméthyl)benzofuran-2-yl)phényl)benzamide] (32e), le chlorhydrate de [3-Fluoro-N-(2-(6-(pipérazin-1-ylméthyl)benzofuran-2-yl)phényl)benzamide] (32f), le chlorhydrate de [2-Bromo-N-(2-(6-(pipérazin-1-ylméthyl)benzofuran-2-yl)phényl)benzamide] (32g), le chlorhydrate de [3-Bromo-N-(2-(6-(pipérazin-1-ylméthyl)benzofuran-2-yl)phényl)benzamide] (32h), le chlorhydrate de [4-Bromo-N-(2-(6-(pipérazin-1-ylméthyl)benzofuran-2-yl)phényl)benzamide] (32i), le chlorhydrate de [2-Chloro-N-(2-(6-(pipérazin-1-ylméthyl)benzofuran-2-yl)phényl)benzamide] (32j), le chlorhydrate de [3-Chloro-N-(2-(6-(pipérazin-1-ylméthyl)benzofuran-2-yl)phényl)benzamide] (32k), le chlorhydrate de [4-Chloro-N-(2-(6-(pipérazin-1-ylméthyl)benzofuran-2-yl)phényl)benzamide] (32l), le chlorhydrate de [3-Nitro-N-(2-(6-(pipérazin-1-ylméthyl)benzofuran-2-yl)phényl)benzamide] (32m), le chlorhydrate de [4-Nitro-N-(2-(6-(pipérazin-1-ylméthyl)benzofuran-2-yl)phényl)benzamide] (32n), le chlorhydrate de [3,4,5-Triméthoxy-N-(2-(6-(pipérazine-1-ylméthyl)benzofuran-2-yl)phényl)benzamide] (32o), le chlorhydrate de [2,4-Dichloro-N-(2-(6-(pipérazin-1-ylméthyl)benzofuran-2-yl)phényl)benzamide] (32p), le chlorhydrate de [3,4-Dichloro-N-(2-(6-(pipérazin-1-ylméthyl)benzofuran-2-yl)phényl)benzamide] (32q), le chlorhydrate de [N-(2-(6-(pipérazin-1-ylméthyl)benzofuran-2-yl)phényl)nicotinamide] (32r), le chlorhydrate de [N-(2-(6-(Pipérazin-1-ylméthyl)benzofuran-2-yl)phényl)isonicotinamide] (32s), le chlorhydrate de [N-(2-(6-(Pipérazin-1-ylméthyl)benzofuran-2-yl)phényl)-1-naphtamide] (32t), le chlorhydrate de [N-(2-(6-(Pipérazin-1-ylméthyl)benzofuran-2-yl)phényl)-2-naphtamide] (32u), le chlorhydrate de [N-(2-(6-(Pipérazin-1-ylméthyl)benzofuran-2-yl)phényl)quinoléine-2-carboxamide] (32v), le chlorhydrate de [N-(2-(6-(Pipérazin-1-ylméthyl)benzofuran-2-yl)phényl)quinoxaline-2-carboxamide] (32w), le chlorhydrate de [N-(2-(6-(Pipérazin-1-ylméthyl)benzofuran-2-yl)phényl)biphényl-4-carboxamide (32x), le chlorhydrate de [N-(2-(6-(Pipérazin-1-ylméthyl)benzofuran-2-yl)phényl)cinnamamide] (32y), le chlorhydrate de [(3r,5r,7r)-N-(2-(6-(Pipérazin-1-ylméthyl)benzofuran-2-yl)phényl)adamantane-1-carboxamide] (32z), le chlorhydrate de [1-Phényl-3-(2-(6-(pipérazine-1-ylméthyl)benzofuran-2-yl)phényl)urée] (32A), le chlorhydrate de [1-(naphtalène-1-yl)-3-(2-(6-(pipérazine-1-ylméthyl)benzofuran-2-yl)phényl)urée] (32B) et le chlorhydrate de [1-Benzyl-3-(2-(6-(pipérazin-1-ylméthyl)benzofuran-2-yl)phényl)urée] (32C).

3. Composé représenté par la formule chimique 4-4 suivante, une forme cristalline de celui-ci, un hydrate de celui-ci ou un sel de celui-ci : 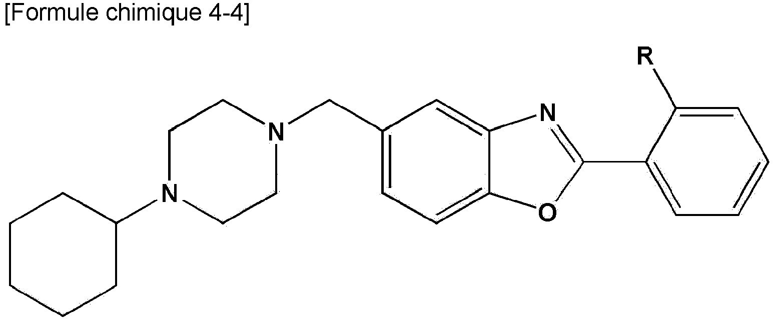 dans laquelle R est choisi parmi le groupe constitué par le benzamide, le naphtaamide et le quinoxaline-2-carboxamide non substitué ou substitué par un ou plusieurs substituants choisis parmi les alcoxy en C1-C4.

4. Composé, forme cristalline de celui-ci, hydrate de celui-ci ou sel de celui-ci selon la revendication 3, dans lequel le composé représenté par la formule chimique 4-4 est choisi parmi le groupe constitué par le [N-(2-(5-((4-Cyclohexylpipérazin-1-yl)méthyl)benzo[d]oxazol-2-yl)phényl)benzamide] (70a), le [N-(2-(5-((4-Cyclohexylpiperazin-1-yl)méthyl)benzo[d]oxazol-2-yl)phényl)-3,4,5-triméthoxybenzamide] (70b), le [N-(2-(5-((4-cyclohexylpipérazin-1-yl)méthyl)benzo[d]oxazol-2-yl)phényl)-2-naphtamide] (70c) et le [N-(2-(5-((4-Cyclohexylpipérazin-1-yl)méthyl)benzo[d]oxazol-2-yl)phényl)quinoxaline-2-carboxamide] (70d).

5. Un composé pharmaceutique pour utilisation dans la prévention ou le traitement des maladies métaboliques, comprenant le composé, une forme cristalline de celui-ci, un hydrate de celui-ci, ou un sel de celui-ci selon l'une quelconque des revendications 1 à 4 comme ingrédient actif.

6. Un composé pharmaceutique pour utilisation dans la prévention ou le traitement des maladies métaboliques selon la revendication 5, dans lequel la maladie métabolique est choisie parmi le groupe constitué par l'obésité, le diabète, l'hyperinsulinémie, l'hyperuricémie, l'hyperlipidémie, l'hypercholestérolémie, l'hypertriglycéridémie, le syndrome métabolique et le dysfonctionnement endothélial.

7. Un composé pharmaceutique pour utilisation dans la prévention ou le traitement des maladies du foie, comprenant le composé, la forme cristalline de celui-ci, l'hydrate de celui-ci, ou le sel de celui-ci de l'une quelconque des revendications 1 à 4 comme ingrédient actif.

8. Un composé pharmaceutique pour utilisation dans la prévention ou le traitement des maladies du foie selon la revendication 7, dans lequel la maladie du foie est choisie parmi le groupe constitué du foie gras alcoolique, du foie gras non alcoolique et de l'hépatite grasse.

9. Un composé cosmétique pour prévenir ou améliorer des rides, comprenant le composé, forme cristalline de celui-ci, hydrate de celui-ci, ou sel de celui-ci de l'une quelconque des revendications 1 à 4 comme ingredient actif.

10. Un composé alimentaire sain comprenant le composé, forme cristalline de celui-ci, hydrate de celui-ci, ou sel de celui-ci de l'une quelconque des revendications 1 à 4 comme ingrédient actif.
